(19) Europäisches Patentamt
European Patent Office
Office européen des brevets

(11) EP 3 950 678 A1

(12) **EUROPEAN PATENT APPLICATION**
published in accordance with Art. 153(4) EPC

(43) Date of publication:
**09.02.2022 Bulletin 2022/06**

(21) Application number: **20784231.1**

(22) Date of filing: **31.03.2020**

(51) International Patent Classification (IPC):
***C07D 231/12*** (2006.01) ***C07D 261/08*** (2006.01)
***C07D 413/04*** (2006.01) ***C07D 413/12*** (2006.01)
***C07D 413/14*** (2006.01) ***C07D 401/14*** (2006.01)
***C07D 403/12*** (2006.01) ***C07D 403/14*** (2006.01)
***C07D 417/12*** (2006.01) ***C07D 498/04*** (2006.01)
***A61K 31/4155*** (2006.01) ***A61K 31/422*** (2006.01)
***A61K 31/423*** (2006.01) ***A61K 31/427*** (2006.01)
***A61K 31/4439*** (2006.01) ***A61K 31/497*** (2006.01)
***A61K 31/501*** (2006.01) ***A61K 31/506*** (2006.01)
***A61K 31/4355*** (2006.01) ***A61P 35/00*** (2006.01)

(52) Cooperative Patent Classification (CPC):
**A61K 31/4155; A61K 31/422; A61K 31/423; A61K 31/427; A61K 31/4355; A61K 31/4439; A61K 31/497; A61K 31/501; A61K 31/506; A61P 35/00; C07B 59/00; C07D 231/12; C07D 261/08; C07D 263/32; C07D 271/06;** (Cont.)

(86) International application number:
**PCT/CN2020/082513**

(87) International publication number:
**WO 2020/200209 (08.10.2020 Gazette 2020/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.04.2019 CN 201910263315**

(71) Applicant: **Hinova Pharmaceuticals Inc.**
**Chengdu, Sichuan 610041 (CN)**

(72) Inventors:
- **DU, Wu**
  **Chengdu, Sichuan 610041 (CN)**
- **LV, Haibin**
  **Chengdu, Sichuan 610041 (CN)**
- **QIN, Dekun**
  **Chengdu, Sichuan 610041 (CN)**
- **LI, Haibo**
  **Chengdu, Sichuan 610041 (CN)**
- **LI, Yu**
  **Chengdu, Sichuan 610041 (CN)**
- **TU, Zhilin**
  **Chengdu, Sichuan 610041 (CN)**
- **CHEN, Yuanwei**
  **Chengdu, Sichuan 610041 (CN)**
- **LI, Xinghai**
  **Chengdu, Sichuan 610041 (CN)**

(74) Representative: **Altmann Stößel Dick Patentanwälte PartG mbB**
**Theodor-Heuss-Anlage 2**
**68165 Mannheim (DE)**

# (54) AROMATIC AMINE COMPOUND AND USE THEREOF IN PREPARATION OF AR AND BRD4 DUAL INHIBITORS AND REGULATORS

(57) Provided are an aromatic amine compound and a use thereof in the preparation of AR and BRD4 dual inhibitors and regulators. Specificially provided is the the compound shown in formula I, said compound having dual inhibitory effects on AR and BRD4. The compound is not only capable of inhibiting the proliferation of androgen receptor AR multi-expressed prostate cancer cell line LNCAP/AR, but also shows good inhibitory effects on prostate cancer lines VCaP and RRRV1 which are resistant to prostate cancer drugs (enzalutamide) on the market. The compound is itself a compound capable of simultaneously identifying AR and BRD4 dual targets and

EP 3 950 678 A1

can be used as a dual AR/BRD4 inhibitor, and is also capable of being used for preparing proteolysistargeting chimeras (PROTACs) for inducing the degradation of AR/BRD4 dual targets, and has good propects for application in the preparation of drugs for the treatment of AR and BRD4-related diseases.



(Cont. next page)

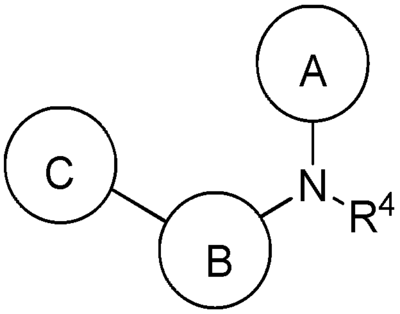

Formula I

(52) Cooperative Patent Classification (CPC): (Cont.)
**C07D 401/14; C07D 403/12; C07D 403/14; C07D 413/04; C07D 413/12; C07D 413/14; C07D 417/12; C07D 498/04**

## Description

### Technical field

The present invention relates to the field of medicinal synthesis, and in particular to aromatic amine compounds and their use in the preparation of AR and BRD4 dual inhibitors and regulators.

### Background technology

With the global population growing and aging, the incidence of prostate cancer continues to increase. At present, the main treatment means for prostate cancer is androgen deprivation therapy. Androgen receptor (AR) belongs to the nuclear receptor family and is a type of ligand-dependent transcription factor. The abnormal regulation of AR signaling pathway plays an important role in the occurrence and development of prostate cancer. Studies have shown that castration-resistant prostate cancer (CRPC) still depends on the role of AR. Androgen receptor contains 918 amino acids and has a similar structure and function to other nuclear receptors. It consists of three key structural domains, namely DNA binding domain (DBD), the ligand binding domain (LBD), and N-terminal domain (NTD), and DBD and LBD are connected by a hinge. The LBD present at the carbon end of AR is the site where AR binds to the ligand, which determines the binding specificity of the ligand and AR, and the ligand binds to the LBD to activate AR. At present, two transcriptional activation domains have been identified in AR, namely activation function 1 (AF1) in the NTD domain and the highly conserved hydrophobic pocket activation function 2 (AF2) in the LBD domain. Before 2010, docetaxel-based chemotherapy was the only treatment that could prolong the survival of patients with metastatic CRPC. Since 2011, FDA has successively approved three AR signaling pathway inhibitors, namely abiraterone acetate and enzalutamide approved in 2011 and 2012 for metastatic castration-resistant prostate cancer, as well as apalutamide, which was just approved for non-metastatic CRPC in 2018.

Although the second-generation AR signaling pathway inhibitors abiraterone and enzalutamide have achieved some success in clinical treatment, drug resistance has appeared in the clinic. The F876L mutation in the ligand binding region is a missense mutation that produces resistance to enzalutamide and turns it from an antagonist to an agonist. In addition, AR splicing mutants, especially AR-v7 mutations lacking a ligand binding region, are important reasons for mediating the resistance of second-generation drug. Therefore, there is an urgent clinical need for novel inhibitors of AR signaling pathway to treat CRPC.

BET (bromodomain and extra-terminal domain) is a type of epigenetic regulatory factor that regulates gene expression by BD1 and BD2 domains recognizing acetylated histones on DNA. BET protein family is composed of BRD2, BRD3, BRD4 and BRDT. In addition to BRDT which only exists in the testis, the other three protein subtypes are widely expressed in various tissues and cells. Studies have shown that BRD2\3\4 can directly bind to AR to regulate the expression of its downstream genes, and this interaction between AR and BD1 can be blocked by BET inhibitors, thereby blocking AR-mediated gene transcription and inhibiting CRPC tumors. The studies have further indicated that this interaction also has a good inhibitory effect on AR-v7 positive and androgen-independent 22Rv1 tumor model. In recent years, a number of BRD protein inhibitors have entered clinical studies for the treatment of CRPC. These inhibitors include OTX-105, ZEN003694, GS-5829, and the similar, among which GS-5829 can also be used for lymphoma.

Studies have also suggested that in advanced prostate cancer, the upregulation of AR enhances the bromodomain-mediated chromatin opening, and AR-overexpressing cells are more sensitive to BET inhibitors. In addition, studies have shown that CRPC cells resistant to enzalutamide are still sensitive to BET inhibitors (such as JQ1). Therefore, the combination of AR and BET inhibitors can better inhibit the growth of prostate cancer tumors compared with the use of anti-androgen drugs alone.

Traditional small molecule inhibitors inhibit the function of the target protein by binding to the target protein. However, the long-term use of small molecule drugs will inevitably lead to drug resistance, and in order to achieve the desired effect, small molecule compounds need to maintain a certain concentration in the cell, and higher concentrations of small molecules will cause adverse reactions due to off-target. Therefore, finding small molecule compounds that can overcome these shortcomings is of great significance in the development of new drugs.

In recent years, proteolytic targeting chimeras (PROTACs) have attracted widespread attention as small molecules that can induce degradation of target proteins. PROTACs, as bifunctional molecules, include a small molecule compound that can bind to the target protein (protein of interest, POI), a linking group introduced at its appropriate position, and a small molecule compound that can bind to E3 ubiquitinase. PROTACs, as small molecule probes, can simultaneously bind to the target protein and E3 ubiquitinating enzyme, thereby promoting the ubiquitination of the target protein, which is thus recognized and degraded by the proteasome.

In the past 10 years, many tumor-related target proteins (including AR, ER, BRD4, ERRa, RIPK2, etc.) have been confirmed to be regulated and degraded based on PROTACs. And the latest research has confirmed the catalytic properties of PROTACs, indicating that the concentration of chimera molecules is lower than that required by a single

inhibitor to achieve the same therapeutic effect. Therefore, a novel tumor-treatment strategy that uses PROTACs to induce protein degradation can regulate the level of target protein through the intracellular ubiquitin-proteasome degradation system, thereby overcoming the shortcomings of traditional small molecule inhibitors. Therefore, the preparation of inhibitors with dual inhibitory effects on AR and BET is of great significance in the preparation of PROTACs that dually target the degradation of AR and BET and in the treatment of malignant tumors (especially prostate cancer).

**Content of the invention:**

The object of the present invention is to provide a compound having a dual inhibitory effect on AR and BET.

The present invention provides a compound of formula I, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereoisomer thereof, or a mixture thereof, or a metabolite thereof, or a metabolic precursor thereof, or an isotope-substituted form thereof:

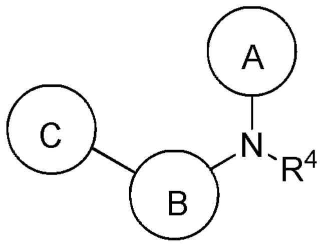

Formula I

wherein

each of rings A, B, and C is independently selected from the group consisting of none, substituted or unsubstituted unsaturated heterocycles, substituted or unsubstituted unsaturated carbocyclic rings and substituted or unsubstituted fused rings; preferably, none, substituted or unsubstituted monocyclic aromatic ring, substituted or unsubstituted monocyclic heteroaromatic ring and substituted or unsubstituted fused ring; more preferably, none, substituted or unsubstituted 3-8 membered monocyclic aromatic ring, substituted or unsubstituted 3-8-membered monocyclic heteroaromatic ring, substituted or unsubstituted heteroaromatic ring-fused heteroaromatic ring, substituted or unsubstituted benzo-aromatic ring, substituted or unsubstituted benzo-heteroaromatic ring, substituted or unsubstituted benzo-saturated carbocyclic ring and substituted or unsubstituted benzo-saturated heterocyclic ring; rings A, B, and C are not none at the same time;

each of the substituents on rings A, B, and C is independently selected from the group consisting of deuterium, halogen, -CN, hydroxyl, nitro, amino,

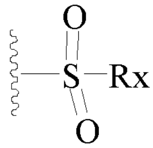

, $-L_0$-OH, $-L_3$-C(O)$R_7$, $-L_4$-CO(O)$R^8$, $-L_5$-(O)CO$R^9$, $-L_6$-NHC(O)$R^{10}$, $-L_7$-C(O)NH$R^{11}$, $-L_8$-CN, an alkenyl substituted with one or more $R^{12}$, an alkynyl substituted with one or more $R^{13}$, an alkyl substituted with one or more $R^1$, an alkoxy substituted with one or more $R^2$, an aryl or an heteroaryl substituted with one or more $R^3$, a cycloalkyl substituted with one or more $R^5$, and a heterocyclic group substituted with one or more $R^6$; each of said Rx, $R^1$, $R^2$, $R^3$, $R^5$, $R^6$, $R^7$, $R^8$, $R^9$, $R^{10}$, $R^{11}$, $R^{12}$, $R^{13}$ is independently selected from the group consisting of H, deuterium, halogen, -CN, hydroxyl, nitro, amino, alkyl or a deuterated or halogenated compound thereof, and -Lo-OH, where each of $L_0$, $L_3$, $L_4$, $L_5$, $L_6$, $L_7$, $L_8$ is independently selected from the group consisting of none, $C_1$-$C_8$ alkyl, and cycloalkyl; $R^4$ is independently selected from the group consisting of H, deuterium, halogen, -CN, hydroxyl, nitro, amino,

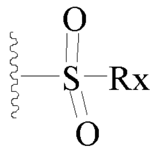

, $-L_0$-OH, $-L_3-C(O)R_7$, $-L_4-CO(O)R^8$, $-L_5-(O)COR^9$, $-L_6-NHC(O)R^{10}$, $-L_7-C(O)NHR^{11}$, an alkenyl substituted with one or more $R^{12}$, an alkynyl substituted with one or more $R^{13}$, an alkyl substituted with one or more $R^1$, an alkoxy substituted with one or more $R^2$, an aryl or an heteroaryl substituted with one or more $R^3$, a cycloalkyl substituted with one or more $R^5$, and a heterocyclic group substituted with one or more $R^6$; each of said $R_X$, $R^1$, $R^2$, $R^3$, $R^5$, $R^6$, $R^7$, $R^8$, $R^9$, $R^{10}$, $R^{11}$, $R^{12}$, $R^{13}$ is independently selected from the group consisting of H, deuterium, halogen, -CN, hydroxyl, nitro, amino, alkyl or a deuterated or halogenated compound thereof, and -Lo-OH, where each of $L_0$, $L_3$, $L_4$, $L_5$, $L_6$, $L_7$ is independently selected from 0-8 methylene groups;

or, any two groups of the substituents on rings A, B, and C, and $R^4$, together with the substituted atom to which they are linked are connected to form a ring.

Further,

said compound has the structure of formula II:

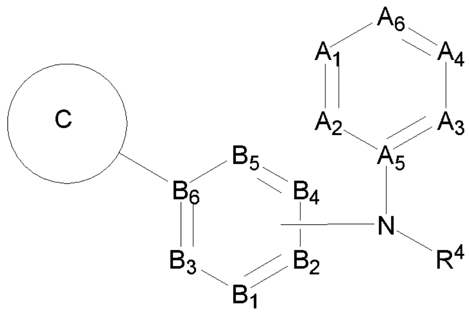

Formula II

each of $B_1$, $B_2$, $B_3$, $B_4$, $B_5$, $B_6$, $A_1$, $A_2$, $A_3$, $A_4$, $A_5$, $A_6$ is independently selected from $CR^0$ or N; $R^0$ is selected from the group consisting of H, -CN, amino, nitro, halogen, $-L_{0}$-OH,

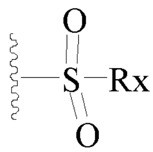

, $-C(O)NHR^{11}$, $C_1$-$C_5$ alkyl or a deuterated or halogenated or cyano-substituted compound thereof, $C_1$-$C_5$ alkoxy or a deuterated or halogenated or cyano-substituted compound thereof, substituted or unsubstituted 3-6 membered cycloalkyl, substituted or unsubstituted 3-6 membered cycloalkyl, or two adjacent substituents on the ring, together with the substituted atom to which they are linked, form a substituted or unsubstituted 3-6 membered heterocyclic ring; wherein, each of the substituents in said 3-6 membered cycloalkyl is independently selected from the group consisting of - CN, amino, nitro, halogen, $C_1$-$C_3$ alkyl or a deuterated or halogenated compound thereof, and $-L_1$-OH; each of said $L_0$ and $L_1$ is independently 0-5 methylene groups; each of said $R_x$ and $R^{11}$ is independently selected from the group consisting of H and $C_1C_3$ alkyl;

ring C and $R^4$ are as described above.

Further,

said compound has the structure of formula III-A:

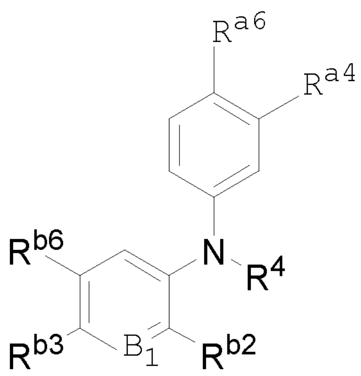

Formula III-A

wherein each of $R^{a4}$ and $R^{a6}$ is independently selected from the group consisting of H, halogen and substituted or unsubstituted five-membered unsaturated heterocyclic ring; wherein the substituents on the five-membered unsaturated heterocyclic ring are selected from the group consisting of deuterated or non-deuterated $C_1$-$C_2$ alkyl groups, and -$L_1$-OH; $L_1$ is 0-2 methylene groups;

$R^4$ is selected from the group consisting of H, deuterated or non-deuterated $C_1$-$C_2$ alkyl groups, and -$L_1$-OH; $L_2$ is 0-5 methylene groups;

$B_1$ is selected from the group consisting of CH and N;

$R^{b2}$ is deuterated or non-deuterated methyl;

each of $R^{b3}$ and $R^{b6}$ is independently selected from the group consisting of H, -CN, amino, nitro, halogen, -Lo-OH,

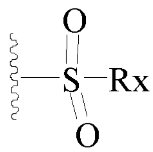

, -C(O)NHR$^{11}$, $C_1$-$C_3$ alkyl or a deuterated or halogenated or cyano-substituted compound thereof, $C_1$-$C_3$ alkoxy or a deuterated or halogenated or cyano-substituted compound thereof, substituted or unsubstituted 3-6-membered saturated cycloalkyl, and substituted or unsubstituted 5-membered unsaturated heterocyclic group, or $R^{b3}$ and $R^{b6}$, together with the substituted atom to which they are linked, form a substituted or unsubstituted 5-membered unsaturated heterocyclic ring; wherein, each of the substituents in said 5-membered unsaturated heterocyclic group and 5-membered unsaturated heterocyclic ring is independently selected from the group consisting of -CN, amino, nitro, halogen, $C_1$-$C_2$ alkyl or a deuterated or halogenated compound thereof, and -$L_1$-OH; $L_1$ is 0-2 methylene groups; the substituent in said 3-6 membered saturated cycloalkyl is independently CN.

Further,

said compound has the structure of formula III-B:

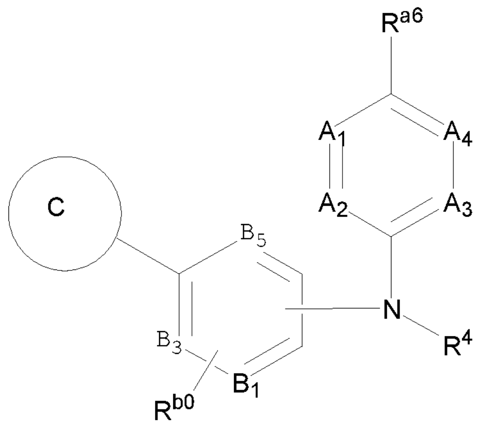

Formula III-B

wherein ring C is selected from the group consisting of

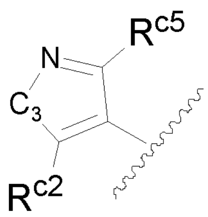

,

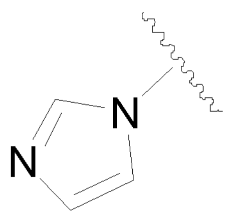

,

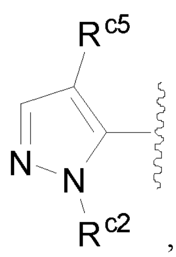

,

and

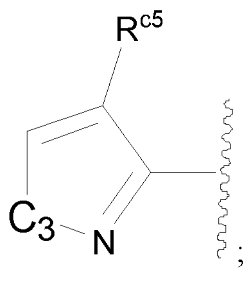

;

$C_3$ is selected from the group consisting of O and S; or each of $NR^{c3}$, $R^{c2}$, $R^{c3}$, and $R^{c5}$ is independently selected from the group consisting of H, halogen, $C_1$-$C_3$ alkoxy or a deuterated or halogenated compound thereof, and $-L_0$-OH; $L_0$ is 0-5 methylene groups;

each of $B_1$, $B_3$, and $B_5$ is independently selected from the group consisting of CH and N;

$R^{b0}$ is selected from the group consisting of H, halogen, and $C_1$-$C_3$ alkoxy or a deuterated or halogenated compound thereof;

$R^4$ is selected from the group consisting of H, $C_1$-$C_2$ alkyl or a deuterated or halogenated compound thereof, and $-L_2$-OH; $L_2$ is 0-5 methylene groups;

each of $A_1$, $A_2$, $A_3$, $A_4$ is independently selected from the group consisting of N and $CR^{a0}$; $A_5$ is C; $A_6$ is selected from the group consisting of N and $CR^{a6}$; wherein, each of $R^{a0}$ and $R^{a6}$ is independently selected from the group consisting of H, -CN, amino, nitro, halogen,

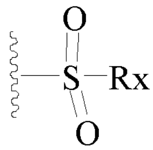

, -C(O)NHR$^{11}$, $C_1$-$C_3$ alkyl or a deuterated or halogenated or cyano-substituted compound thereof, $C_1$-$C_3$ alkoxy or a deuterated or halogenated or cyano-substituted compound thereof, substituted or unsubstituted 3-6-membered cycloalkyl, and substituted or unsubstituted 5-membered heterocyclic group, or $R^{a6}$ and the substituent at the ortho position of $R^{a6}$, together with the substituted atom to which they are linked, form a substituted or unsubstituted 5-membered heterocyclic ring; wherein, each of the substituents in said 5-membered heterocyclic ring, 3-6-membered cycloalkyl, and 5-membered heterocyclic group is independently selected from the group consisting of -CN, amino, nitro, halogen, $C_1$-$C_2$ alkyl or a deuterated or halogenated compound thereof, and -$L_1$-OH; $L_1$ is 0-2 methylene groups; each of $R_x$ and $R^{11}$ is independently selected from the group consisting of H and $C_1$-$C_2$ alkyl;
preferably, the 5-membered heterocyclic ring mentioned above is a 5-membered unsaturated heterocyclic group, the 5-membered heterocyclic group mentioned above is a 5-membered unsaturated heterocyclic group, and the heteroatom is selected from the group consisting of N, S, and O; the 3-6 membered cycloalkyl mentioned above is 3-6 membered saturated cycloalkyl.

Further,

said compound has the structure of formula III-B1:

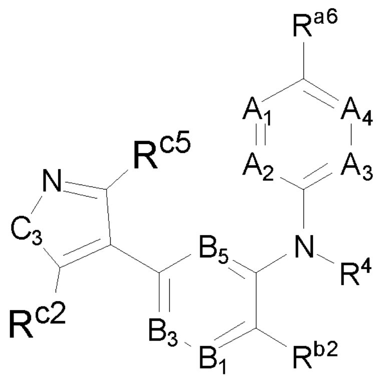

Formula III-B1

wherein $C_3$ is selected from the group consisting of O and S; or each of $NR^{c3}$, $R^{c2}$, $R^{c5}$, and $R^{c3}$ is independently selected from the group consisting of H, halogen, $C_1$-$C_3$ alkyl or a deuterated or halogenated compound thereof, and -$L_0$-OH; $L_0$ is 0-5 methylene groups;
each of $B_1$, $B_3$, and $B_5$ is independently selected from the group consisting of CH and N;
$R^{b2}$ is deuterated or undeuterated methyl;
$R^4$ is selected from the group consisting of H, deuterated or undeuterated $C_1$-$C_2$ alkyl, and -$L_2$-OH; $L_2$ is 0-5 methylene groups;
each of $A_1$, $A_2$, $A_3$, $A_4$ is independently selected from the group consisting of N and $CR^{a0}$; wherein, each of $R^{a0}$ and $R^{a6}$ is independently selected from the group consisting of H, -CN, amino, nitro, halogen,

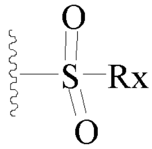

,

-C(O)NHR$^{11}$, $C_1$-$C_3$ alkyl or a deuterated or halogenated or cyano-substituted compound thereof, $C_1$-$C_3$ alkoxy or a deuterated or halogenated or cyano-substituted compound thereof, substituted or unsubstituted 3-6-membered cycloalkyl, and substituted or unsubstituted 5-membered unsaturated heterocyclic group, or $R^{a6}$ and the substituent at the ortho position of $R^{a6}$, together with the substituted atom to which they are linked, form a substituted or unsubstituted 5-membered unsaturated heterocyclic ring; wherein, each of the substituents in said 5-membered unsaturated heterocyclic group and 5-membered unsaturated heterocyclic ring is independently selected from the group consisting of - CN, amino, nitro, halogen, $C_1$-$C_2$ alkyl or a deuterated or halogenated compound thereof, and -$L_1$-OH; $L_1$ is 0-2 methylene groups; the substituent in said 3-6 membered saturated cycloalkyl is -CN; each of $R_x$ and $R^{11}$ is independently selected from the group consisting of H and $C_1$-$C_2$ alkyl.

Further,

said compound has the structure of formula III-B1a:

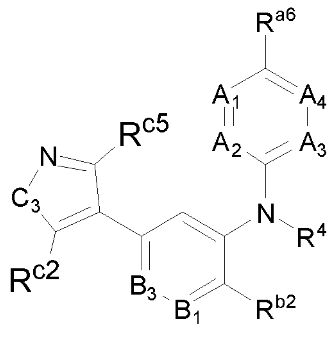

Formula III-B1a

wherein $C_3$ is selected from the group consisting of O and S; or each of $R^{c2}$ and $R^{c5}$ is independently selected from the group consisting of H and $C_1$-$C_3$ alkyl or a deuterated or halogenated compound thereof, and preferably, $R^{c2}$ and $R^{c5}$ are methyl;

each of $B_1$, $B_3$, and $B_5$ is independently selected from CH and N;

$R^{b2}$ is selected from deuterated or undeuterated methyl;

$R^4$ is selected from the group consisting of H, deuterated or undeuterated $C_1$-$C_2$ alkyl, and -$L_2$-OH; $L_2$ is 0-5 methylene groups;

each of $A_1$, $A_2$, $A_3$, $A_4$ is independently selected from the group consisting of N and $CR^{a0}$; wherein each of $R^{a0}$ and $R^{a6}$ is independently selected from the group consisting of H, -CN, amino, nitro, halogen,

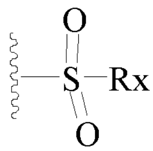

, -C(O)NHR$^{11}$, $C_1$-$C_3$ alkyl or a deuterated or halogenated or cyano-substituted compound thereof, $C_1$-$C_3$ alkoxy or a deuterated or halogenated or cyano-substituted compound thereof, substituted or unsubstituted 3-6-membered saturated cycloalkyl, and substituted or unsubstituted 5-membered unsaturated heterocyclic group, or $R^{a6}$ and the substituent at the ortho position of $R^{a6}$, together with the substituted atom to which they are linked, form a substituted or unsubstituted 5-membered unsaturated heterocyclic ring; wherein, each of the substituents in said 5-membered unsaturated heterocyclic group and 5-membered unsaturated heterocyclic ring is independently selected from the group consisting of - CN, amino, nitro, halogen, $C_1$-$C_2$ alkyl or a deuterated or halogenated compound thereof, and -$L_1$-OH; $L_1$ is 0-2 methylene groups; the substituent in said 3-6 membered saturated cycloalkyl is -CN; each of $R_x$ and $R^{11}$ is independently selected from the group consisting of H and $C_1$-$C_2$ alkyl;

or, the compound has a structure of formula III-B1b:

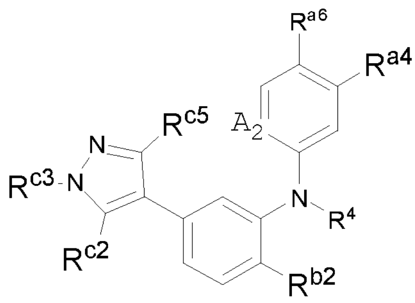

Formula III-B1b

wherein each of $R^{c2}$, $R^{c3}$ and $R^{c5}$ is independently selected from the group consisting of H, $C_1$-$C_3$ alkyl or a deuterated or halogenated compound thereof, and -$L_0$-OH; preferably, $R^{c3}$ is selected from the group consisting of H, $C_1$-$C_3$ alkyl or a deuterated or halogenated compound thereof, and -Lo-OH, while $R^{c2}$ and $R^{c5}$ are methyl; wherein, $L_0$ is 0-5 methylene groups;

$R^4$ is selected from the group consisting of H, deuterated or undeuterated $C_1$-$C_2$ alkyl, and -$L_2$-OH; $L_2$ is 0-5 methylene groups;

$R^{b2}$ is deuterated or undeuterated methyl;

$A_2$ is N or CH;

wach of $R^{a4}$ and $R^{a6}$ is independently selected from the group consisting of H, -CN, amino, nitro, halogen,

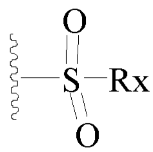

, -C(O)NHR$^{11}$, $C_1$-$C_3$ alkyl or a deuterated or halogenated or cyano-substituted compound thereof, $C_1$-$C_3$ alkoxy or a deuterated or halogenated or cyano-substituted compound thereof, substituted or unsubstituted 3-6-membered saturated cycloalkyl, and substituted or unsubstituted 5-membered unsaturated heterocyclic group, or $R^{a6}$ and the substituent at the ortho position of $R^{a6}$, together with the substituted atom to which they are linked, form a substituted or unsubstituted 5-membered unsaturated heterocyclic ring; wherein each of the substituents in said 5-membered unsaturated heterocyclic group and 5-membered unsaturated heterocyclic ring is independently selected from the group consisting of - CN, amino, nitro, halogen, $C_1$-$C_2$ alkyl or a deuterated or halogenated compound thereof, and -$L_1$-OH; $L_1$ is 0-2 methylene groups; the substituent in said 3-6 membered saturated cycloalkyl is -CN; each of $R_x$ and $R^{11}$ is independently selected from the group consisting of H and $C_1$-$C_2$ alkyl.

Further,

said compound has the structure of formula III-B2:

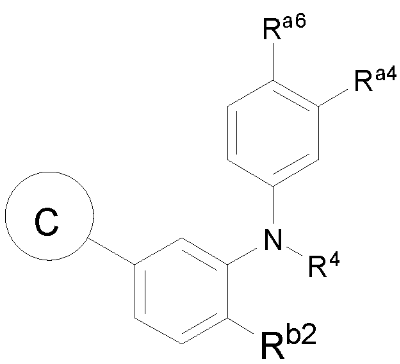

Formula III-B2

wherein ring C is selected from the group consisting of

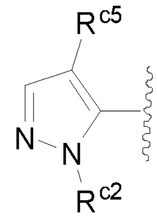

and

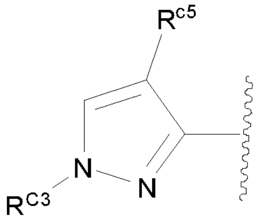
;

each of $R^{c2}$, $R^{c3}$, and $R^{c5}$ is independently selected from the group consisting of H, and $C_1$-$C_3$ alkyl or a deuterated or halogenated compound thereof, and preferably selected from the group consisting of H and methyl;
$R^4$ is selected from the group consisting of H, deuterated or undeuterated $C_1$-$C_2$ alkyl, and $-L_2$-OH; $L_2$ is 0-5 methylene groups;
$R^{b2}$ is deuterated or undeuterated methyl;
each of $R^{a4}$ and $R^{a6}$ is independently selected from the group consisting of H, -CN, amino, nitro, halogen,

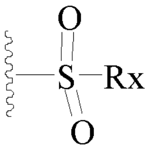

, -C(O)NHR$^{11}$, $C_1$-$C_3$ alkyl or a deuterated or halogenated or cyano-substituted compound thereof, $C_1$-$C_3$ alkoxy or a deuterated or halogenated or cyano-substituted compound thereof, substituted or unsubstituted 3-6-membered saturated cycloalkyl, and substituted or unsubstituted 5-membered unsaturated heterocyclic group, or $R^{a6}$ and the substituent at the ortho position of $R^{a6}$, together with the substituted atom to which they are linked, form a substituted or unsubstituted 5-membered unsaturated heterocyclic ring; wherein each of the substituents in said 5-membered unsaturated heterocyclic group and 5-membered unsaturated heterocyclic ring is independently selected from the group consisting of - CN, amino, nitro, halogen, $C_1$-$C_2$ alkyl or a deuterated or halogenated compound thereof, and $-L_1$-OH; $L_1$ is 0-2 methylene groups; the substituent in said 3-6 membered saturated cycloalkyl is -CN; each of $R_x$ and $R^{11}$ is independently selected from the group consisting of H and $C_1$-$C_2$ alkyl.

Further,

the structure of said compound is selected from the group consisting of:

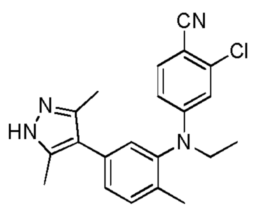

**1**

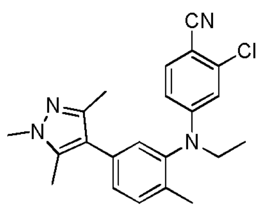

**2**

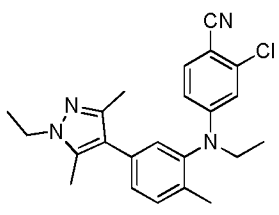

**3**

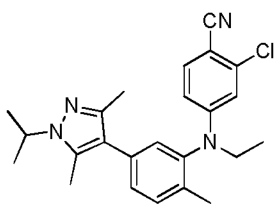

**4**

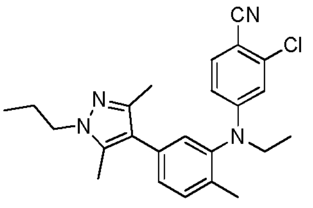

**5**

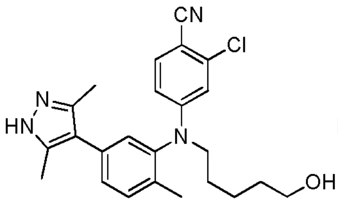

**6**

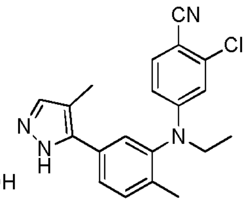

**7**

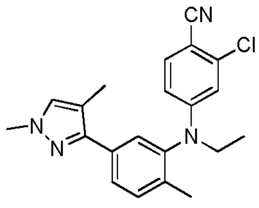

**8**

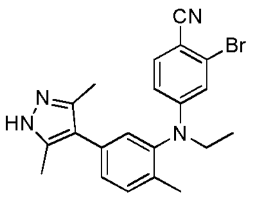

**9**

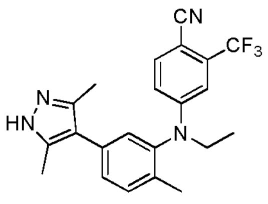

**10**

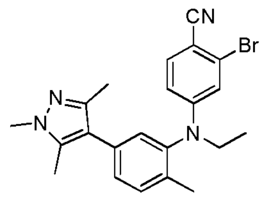

**11**

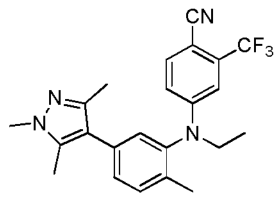

**12**

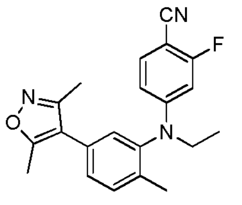

**13**

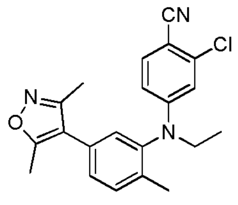

**14**

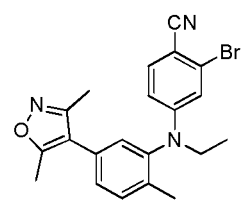

**15**

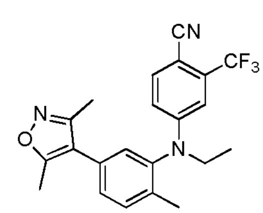

**16**

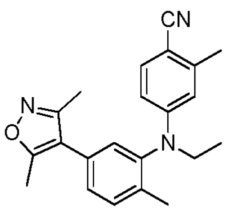

**17**

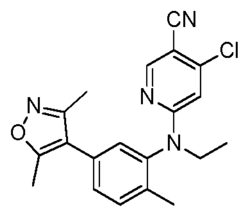

**18**

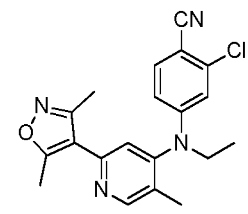

**19**

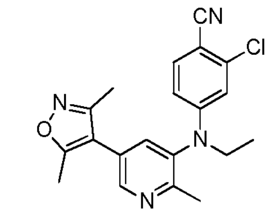

**20**

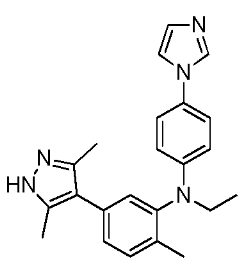

**21**

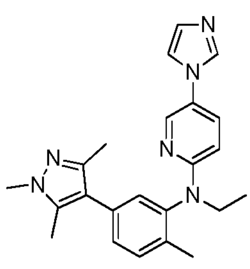

**22**

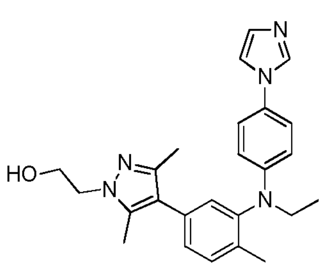

**23**

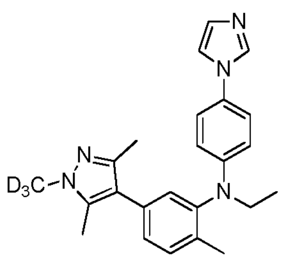

**24**

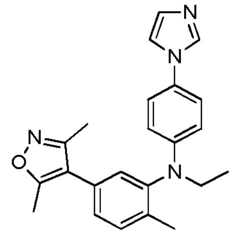

**25**

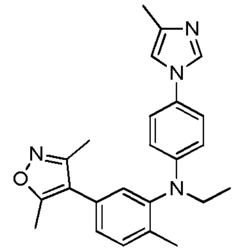

**26**

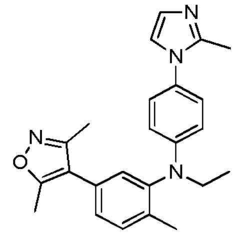

**27**

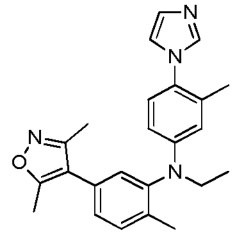

**28**

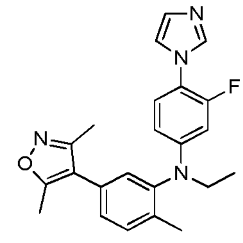

29

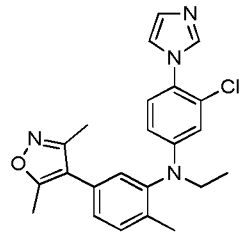

30

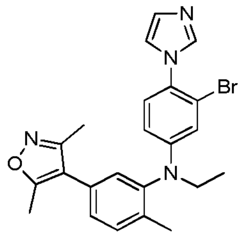

31

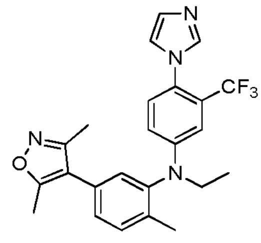

32

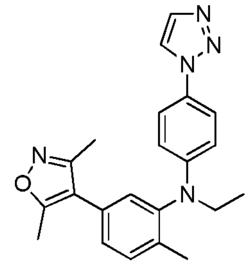

33

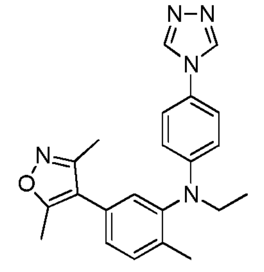

34

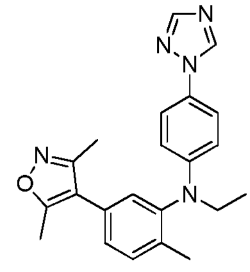

35

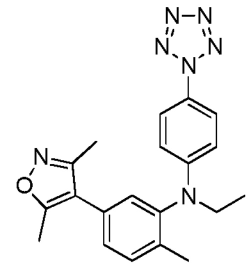

36

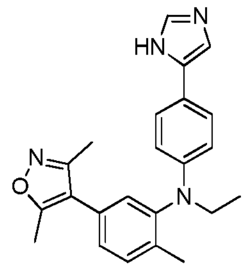

37

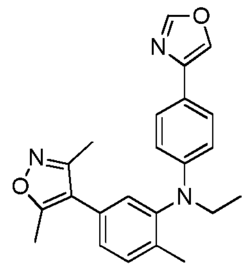

38

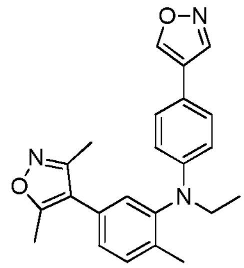

39

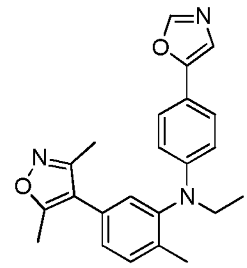

40

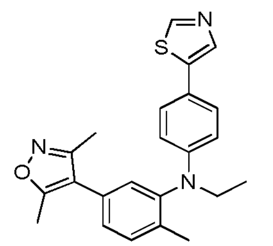

41

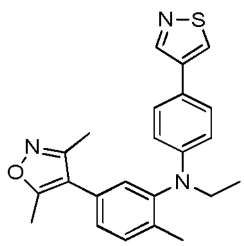

42

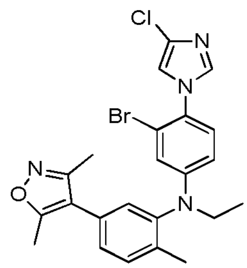

43

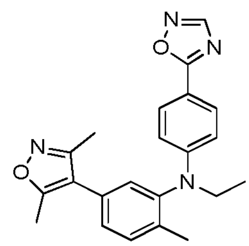

44

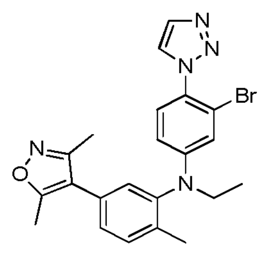

45

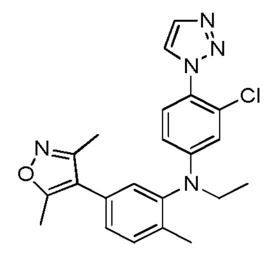

46

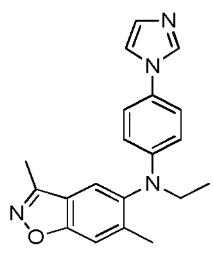

47

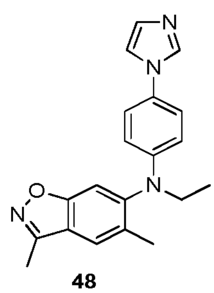

48

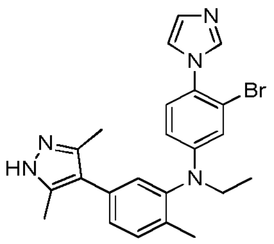

49

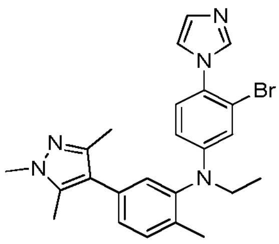

50

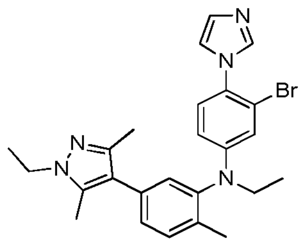

51

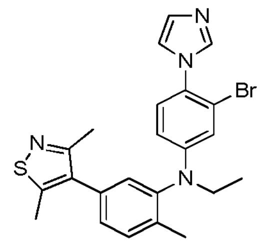

52

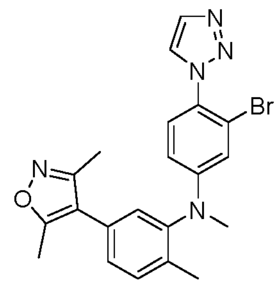

53

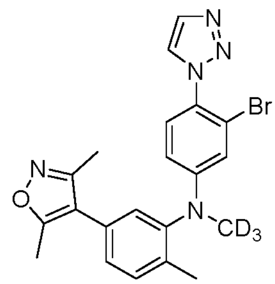

54

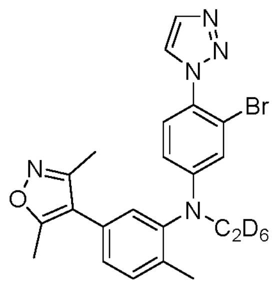

55

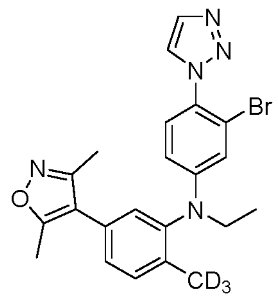

56

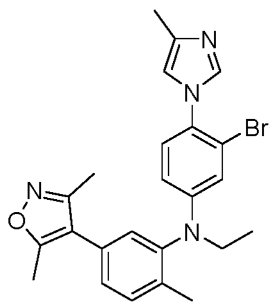

57

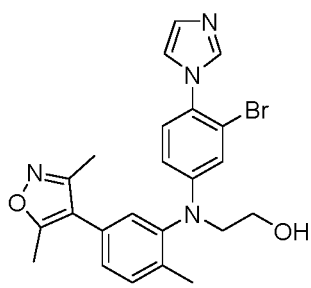

58

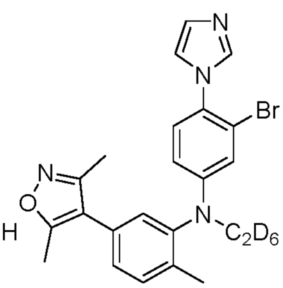

59

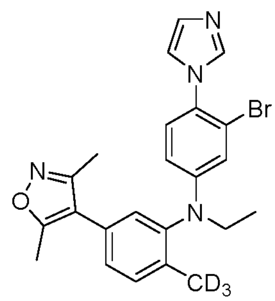

60

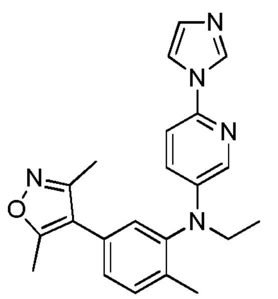

61

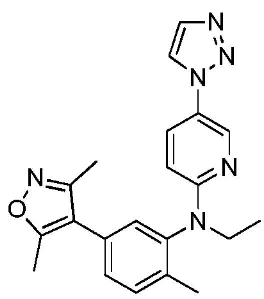

62

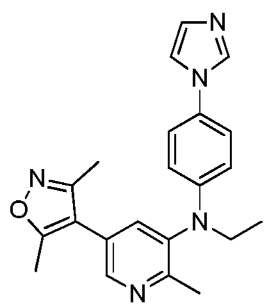

63

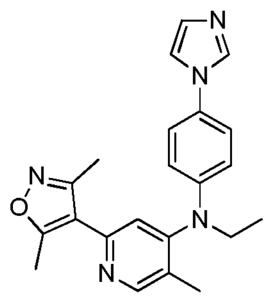

64

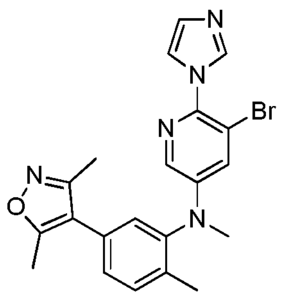

**65**

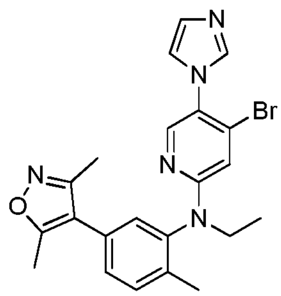

**66**

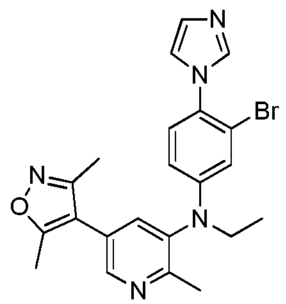

**67**

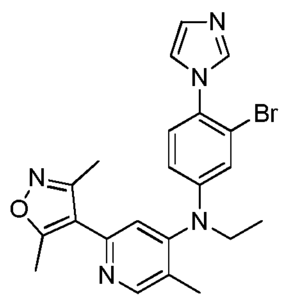

**68**

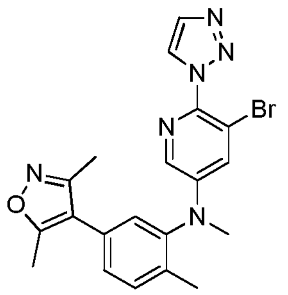

**69**

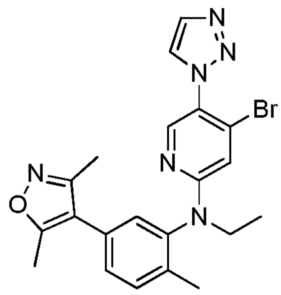

**70**

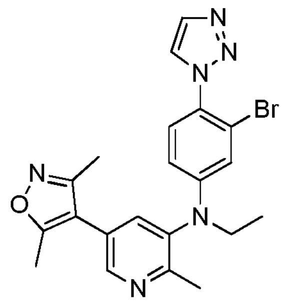

**71**

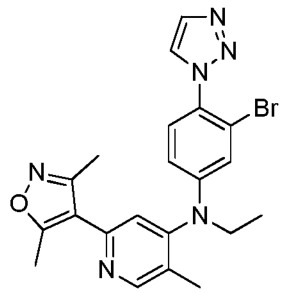

**72**

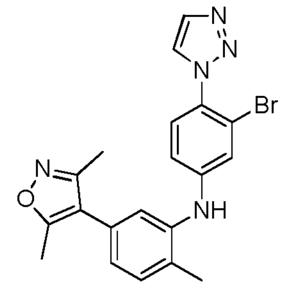

**73**

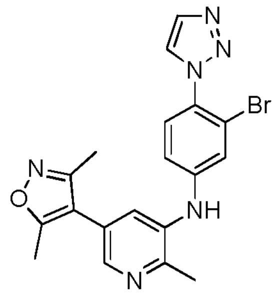

**74**

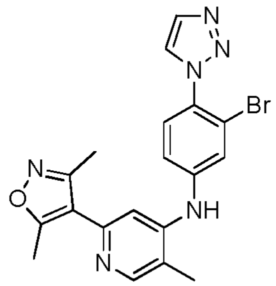

**75**

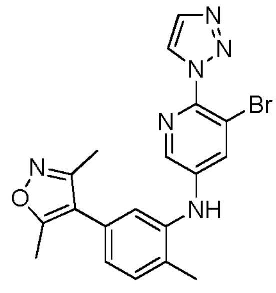

**76**

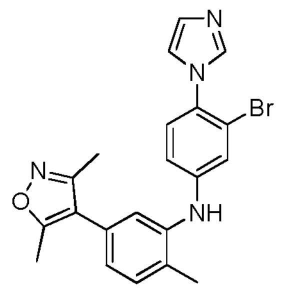

**77**

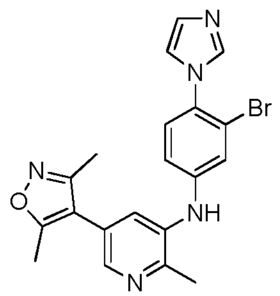

**78**

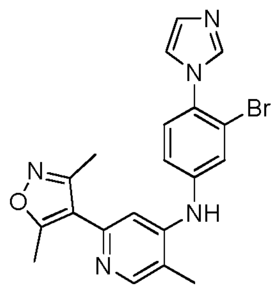

**79**

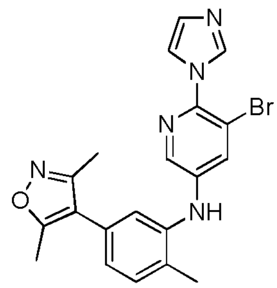

**80**

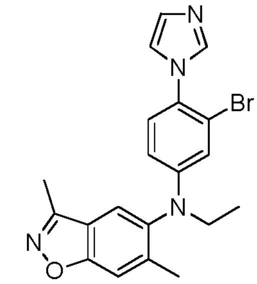
81

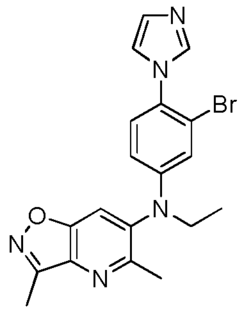
82

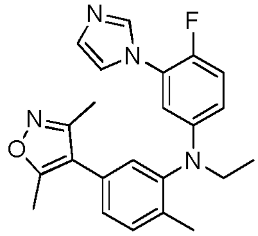
83

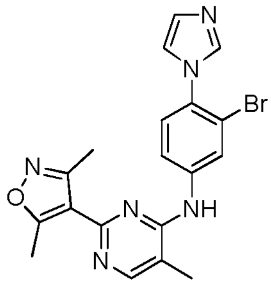
84

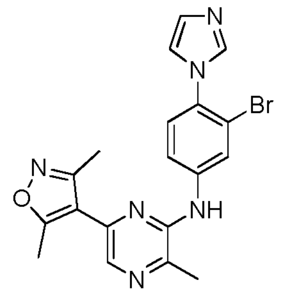
85

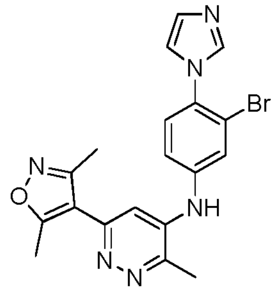
86

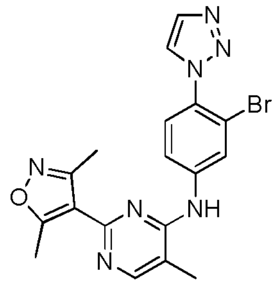
87

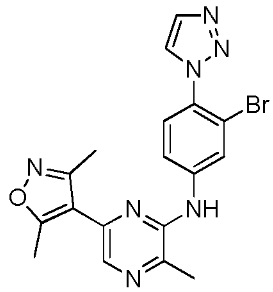
88

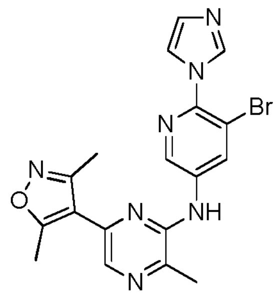
89

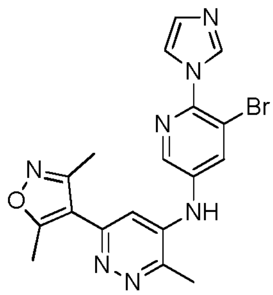
90

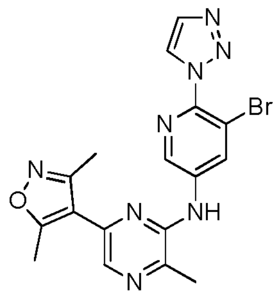
91

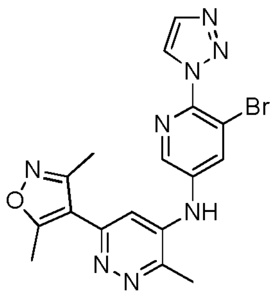
92

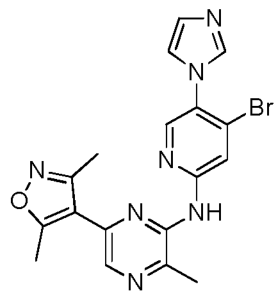
93

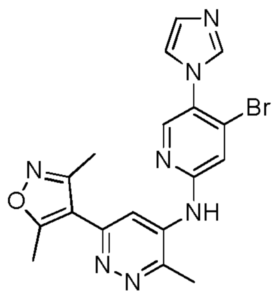
94

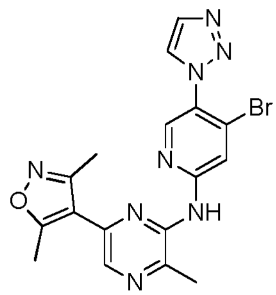
95

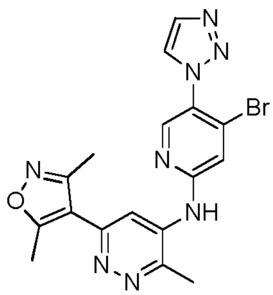
96

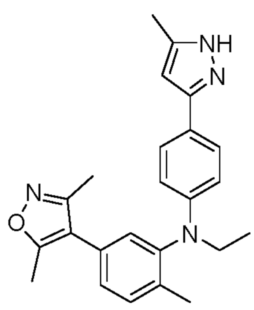

97

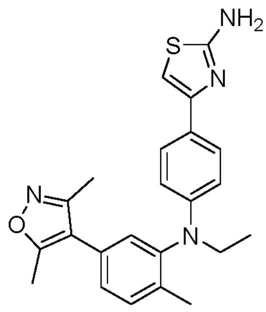

98

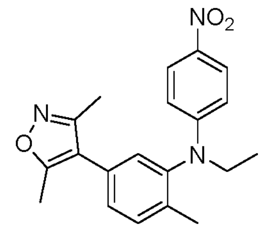

99

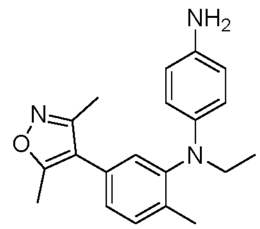

100

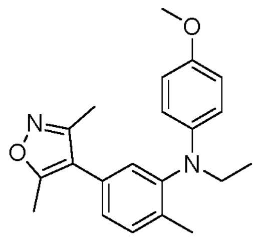

101

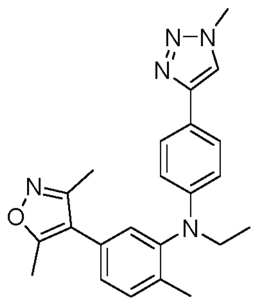

102

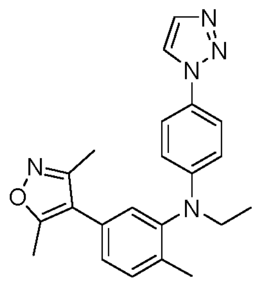

103

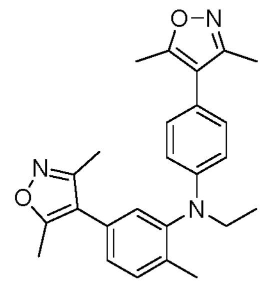

104

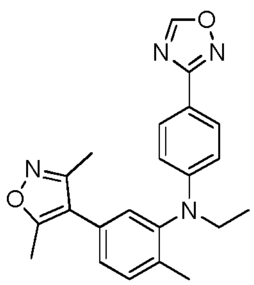

105

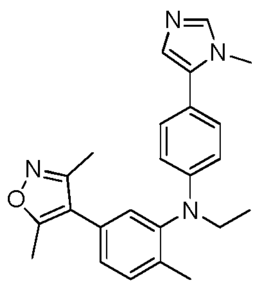

106

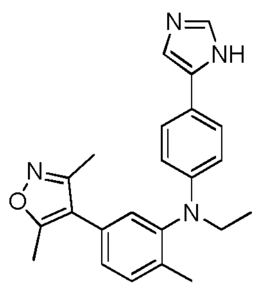

107

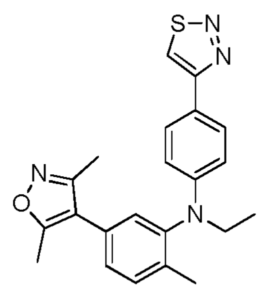

108

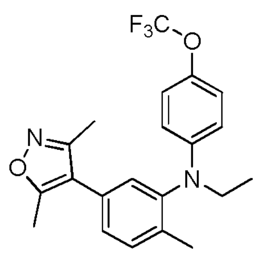

109

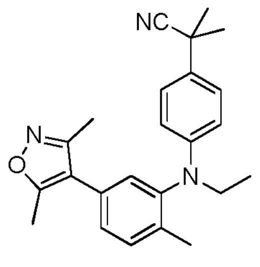

110

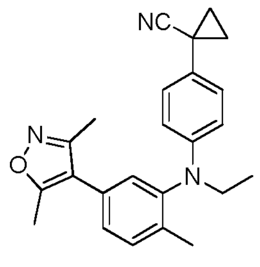

111

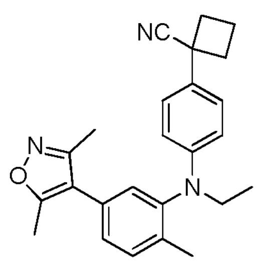

112

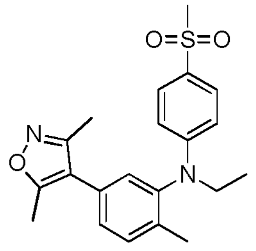

113

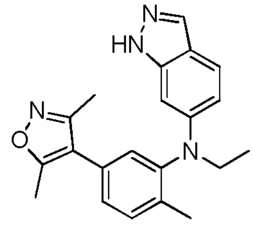

114

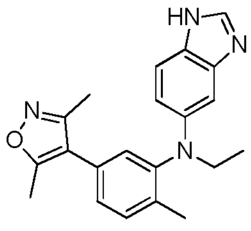

115

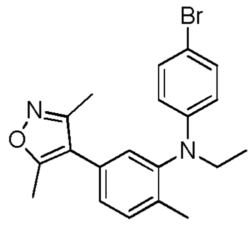

116

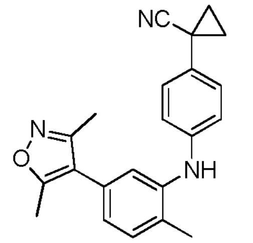

117

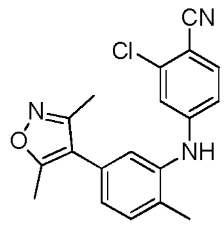

118

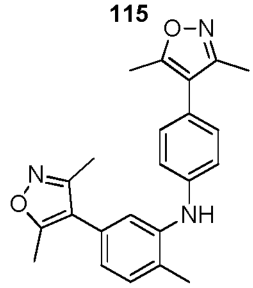

119

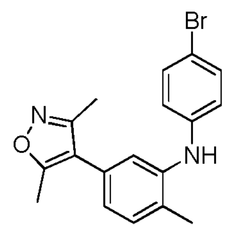

120

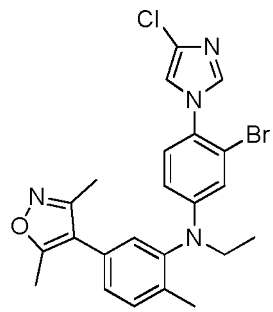

121

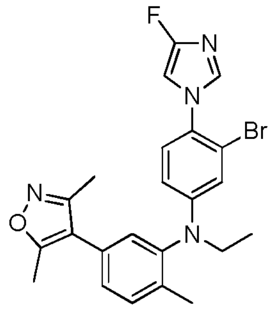

122

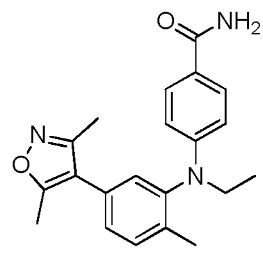

123

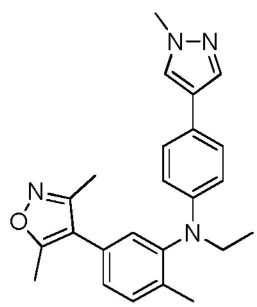

124

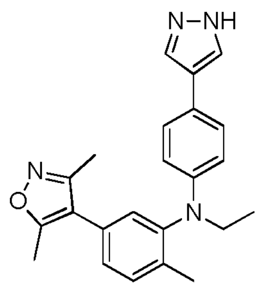

125

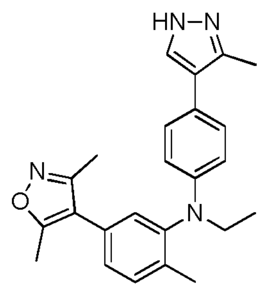

126

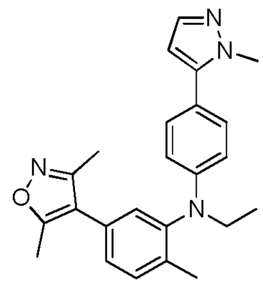

127

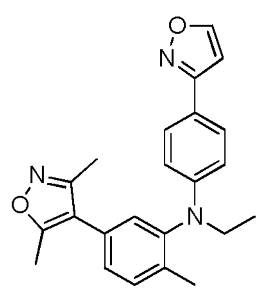

128

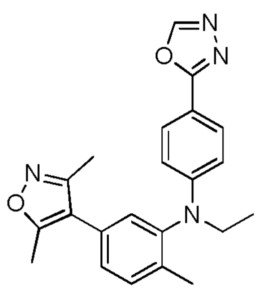

129

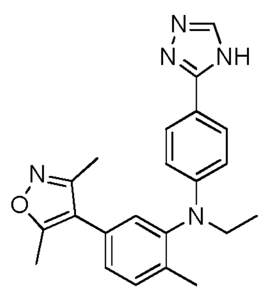

130

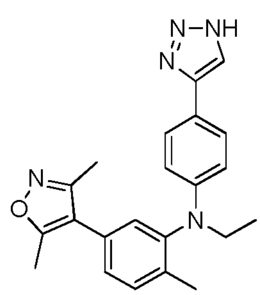

131

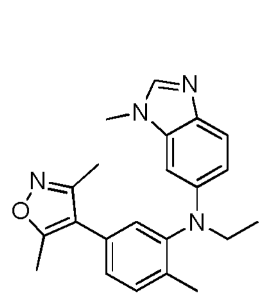

132

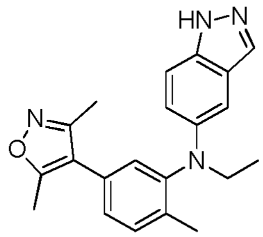

**133**

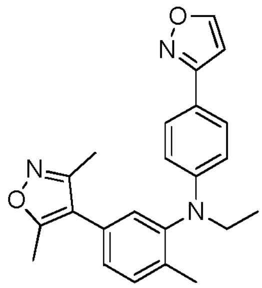

**134**

The experimental results show that the compound provided in the present invention has a dual inhibitory effect on AR and BRD4. The compound of the present invention can not only inhibit the proliferation of the prostate cancer cell line LNCaP/AR with overexpression of the androgen receptor AR, but also have good inhibitory effect on the prostate cancer cell lines VCaP and 22RV1 that are resistant to the marketed prostate cancer drug (enzalutamide). The compound of the present invention itself, as a compound that can recognize both AR and BRD4 dual targets, can be used as AR/BRD4 dual inhibitors, and can also be used to prepare proteolytic targeting chimeras (PROTACs) that induce the degradation of AR/BRD4 dual targets, as well as has a good application prospect in the preparation of drugs for the treatment of AR- and BRD4-related diseases.

In the present invention, "substitution" means that one, two or more hydrogens in a molecule are substituted by other different atoms or molecules, including one, two or more substitutions on the same or different atoms in the molecule.

In the present invention, $C_1$-$C_5$ alkyl means $C_1$, $C_2$, $C_3$, $C_4$, and $C_5$ alkyl, namely a straight or branched alkyl containing 1-5 carbon atoms, such as methyl, ethyl, propyl, butyl, isobutyl, t-butyl, sec-butyl, pentyl, and so on. Similarly, $C_1$-$C_3$ alkoxy means $C_1$, $C_2$, and $C_3$ alkoxy.

In the present invention, "pharmaceutically acceptable" denotes a certain carrier, vehicle, diluent, excipient, and/or formed salt is usually chemically or physically compatible with other ingredients constituting a certain pharmaceutical dosage form, as well as physiologically compatible with the recipient.

In the present invention, "salt" means acid and/or basic salt that is formed by reaction of compound or its stereoisomer with inorganic and/or organic acid and/or base, and also includes zwitterionic salts (inner salts), and further includes quaternary ammonium salts, such as alkylammonium salt. These salts can be directly obtained during the final isolation and purification of a compound. The salts can also be obtained by mixing the compound or its stereoisomers with a certain amount of acid or base appropriately (for example, in equivalent). These salts may form a precipitate in the solution, and be collected by filtration, or recovered after evaporation of the solvent, or obtained by freeze-drying after reaction in an aqueous medium. The salt in the present invention may be hydrochloride, sulfate, citrate, benzenesulfonate, hydrobromide, hydrofluoride, phosphate, acetate, propionate, succinate, oxalate, malate, succinate, fumarate, maleate, tartrate or trifluoroacetate.

In the present invention, "aromatic ring" denotes an all-carbon monocyclic or condensed polycyclic ring having a conjugated $\pi$-electron system, such as benzene and naphthalene. The aromatic ring can be fused to other cyclic structures (including saturated and unsaturated rings), but cannot contain heteroatoms such as nitrogen, oxygen or sulfur, and the point of connection to the parent must be on the carbon atom of the ring with a conjugated $\pi$-electron system; "monocyclic aromatic ring" denotes an all-carbon monocyclic ring with a conjugated $\pi$-electron system.

"Heteroaromatic ring" denotes a monocyclic or fused polycyclic ring having a conjugated $\pi$-electron system and containing one to more heteroatoms. It contains at least one ring heteroatom selected from the group consisting of N, O and S, and the rest of the ring atoms are C, and additionally have a fully conjugated $\pi$-electron system. For example, furan, pyrrole, quinoline, thiophene, pyridine, pyrazole, N-alkylpyrrole, pyrimidine, pyrazine, imidazole, tetrazole, thienopyridyl and the like. The heteroaromatic ring may be fused to an aromatic ring, a heterocyclic ring, or an alkane ring; a "monocyclic heteroaromatic ring" denotes a single ring containing one to more heteroatoms and having a conjugated $\pi$-electron system.

In the present invention, said "substituents on rings A, B, and C, as well as any two groups in $R^4$, together with the substituted atoms to which they are respectively linked, are connected to form a ring" means the substituents on rings A, B, and C, two optional substituents in $R^4$, together with the substituted atom to which they are respectively linked, are connected to form another ring, such as two substituents $R^{a4}$ and $R^{a6}$ in the structure

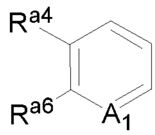,

together with the carbon atom to which the substituent is each linked, are connected to form the following structure

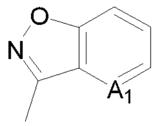.

In the present invention, the "substituent at the ortho position of $R^{a6}$" means a substituent on another atom adjacent to the atom substituted with $R^{a6}$, such as in the structure of

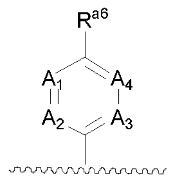

, the "substituent at the ortho position of $R^{a6}$" denotes the substituent at $A_1$ or $A_4$.

Obviously, based on above content of the present invention, according to the common technical knowledge and the conventional means in the field, without department from above basic technical spirits, other various modifications, alternations or changes can further be made.

By following specific examples of said embodiments, the above content of the present invention is further illustrated. But it should not be construed that the scope of above subject of the present invention is limited to following examples. The techniques realized based on above content of the present invention are all within the scope of the present invention.

Examples

The starting materials and equipments used in the present invention are all known products and can be obtained by purchasing commercially available products.

**Example 1 Synthesis of N-(3-bromo-4-(1H-imidazol-1-yl)phenyl)-5-(3,5-dimethylisoxazol-4-yl)-N-ethyl-2-methylaniline (compound 31)**

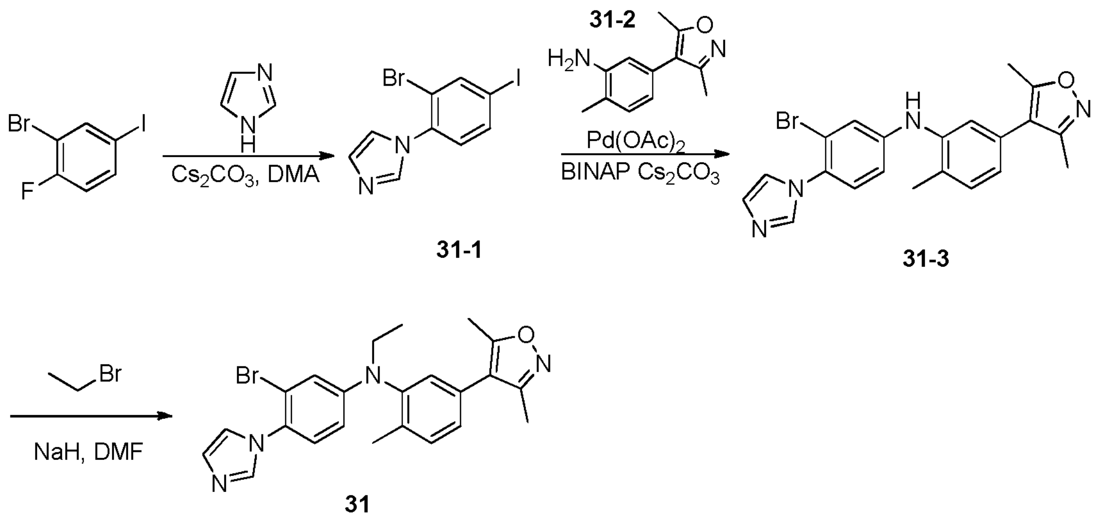

31-1 31-2 31-3 31

Step 1:

2-Bromo-1-fluoro-4-iodobenzene (3.0 g, 10 mmol), imidazole (680 mg, 10 mmol), and cesium carbonate (4.9 g, 15 mmol) were successively added to 50 mL of DMA, and under the protection of nitrogen, the reaction was heated to 120 °C overnight. After completion of the reaction by TLC detection, the reaction was cooled to room temperature naturally, extracted with 100 mL ethyl acetate, washed with saturated brine (3*50 mL), dried over anhydrous sodium sulfate, concentrated, and separated and purified by chromatographic column (PE/EA=4/1) to obtain 3.4 g of compound 1-(2-bromo-4-iodobenzene)-1H-imidazole, with a yield of 95%.

Step 2:

(1) Synthesis of the starting material 5-(3,5-dimethylisoxazol-4-yl)-2-methylaniline (compound **31-2**): (3,5-dimethylisoxazol-4-yl)boric acid (16.9 g, 120 mmol) and 5-bromo-2-methylaniline (22.3 g, 120 mmol) were dissolved in 1,4-dioxane (240 ml), to which were added 80 ml of water, potassium carbonate (41.5 g, 300 mmol), and tetrakis(triphenylphosphine) palladium (4.16 g, 3.6 mmol), and under argon protection, the mixture was allowed to react overnight at 85 °C. Then, the aqueous layer was separated. The organic layer was concentrated to dryness, washed with 300 ml of water, and extracted with 300 ml of ethyl acetate. The aqueous layer was re-extracted once, and then the organic layers were combined, washed with saturated brine, dried with anhydrous sodium sulfate, concentrated, and purified by chromatographic column, to obtain 13.5 g of compound 5-(3,5-dimethylisoxazol-4-yl)-2-methylaniline with a yield of 56%.

(2) 5-(3,5-Dimethylisoxazol-4-yl)-2-methylaniline (606 mg, 3 mmol) was dissolved in 20 ml of 1,4-dioxane, to which were added 1-(2-bromo-4-iodobenzene)-1H-imidazole (1.06 g, 3 mmol), cesium carbonate (2.45 g, 7.5 mmol), BINAP (93mg, 0.15 mmol), and palladium acetate (36 mg, 0.15 mmol), and under argon protection, the mixture was allowed to react at 110 °C overnight. The reaction solution was filtered, and the filtrate was washed with 50 ml of water, extracted with 50 ml of ethyl acetate, and then washed with saturated brine, dried with anhydrous sodium sulfate, concentrated, and purified by chromatographic column, to obtain 790 mg of compound N-(3-bromo-4-(1H-imidazol-1-yl)phenyl)-5-(3,5-dimethylisoxazol-4-yl)-2-methylaniline, with a yield of 62%.

Step 3:

60% Sodium hydride (40 mg, 1.0 mmol) was dissolved in 3 ml of dimethyl sulfoxide, to which was added N-(3-bromo-4-(1H-imidazol-1-yl)phenyl)-5- (3,5-dimethylisoxazol)-4-yl)-2-methylaniline (213 mg, 0.5 mmol), and the reaction was stirred at room temperature for 10 min, then bromoethane (273 mg, 2.5 mmol) was added. The mixture was allowed to react at room temperature for 1 h, washed with 10 ml of water, and extracted with 10 ml of ethyl acetate. The organic layer was concentrated under reduced pressure, and then separated and purified by thin layer chromatography, to provide 183 mg of compound N-(3-bromo-4-(1H-imidazol-1-yl)phenyl)-5-(3,5-dimethylisoxazol-4-yl)-N-ethyl-2-methylaniline, with a yield of 81%. $^1H$ NMR (400 MHz, $CDCl_3$) $\delta$7.66 (s, 1H), 7.43 (d, *J*= 7.8 Hz, 1H), 7.18 (dd, *J*= 7.7, 1.8 Hz, 2H), 7.10 - 7.04 (m, 2H), 7.03 (d, *J* = 1.7 Hz, 1H), 6.80 (d, *J* = 2.7 Hz, 1H), 6.45 (dd, *J* = 8.8, 2.7 Hz, 1H), 3.70 (q, *J* =

7.1 Hz, 2H), 2.43 (s, 3H), 2.27 (s, 3H), 2.21 (s, 3H), 1.27 (dt, *J*= 9.7, 7.1 Hz, 3H). LC/MS (ESI+) calcd for $C_{23}H_{23}FN_4O$ $[M+H]^+$) *m/z:* 451.1; found 451.1。

**Example 2 Synthesis of 5-(3,5-dimethylisoxazol-4-)-*N*-ethyl-2-methyl-*N*- (4-(5-methyl-1*H*-pyrazol-3-yl)phenyl)aniline (compound 97)**

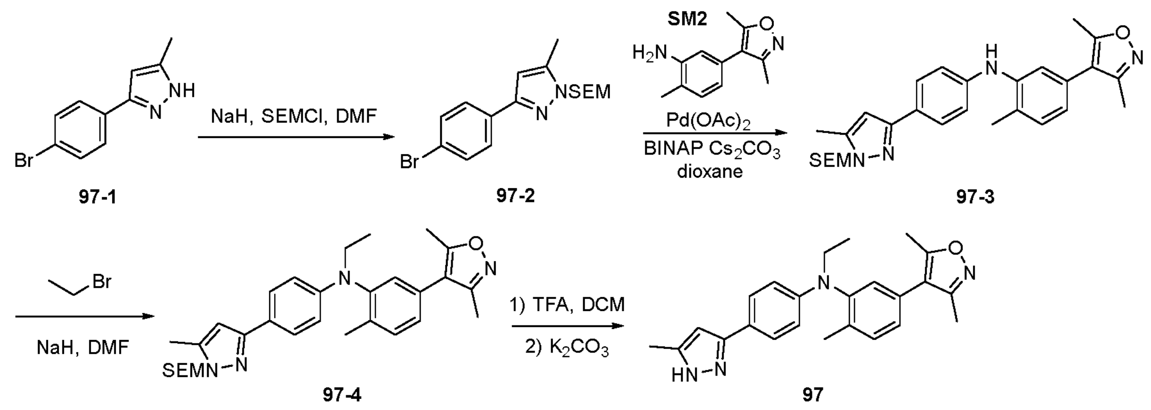


Step 1:

60% sodium hydride (80 mg, 2 mmol) was dissolved in 10 ml of *N,N*-dimethylformamide, to which was added 3-(4-bromophenyl)-5-methyl- 1H-pyrazole (236 mg, 1 mmol). The mixture was stirred at room temperature for 10 min, and then 2-(trimethylsilyl)ethoxymethyl chloride (182 mg, 1.1 mmol) was added. The reaction was allowed to react 3 h at room temperature, washed with 10 ml of water, and extracted with 10 ml of ethyl acetate. The organic layer was concentrated under reduced pressure, and then separated and purified by thin layer chromatography, to provide 341 mg of compound 3-(4-bromophenyl)-5-methyl-1-((2-(trimethylsilyl) ethoxy)methyl)-1*H*-pyrazole, with a yield of 93%.

Step 2:

5-(3,5-Dimethylisoxazol-4-yl)-2-methylaniline (202 mg, 1 mmol) was dissolved in 5 ml 1,4-dioxane, to which were added 3-(4-bromophenyl)-5-methyl-1-((2-(trimethylsilyl)ethoxy)methyl)-1*H*-pyrazole (341 mg, 0.93 mmol), cesium carbonate (650 mg, 2 mmol), BINAP (62 mg, 0.1 mmol), palladium acetate (11 mg, 0.05 mmol). Under argon protection, the mixture was allowed to react overnight at 110 °C. The reaction solution was filtered. The filtrate was washed with 10 ml of water, extracted with 10 ml of ethyl acetate, and then washed with saturated brine, dried with anhydrous sodium sulfate, concentrated, and purified by chromatographic column to obtain 308 mg of 5-(3,5-dimethylisoxazol-4-)-2-methyl-*N*-(4-(5-methyl-1-((2-(trimethylsilyl)ethoxy)methyl)-1*H*-pyrazol-3-yl)phenyl)aniline, with a yield of 68%.

Step 3:

60% sodium hydride (8 mg, 0.2 mmol) was dissolved in 3 ml of *N,N*-dimethylformamide, to which was added 5-(3,5-dimethylisoxazol-4-)-2-methyl- *N*-(4-(5-methyl-1-((2-(trimethylsilyl)ethoxy)methyl)-1*H*-pyrazol-3-yl)phenyl)aniline (49 mg, 0.1 mmol), and the reaction was stirred at room temperature for 10 min, then bromoethane (22 mg, 0.2 mmol) was added. The mixture was allowed to react at room temperature for 1 h, washed with 5 ml of water, and extracted with 5 ml of ethyl acetate. The organic layer was concentrated under reduced pressure, and then separated and purified by thin layer chromatography, to provide 43 mg of compound 5-(3,5-dimethylisoxazol-4-)-*N*-ethyl-2-methyl-*N*-(4-(5-methyl-1-((2-(trimethylsilyl)ethoxy)methyl)-1*H*-pyrazol-3-yl)phenyl)aniline, with a yield of 84%.

Step 4:

Compound 5-(3,5-dimethylisoxazol-4-)-*N*-ethyl-2-methyl-*N*-(4-(5-methyl-1- ((2-(trimethylsilyl)ethoxy)methyl)-1*H*-pyrazol-3-yl)phenyl)aniline (43 mg, 0.084 mmol) was dissolved in 2 ml dichloromethane, to which was add 2 ml of trifluoroacetic acid. The mixture was stirred at room temperature for 1 h. Dichloromethane and trifluoroacetic acid in the reaction system were removed by concentration under reduced pressure, and then 5 ml of dichloromethane was added, followed by addition of potassium carbonate (28 mg, 0.2 mmol). The mixture was stirred at room temperature for half

an hour. The reaction solution was filtered, concentrated under reduced pressure, and then separated and purified by thin layer chromatography to obtain 28 mg of compound 5-(3,5-dimethylisoxazol-4-)-*N*-ethyl- 2-methyl-*N*-(4-(5-methyl-1*H*-pyrazol-3-yl)phenyl)aniline, with a yield of 88%. $^1H$ NMR (400 MHz, $CDCl_3$) $\delta$ 7.54 (m, 2H), 7.38 (dd, *J*= 8.0, 2.4 Hz, 1H), 7.12 (m, 1H), 7.04 (d, *J*= 2.0 Hz, 1H), 6.56 (dd, *J* = 8.4, 2.0 Hz, 1H), 6.24 (d, *J* = 7.2 Hz, 1H), 3.71 (q, *J* =7.2 Hz, 2H), 2.40 (s, 3H), 2.32 (s, 3H), 2.27 (s, 3H), 2.15 (s, 3H), 1.26 (td, *J* = 7.2, 2.0 Hz, 3H). LC/MS (ESI+) calcd for $C_{24}H_{27}N_4O$ ($[M+H]^+$) *m/z:* 387.2; found 387.20◦

**Example 3 Synthesis of 4-(4-((5-(3,5-dimethylisoxazol-4-)-2-methylphenyl)(ethyl)amino)phenyl)thiazol-2-amine (compound 98)**

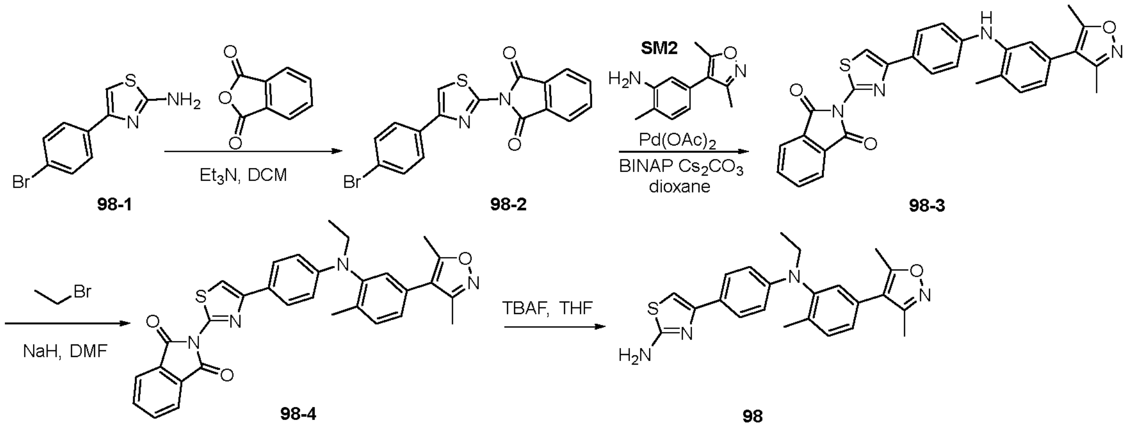


Step 1:

4-(4-Bromophenyl)thiazol-2-amine (254 mg, 1 mmol) was dissolved in 10 ml of dichloromethane, to which were added phthalic anhydride (140 mg, 0.96 mmol) and triethylamine (202 mg, 2 mmo), and then the mixture was stirred under reflux for 6 h. The reaction solution was washed with 10 ml of 1 *N* hydrochloric acid, and extracted with 10 ml of ethyl acetate, then washed with 10 ml saturated sodium bicarbonate and 10 ml saturated brine, respectively. The organic layer was concentrated under reduced pressure, and then separated and purified by thin-layer chromatography to obtain 360 mg of compound 2-(4-(4-bromophenyl)thiazol-2-yl)isoindolin-1,3-dione, with a yield of 94%.

Step 2:

5-(3,5-Dimethylisoxazol-4-)-2-methylaniline (202 mg, 1 mmol) was dissolved in 5 ml of 1,4-dioxane, to which were added 2-(4-(4-bromophenyl)thiazol-2-yl)isoindolin- 1,3-dione (360 mg, 0.94 mmol), cesium carbonate (650 mg, 2 mmol), BINAP (62 mg, 0.1 mmol), and palladium acetate (11 mg, 0.05 mmol). Under argon protection, the mixture was allowed to react overnight at 110 °C. The reaction solution was filtered. The filtrate was washed with 10 ml of water, extracted with 10 ml of ethyl acetate, and then washed with saturated brine, dried with anhydrous sodium sulfate, concentrated, and purified by chromatographic column to obtain 261 mg of 2-(4-(4-((5-(3,5-dimethylisoxazol-4-)-2-methylphenyl)amino)phenyl)thiazol-2-yl) isoindolin-1,3-dione, with a yield of 55%.

Step 3:

60% sodium hydride (8 mg, 0.2 mmol) was dissolved in 3 ml *N*,*N*-dimethylformamide, to which was added 2-(4-(4-((5-(3,5-dimethylisoxazol- 4-)-2-methylphenyl)amino)phenyl)thiazol-2-yl)isoindolin-1,3-dione (50 mg, 0.1 mmol), and the reaction was stirred at room temperature for 10 min, then bromoethane (22 mg, 0.2 mmol) was added. The mixture was allowed to react at room temperature for 1 h, washed with 5 ml of water, and extracted with 5 ml of ethyl acetate. The organic layer was concentrated under reduced pressure, and then separated and purified by thin layer chromatography, to provide 41 mg of compound 2-(4-(4-((5-(3,5-dimethylisoxazol-4-)-2-methylphenyl)(ethyl)amino)phenyl)thiazol-2-yl)isoindolin-1,3-dione, with a yield of 77%.

Step 4:

Compound 2-(4-(4-((5-(3,5-dimethylisoxazol-4-)-2-methylphenyl)(ethyl)amino) phenyl)thiazol-2-yl)isoindolin-1,3-dione (41 mg, 0.077 mmol) was dissolved in 2 ml of tetrahydrofuran, to which was added 39 mg of tetrabutylammonium fluoride (41 mg, 1.5 mmol). The mixture was stirred at room temperature for 2 h. Tetrahydrofuran in the reaction system was removed by concentration under reduced pressure, and then purified by thin-layer chromatography, to provide 25 mg of compound 4-(4-((5-(3,5-dimethylisoxazol-4-)-2-methylphenyl)(ethyl)amino)phenyl)thiazol-2-amine, with a yield of 80%. $^1$H NMR (400 MHz, $CDCl_3$) $\delta$ 7.69 (m, 2H), 7.20 (dd, *J* = 7.6, 2.0 Hz, 1H), 7.11 (m, 1H), 6.96 (m, 2H), 6.81 (m, 2H), 3.70 (q, *J* =7.2 Hz, 2H), 2.32 (s, 3H), 2.29 (s, 3H), 2.25 (s, 3H), 1.26 (t, *J* = 6.4 Hz, 3H). LC/MS (ESI+) calcd for $C_{23}H_{25}N_4OS$( $[M+H]^+$) *m/z:* 405.2; found 405.2.

**Example 4 Synthesis of *N*-(4-(1,2,3-thiazol-4-yl)phenyl)-5-(3,5-dimethylisoxazol-4-yl)-*N*-ethyl- 2-methylaniline (compound 108)**

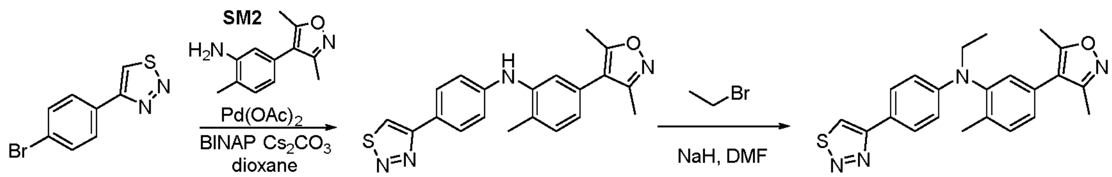


108-1 108-3 108

Step 1:

5-(3,5-Dimethylisoxazol-4-yl)-2-methylaniline (202 mg, 1 mmol) was dissolved in 5 ml of 1,4-dioxane, to which were added 4-(4-bromophenyl)-1,2,3-thiazole, cesium carbonate (650 mg, 2 mmol), BINAP (62 mg, 0.1 mmol), and palladium acetate (11 mg, 0.05 mmol). Under argon protection, the mixture was allowed to react overnight at 110 °C. The reaction solution was filtered. The filtrate was washed with 10 ml of water, extracted with 10 ml of ethyl acetate, and then washed with saturated brine, dried with anhydrous sodium sulfate, concentrated, and purified by chromatographic column to obtain 260 mg of compound *N*-(4-(1,2,3-thiazol-4-yl)phenyl)-5-(3,5-dimethylisoxazol-4-yl)-2-methylaniline, with a yield of 72%.

Step 2:

60% sodium hydride (8 mg, 0.2 mmol) was dissolved in 3 ml of *N,N*-dimethylformamide, to which was added *N*-(4-(1,2,3thiazole-4-yl)phenyl)-5- (3,5-dimethylisoxazol-4-yl)-2-methylaniline (36 mg, 0.1 mmol), and the reaction was stirred at room temperature for 10 min, then bromoethane (22 mg, 0.2 mmol) was added. The mixture was allowed to react at room temperature for 1 h, washed with 5 ml of water, and extracted with 5 ml of ethyl acetate. The organic layer was concentrated under reduced pressure, and then separated and purified by thin layer chromatography, to provide 30 mg of compound *N*-(4-(1,2,3-thiazol-4-yl)phenyl)-5-(3,5-dimethylisoxazol-4-yl)-*N*-ethyl-2-methylaniline, with a yield of 77%. $^1$H NMR (400 MHz, $CDCl_3$) $\delta$ 8.19 (d, *J* = 1.6 Hz, 1H), 7.73 (d, *J* = 8.8 Hz, 2H), 7.37 (d, *J* = 8.0 Hz, 1H), 7.23 (dd, *J* = 7.6, 2.0 Hz, 1H), 6.99 (d, *J*= 1.6 Hz, 1H), 6.57 (m, 2H), 3.63 (q, *J*= 6.8 Hz, 2H), 2.48 (s, 3H), 2.25 (s, 3H), 2.17 (s, 3H), 1.24 (t, *J* = 6.8 Hz, 3H). LC/MS (ESI+) calcd for $C_{22}H_{23}N_4OS$ ( $[M+H]^+$) *m/z:* 391.2; found 391.2.

**Example 5 Synthesis of N-1-(5-(3,5-dimethylisoxazol-4-yl)-2-methylphenyl)- N1-ethylbenzene-1,4-diamine (100)**

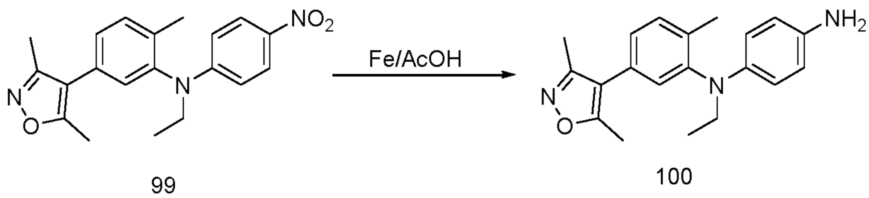


99 100

5-(3,5-Dimethylisoxazol-4-yl)-N-ethyl-2-methyl-N-(4-nitrophenyl)aniline (30 mg, 0.085 mmol) was dissolved in 3 ml of acetic acid, to which was added iron powder (15 mg, 0.27 mmol), and the mixture reacted at 65 °C for 4 h. The reaction solution was cooled to room temperature, and filtered. The filtrate was concentrated to dryness under reduced pressure, and then 5ml of ethyl acetate was added. The resultant solution was washed once with saturated aqueous solution of sodium carbonate, and then once with saturated brine, dried with anhydrous sodium sulfate, concentrated to dryness under reduced pressure, and separated by thin-layer chromatography to obtain the product N1-(5-(3,5-dimethylisoxazol-4-yl)-2-methylphenyl)-N1-ethylbenzene- 1,4-diamine (8 mg), with a yield of 29.3%. MS (ESI) *m/z* 322.1 $[M+H]^+$.

**Example 6 Synthesis of 5-(3,5-dimethylisoxazol-4-yl)-*N*-ethyl-2-methyl-*N*- (4-(1-methyl-1*H*-1,2,3-triazol-4-yl)phenyl)aniline (102)**

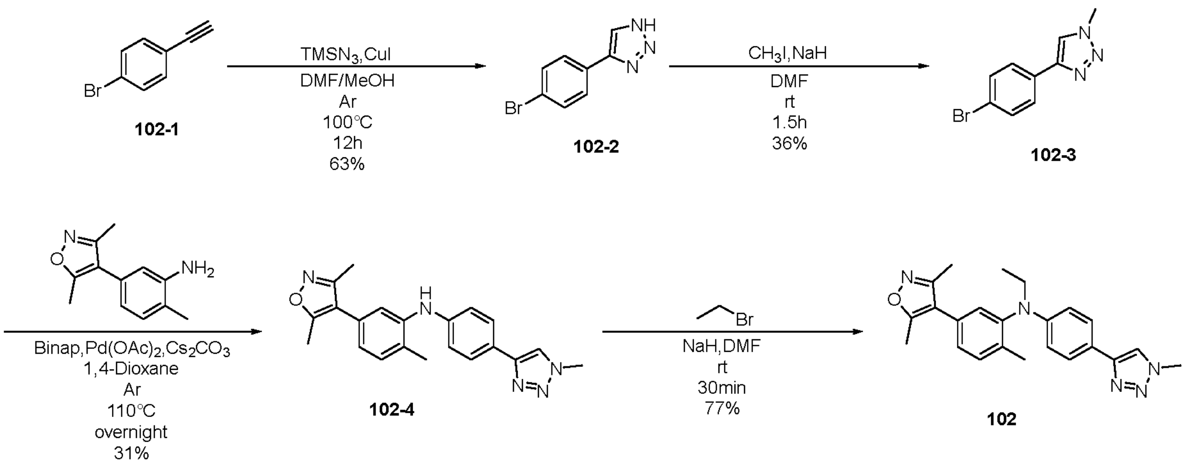


Step 1: Synthesis of compound 4-(4-bromophenyl)-1*H*-1,2,3-triazole

p-Bromophenyl acetylene (815 mg, 4.50 mmol) and cuprous iodide (44 mg, 0.23 mmol) were weighed and placed in a seal tube, to which was add 9 mL of a mixed solution of DMF and methanol (9:1), and the mixture was stirred well at room temperature. Then, trimethylsilyl azide (778 mg, 6.75 mmol) was added to the system. After that, the system was evacuated, and then argon was purged, that was repeated 5 times to ensure an inert gas atmosphere in the system. Then, the system was transferred to an oil bath at 100 °C, and the reaction was stirred under heating. After 12 h, the reaction was completed by TLC detection. After cooling to room temperature, the system was transferred to a round-bottom flask containing ethyl acetate (30 mL) and water (15 mL), stirred vigorously, and then allowed to stand for separation of layers. The aqueous phase was extracted with ethyl acetate (10 mL*3), and the organic phases were combined, successively washed with water (10 mL*3) and saturated brine (15 mL), dried over anhydrous sodium sulfate. The solvent was removed by rotary evaporation to obtain the crude product, which was separated and purified by column chromatography, to provide the compound 4-(4-bromophenyl)-1*H*-1,2,3-triazole (631 mg), with a yield of 63%. LC/MS (ESI+) calcd for $C_8H_6BrN_3^+$ $(M + H^+)$ m/z, 224.1; found, 224.0.

Step 2: Synthesis of compound 4-(4-bromophenyl)-1-methyl-1*H*-1,2,3-triazole

4-(4-Bromophenyl)-1*H*-1,2,3-triazole (224 mg, 1.00 mmol) was weighed and placed in a 25 mL single-neck round-bottom flask, to which was added DMF (4 mL), and then the mixture was stirred at room temperature to dissolve and obtain a clear solution. Then, the system was moved in an ice water bath to cool down under stirring. When the internal temperature of the system was reduced to about 0 °C, sodium hydride (60 mg, 1.50 mmol) was added to the system. After that, the system was allowed to react under the conditions of insulation and stirring. After 15 min, the solution of methyl iodide (170 mg, 1.20 mmol) in DMF (1 mL) was added dropwise to the system, and after addition, the ice-water bath was removed. The system was allowed to react at room temperature. After 1.5 h, TLC showed that the raw materials were almost completely consumed, and the reaction was stopped. Ethyl acetate (15 mL) and water (15 mL) were added to the system, stirred vigorously, and then stood for separation of layers. The aqueous phase was extracted with ethyl acetate (10 mL*3), and the organic phases were combined, successively washed with water (10 mL*3) and saturated brine (15 mL), dried over anhydrous sodium sulfate. The solvent was removed by rotary evaporation

to obtain the crude product, which was separated and purified by Pre-TLC, to provide compound 4-(4-bromophenyl)-1-methyl-1*H*-1,2,3-triazole (86 mg), with a yield of 36%.

LC/MS (ESI+) calcd for $C_9H_8BrN_3^+$ (M + $H^+$) m/z, 238.1; found, 238.1.

Step 3: Synthesis of compound 5-(3,5-dimethylisoxazol-4-yl)-2-methyl-*N*- (4-(1-methyl-1*H*-1,2,3-triazol-4-yl)phenyl)aniline

5-(3,5-Dimethylisoxazol-4-yl)-2-methylaniline (73 mg, 0.36 mmol), 4-(4-bromophenyl)-1-methyl-1*H*-1,2,3-triazole (86 mg, 0.36 mmol), Binap (12 mg, 0.02 mmol), palladium acetate (5 mg, 0.02 mmol), and cesium carbonate (293 mg, 0.90 mmol) were successively weighed and placed in a 25 mL single-neck round-bottom flask, to which was added 5 mL of dioxane. After that, the system was evacuated, and then argon was purged, that was repeated 5 times to ensure an inert gas atmosphere in the system. Then, the system was transferred to an oil bath at 110 °C, and the reaction was stirred under heating. Next day, the reaction was completed by TLC detection. Heating was stopped, and the system was cooled to room temperature, to which were added ethyl acetate (30 mL) and water (15 mL), then stirred vigorously, followed by standing for separation of layers. The aqueous phase was extracted with ethyl acetate (10 mL*3), and the organic phases were combined, successively washed with water (10 mL*3) and saturated brine (15 mL), dried over anhydrous sodium sulfate. The solvent was removed by rotary evaporation to obtain the crude product, which was separated and purified by column chromatography, to provide compound 5-(3,5-dimethylisoxazol-4-yl)-2-methyl-*N*-(4-(1-methyl-1*H*-1,2,3-triazol-4-yl)phenyl)aniline (40 mg), with a yield of 31%.

LC/MS (ESI+) calcd for $C_{21}H_{21}N_5O^+$ (M + H+) m/z, 359.4; found, 360.2.

Step 4: Synthesis of compound 5-(3,5-dimethylisoxazol-4-yl)-*N*-ethyl-2-methyl-*N*-(4-(1-methyl-1*H*-1,2,3-triazol-4-yl)phenyl)aniline

5-(3,5-Dimethylisoxazol-4-yl)-2-methyl-*N*-(4-(1-methyl-1*H*-1,2,3-triazol-4-yl)phenyl)aniline (40 mg, 0.11 mmol) was weighed and placed in a 25 mL single-neck round-bottom flask, to which was added DMF (4 mL), and then the mixture was stirred at room temperature to dissolve and obtain a clear solution. Then, the system was moved in an ice-water bath to cool down under stirring. When the internal temperature of the system was reduced to about 0 °C, sodium hydride (7 mg, 0.17 mmol) was added to the system. After that, the system was allowed to react under the conditions of insulation and stirring. After 15 min, the solution of ethyl iodide (14 mg, 0.13 mmol) in DMF (1 mL) was added dropwise to the system, and after addition, the ice-water bath was removed. The system was allowed to react at room temperature. After 30 min, TLC showed that the raw materials were almost completely consumed, and the reaction was stopped. Ethyl acetate (15 mL) and water (15 mL) were added to the system, and the system was stirred vigorously, then stood for separation of layers. The aqueous phase was extracted with ethyl acetate (10 mL*3), and the organic phases were combined, successively washed with water (10 mL*3) and saturated brine (15 mL), dried over anhydrous sodium sulfate. The solvent was removed by rotary evaporation to obtain the crude product, which was separated and purified by Pre-TLC, to provide compound 5-(3,5-dimethylisoxazol-4-yl)-*N*-ethyl-2-methyl-*N*-(4-(1-methyl-1*H*-1,2,3-triazol- 4-yl)phenyl)aniline (33 mg), with a yield of 77%.

LC/MS (ESI+) calcd for $C_{23}H_{25}N_5O^+$ (M + H+) m/z, 387.5; found, 388.2.

$^1$H NMR (400 MHz, $CDCl_3$) $\delta$ 7.66 (d, *J* = 8.7 Hz, 2H), 7.39 (d, *J*= 7.8 Hz, 1H), 7.13 (dd, *J* = 7.8, 1.7 Hz, 1H), 7.05 (d, *J* = 1.7 Hz, 1H), 6.59 (d, *J* = 8.5 Hz, 2H), 4.14 (s, 3H), 3.72 (q, *J* = 7.1 Hz, 2H), 2.41 (s, 3H), 2.28 (s, 3H), 2.18 (s, 3H), 1.27 (t, *J* = 7.1 Hz, 3H).

**Example 7 Synthesis of *N*-(4-(1,2,4-oxadiazole-3-yl)phenyl)-5-(3,5-dimethylisoxazol-4-yl)-*N*-ethyl-2-methylaniline (105)**

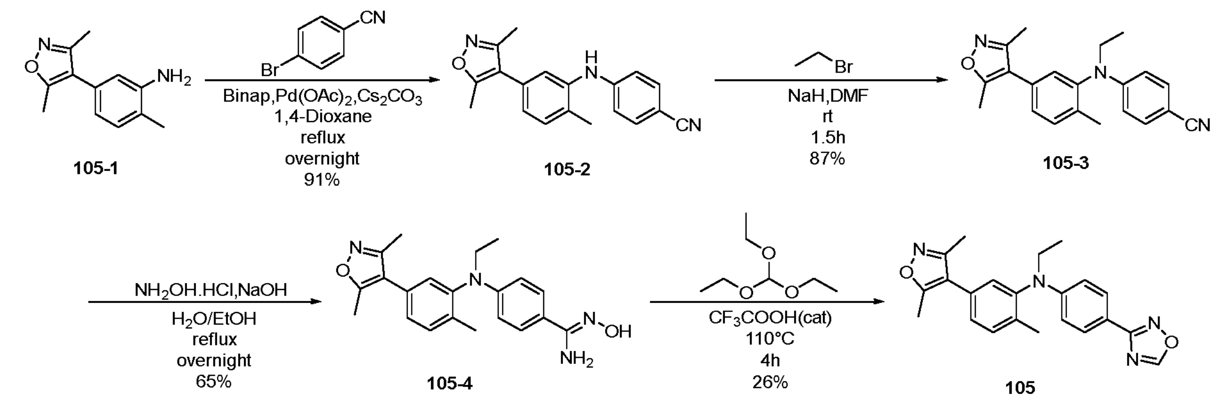


105-1 105-2 105-3 105-4 105

Step 1: Synthesis of compound 4-((5-(3,5-dimethyl-2,3-dihydroisoxazol-4-yl)-2-methylphenyl)amino)benzonitrile

5-(3,5-Dimethylisoxazol-4-yl)-2-methylaniline (500 mg, 2.47 mmol), p-bromobenzonitrile (450 mg, 2.47 mmol), Binap (75 mg, 0.12 mmol), palladium acetate (27 mg, 0.12 mmol), and cesium carbonate (2.01 g, 6.18 mmol) were successively weighed and placed in a 50 mL single-neck round-bottom flask, to which was added 15 mL of dioxane. After that, the system was evacuated, and then argon was purged, that was repeated 5 times to ensure an inert gas atmosphere in the system. Then, the system was transferred to an oil bath at 110 °C, and the reaction was stirred under heating. Next day, the reaction was completed by TLC detection. Heating was stopped, and the system was cooled to room temperature, to which were added ethyl acetate (30 mL) and water (15 mL), then stirred vigorously, followed by standing for separation of layers. The aqueous phase was extracted with ethyl acetate (10 mL*3), and the organic phases were combined, successively washed with water (10 mL*2) and saturated brine (15 mL), dried over anhydrous sodium sulfate. The solvent was removed by rotary evaporation to obtain the crude product, which was separated and purified by column chromatography, to provide compound 4-((5-(3,5-dimethyl-2,3-dihydroisoxazol-4-yl)-2-methylphenyl)amino)benzonitrile (690 mg), with a yield of 91%.

LC/MS (ESI+) calcd for $C_{19}H_{17}N_3O^+$ (M + $H^+$) m/z, 303.4; found, 304.1.

Step 2: Synthesis of compound 4-((5-(3,5-dimethylisoxazol-4-yl)-2-methylphenyl) (ethyl)amino)benzonitrile

4-((5-(3,5-Dimethyl-2,3-dihydroisoxazol-4-yl)-2-methylphenyl)amino)benzonitrile (100 mg, 0.33 mmol) was weighed and placed in a 25 mL single-neck round-bottom flask, to which was added DMF (4 mL), and then the mixture was stirred at room temperature to dissolve and obtain a clear solution. Then, the system was moved in an ice-water bath to cool down under stirring. When the internal temperature of the system was reduced to about 0 °C, sodium hydride (40 mg, 0.99 mmol) was added to the system. After that, the system was allowed to react under the conditions of insulation and stirring. After 15 min, the solution of ethyl bromide (108 mg, 0.99 mmol) in DMF (1 mL) was added dropwise to the system, and after addition, the ice-water bath was removed. The system was allowed to react at room temperature. After 1.5 h, TLC showed that the raw materials were almost completely consumed, and the reaction was stopped. Ethyl acetate (15 mL) and water (15 mL) were added to the system, and the system was stirred vigorously, then stood for separation of layers. The aqueous phase was extracted with ethyl acetate (10 mL*3), and the organic phases were combined, successively washed with water (10 mL*3) and saturated brine (15 mL), dried over anhydrous sodium sulfate. The solvent was removed by rotary evaporation to obtain the crude product, which was separated and purified by Pre-TLC, to provide compound 4-((5-(3,5-dimethylisoxazol-4-yl)-2-methylphenyl) (ethyl)amino)benzonitrile (95 mg), with a yield of 87%。

LC/MS (ESI+) calcd for $C_{21}H_{21}N_3O^+$ (M + $H^+$) m/z, 331.4; found, 332.3.

Step 3: Synthesis of compound 4-((5-(3,5-dimethylisoxazol-4-yl)-2-methylphenyl)(ethyl)amino)-*N*'-hydroxybenzamidine

4-((5-(3,5-dimethylisoxazol-4-yl)-2-methylphenyl)(ethyl)amino)benzonitrile (95 mg, 0.29 mmol) and hydroxylamine hydrochloride (44 mg, 0.64 mmol) were weighed and placed in a 50 mL single-neck round-bottom flask, to which was added ethanol (10 mL), and the mixture was stirred well at room temperature. Then, the solution of sodium hydroxide (26 mg, 0.64 mmol) in water (1 mL) was added to the system, and after addition, the system was moved to an oil bath and reacted under stirring and refluxing. Next day, the reaction was completed by TLC detection. The oil bath was removed, and the system was cooled to room temperature, to which were added ethyl acetate (30 mL) and water (15 mL), then stirred vigorously, followed by standing for separation of layers. The aqueous phase was extracted with ethyl acetate (10 mL*3), and the organic phases were combined, successively washed with water (15 mL*3) and saturated

brine (15 mL), dried over anhydrous sodium sulfate. The solvent was removed by rotary evaporation to obtain the crude product, which was separated and purified by Pre-TLC, to provide compound 4-((5-(3,5-dimethylisoxazol-4-yl)-2-methylphenyl)(ethyl)amino)-*N*'-hydroxybenzamidine (68 mg), with a yield of 65%.

LC/MS (ESI+) calcd for $C_{21}H_{24}N_4O_2^+$ (M + H+) m/z, 364.4; found, 365.2.

Step 4: Synthesis of compound *N*-(4-(1,2,4-oxadiazole-3- 基 )phenyl)-5-(3,5-dimethylisoxazol-4-yl)-*N*-ethyl-2-methylaniline

4-((5-(3,5-Dimethylisoxazol-4-yl)-2-methylphenyl)(ethyl)amino)-*N*'-hydroxybenzamidine (68 mg, 0.19 mmol) was weighed and placed in a 25 mL single-neck round-bottom flask, to which was added triethyl orthoformate (5 mL), and the mixture was stirred well at room temperature. Subsequently, a drop of trifluoroacetic acid was added to the system. After completion, the system was moved to an oil bath at 110 °C and allowed to react under heating and stirring. After 4 h, the sample was collected and subjected to TLC. TLC showed that the raw material disappeared. The oil bath was removed, and the system was cooled to room temperature, to which were added ethyl acetate (20 mL) and water (10 mL), then stirred vigorously, followed by standing for separation of layers. The aqueous phase was extracted with ethyl acetate (10 mL*3), and the organic phases were combined, successively washed with water (10 mL*3) and saturated brine (15 mL), dried over anhydrous sodium sulfate. The solvent was removed by rotary evaporation to obtain the crude product, which was separated and purified by Pre-TLC, to provide compound *N*-(4-(1,2,4-oxadiazole-3-yl)phenyl)-5-(3,5-dimethylisoxazol-4-yl)-*N*-ethyl-2-methylaniline (18 mg), with a yield of 26%.

LC/MS (ESI+) calcd for $C_{22}H_{22}N_4O_2^+$ (M + $H^+$) m/z, 374.4; found, 375.1.

$^1$H NMR (400 MHz, $CDCl_3$) $\delta$8.65 (s, 1H), 7.91 (d, *J* = 9.0 Hz, 2H), 7.41 (d, *J* = 7.9 Hz, 1H), 7.16 (dd, *J* = 7.8, 1.7 Hz, 1H), 7.05 (d, *J* = 1.7 Hz, 1H), 6.59 (d, *J* = 9.0 Hz, 2H), 3.74 (q, *J* = 7.1 Hz, 2H), 2.42 (s, 3H), 2.28 (s, 3H), 2.18 (s, 3H), 1.29 (t, *J* = 7.1 Hz, 3H).

**Example 8 Synthesis of *N*-(4-(1,2,3-thiazol-4-yl)phenyl)-5-(3,5-dimethylisoxazol-4-yl)-*N*-ethyl-2-methylaniline (compound 111)**

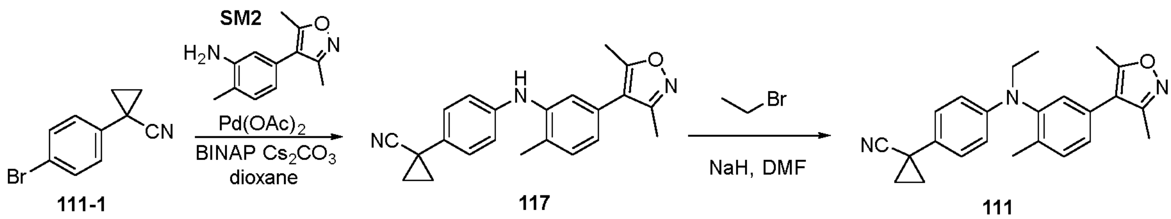


111-1 117 111

Step 1:

5-(3,5-Dimethylisoxazol-4-yl)-2-methylaniline (202 mg, 1 mmol) was dissolved in 5 ml of 1,4-dioxane, to which was added 1-(4-bromophenyl)cyclopropanecarbonitrile (221 mg, 1 mmol), and then cesium carbonate (650 mg, 2 mmol), BINAP (62 mg, 0.1 mmol), and palladium acetate (11 mg, 0.05 mmol) were added to the system. Under argon protection, the mixture was allowed to react overnight at 110 °C. The reaction solution was filtered. The filtrate was washed with 10 ml of water, extracted with 10 ml of ethyl acetate, and then washed with saturated brine, dried with anhydrous sodium sulfate, concentrated, and purified by chromatographic column to obtain 274 mg of compound 1-(4-((5-(3,5-dimethylisoxazol-4-)-2-methylphenyl)amino) phenyl)cyclopropanecarbonitrile, with a yield of 80%. $^1$H NMR (400 MHz, $CDCl_3$): $\delta$7.26 (d, *J*= 8.0 Hz, 1H), 7.20 (m, 2H), 7.07 (d, *J*= 2.0 Hz, 1H), 6.95 (m, 2H), 6.83 (dd, *J* = 7.6, 1.6 Hz, 1H), 2.38 (s, 3H), 2.29 (s, 3H), 2.25 (s, 3H), 1.66 (q, *J* = 4.0 Hz, 2H), 1.35 (q, *J* = 4.0 Hz, 2H). LC/MS (ESI+) calcd for $C_{22}H_{22}N_3O$ ( $[M+H]^+$) *m/z:* 344.2; found 344.2.

Step 2:

60% sodium hydride (8 mg, 0.2 mmol) was dissolved in 3 ml of *N,N*-dimethylformamide, to which was added 1-(4-((5-(3,5-dimethylisoxazol-4-)-2-methylphenyl)amino)phenyl)cyclopropanecarbonitrile (34 mg, 0.1 mmol), and the reaction was stirred at room temperature for 10 min, then bromoethane (22 mg, 0.2 mmol) was added. The mixture was allowed to react at room temperature for 1 h, washed with 5 ml of water, and extracted with 5 ml of ethyl acetate. The organic layer was concentrated under reduced pressure, and then separated and purified by thin layer chromatography, to provide 35 mg of compound 1-(4-((5-(3,5-dimethylisoxazol- 4-)-2-methylphenyl)(ethyl)amino)phenyl)cyclopropanecarbonitrile, with a yield of 94%. $^1$H NMR (400 MHz, $CDCl_3$): $\delta$7.38 (d, *J*= 8.0 Hz, 1H), 7.11 (m, 3H), 7.00 (d, *J*= 2.0 Hz,

1H), 6.48 (m, 2H), 3.66 (q, *J* = 6.8 Hz, 2H), 2.40 (s, 3H), 2.26 (s, 3H), 2.15 (s, 3H), 1.59 (m, 2H), 1.28 (m, 2H), 1.25 (t, *J* = 6.8 Hz, 3H). LC/MS (ESI+) calcd for $C_{24}H_{25}N_3O$ ( $[M+H]^+$) *m/z:* 371.2; found 371.2.

**Example 9 Synthesis of *N*-(5-(3,5-dimethylisoxazol-4-yl)-2-methylphenyl)- N-ethyl-1H-benzo[d]imidazole-6-amine (115)**

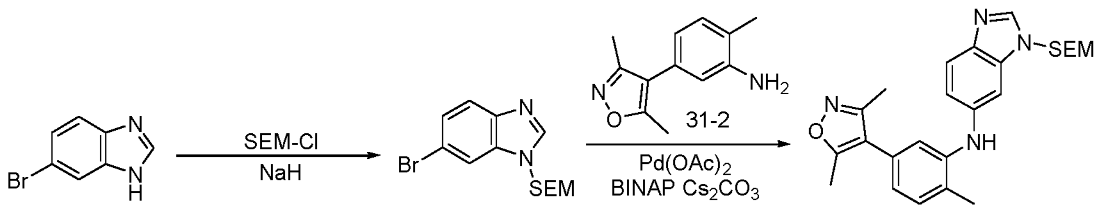


115-1

115-2

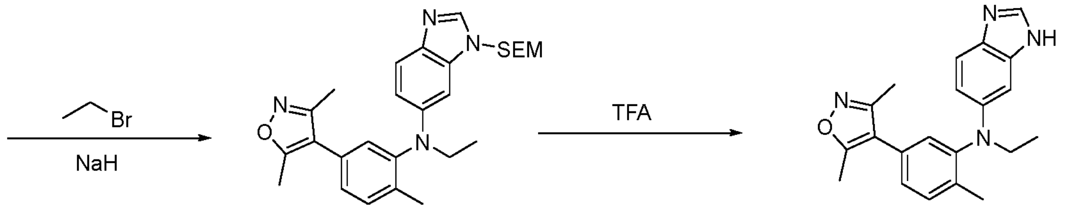


115-3

115

Step 1:

6-Bromo-1H-benzoimidazole (788 mg, 4.0 mmol) was dissolved in 10 ml of DMF, to which was added sodium hydride (320 mg, 8.0 mmol). The mixture was stirred 10 min at room temperature, and then SEM-C1 (1.0 g, 6.0 mmol) was drop added. After addition, the mixture was allowed to react at room temperature for 3 h, and then 20 ml water was added. The resultant solution was extracted with 20 ml of ethyl acetate. The organic layer was respectively washed with water and saturated brine, dried over anhydrous sodium sulfate, concentrated under reduced pressure to dryness, and separated by column chromatography to obtain the product 6-bromo-1-((2-(trimethylsilyl)ethoxy)methyl)-1H-benzoimidazole (700 mg), with a yield of 53.5%. MS (ESI) *m/z* 327.0 $[M+H]^+$.

Step 2:

6-Bromo-1-((2-(trimethylsilyl)ethoxy)methyl)-1H-benzoimidazole (164 mg, 0.5 mmol) and 5-(3,5-dimethylisoxazol-4-yl)-2-methylaniline (101 mg, 0.5 mmol) were dissolved in 5 ml of 1,4-dioxane, to which were added cesium carbonate (406 mg, 1.25mmol), BINAP (16 mg, 0.025 mmol), and palladium acetate (6 mg, 0.025 mmol). The system was exchanged with argon for 3 times, and then reacted overnight at 110 °C. The reaction was cooled to room temperature, to which was added 20 ml of water, and then extracted with 20 ml of ethyl acetate. The organic layer was washed with water and saturated brine, dried with anhydrous sodium sulfate, concentrated under reduced pressure to dryness, and separated by thin-layer chromatography, to provide the product N-(5-(3,5-dimethylisoxazol-4-yl)-2-methylphenyl)-1-((2-(trimethylsilyl)ethoxy)methyl)-1H-benzo[d]imidazole -6-amine (50 mg), with a yield of 22.3%. MS (ESI) *m/z* 449.2 $[M+H]^+$.

Step 3:

N-(5-(3,5-dimethylisoxazol-4-yl)-2-methylphenyl)-1-((2-(trimethylsilyl)ethoxy)methyl)-1H-benzo[d]imidazole-6-amine (45 mg, 0.1 mmol) was dissolved in 3 ml of DMF, to which was added sodium hydride (20 mg, 0.5 mmol). The mixture was stirred 10 min at room temperature, and then bromoethane (55 mg, 0.5 mmol) was added. The mixture was allowed to react at room temperature for 2 h, and then 10 ml of water was added. The resultant solution was extracted with 10 ml of ethyl acetate. The organic layer was respectively washed with water and saturated brine, dried over anhydrous sodium sulfate, concentrated under reduced pressure to dryness, to provide compound **115-3**.

Step 4:

Compound **115-3** obtained in step 3 was dissolved in 3 ml of dichloromethane, to which was added 3 ml of trifluoroacetic acid, and the mixture was stirred overnight at room temperature, then concentrated to dryness under reduced pressure. 10 ml of ethyl acetate was added, and the resultant solution was washed once with saturated aqueous solution of sodium carbonate, and then washed once with saturated brine. The reaction solution was dried over anhydrous sodium sulfate, concentrated under reduced pressure to dryness, and separated by thin layer chromatography, to provide the product N-(5-(3,5-dimethylisoxazol-4-yl)-2-methylphenyl)-N-ethyl-1H- benzo[d]imidazole-6-amine (13 mg), with a yield of 37.5%. MS (ESI) *m/z* 347.2 $[M+H]^+$. $^1H$ NMR (400 MHz, $CDCl_3$) $\delta$ 8.17 (s, 1H), 7.46 (d, J = 7.8 Hz, 1H), 7.37 (d, J = 7.8 Hz, 1H), 7.10 (d, J = 7.5 Hz, 1H), 7.04 (s, 1H), 6.80 (s, 1H), 6.60 (d, J = 8.4 Hz, 1H), 3.73 (d, J = 6.9 Hz, 2H), 2.40 (s, 3H), 2.26 (s, 3H), 2.15 (s, 3H), 1.29 - 1.25 (m, 3H).

With reference to the methods in above examples, the remaining compounds in the present invention were synthesized, and the raw materials used in the synthesis were all commercially available products. The structure, mass spectrum and nuclear magnetic characterization data of each compound are shown in Table 1.

Table 1. Structural characterization of the compounds according to the present invention.

| **Compound No.** | **Structure** | **LC-MS** $(M+H)^+$ | **$^1H$ NMR** |
|---|---|---|---|
| **1** | 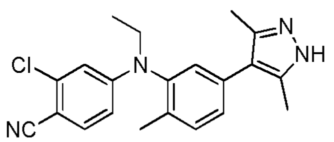 | 365.2 | $^1H$ NMR (400 MHz, CDCl3) δ 7.80 (d, J = 2.0 Hz, 1H), 7.75 (d, J = 8.5 Hz, 1H), 7.58 (dd, J = 8.5, 2.0 Hz, 1H), 7.13 (d, J = 7.5 Hz, 1H), 6.56 (d, J = 23.1 Hz, 2H), 3.22 (s, 2H), 2.41 (s, 3H), 2.32 (s, 3H), 2.20 (s, 3H), 1.34 (t, J = 7.1 Hz, 3H). |
| **2** | 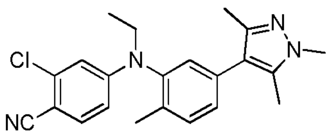 | 379.2 | $^1H$ NMR (400 MHz, CDCl3) δ 7.38 (s, 2H), 7.20 (s, 1H), 6.98 (d, J = 14.6 Hz, 1H), 6.54 (s, 1H), 6.37 (d, J = 7.7 Hz, 1H), 3.85 (s, 3H), 3.75 (s, 2H), 2.28 (s, 6H), 2.14 (s, 3H), 1.26 (d, J = 8.7 Hz, 3H). |
| **3** | 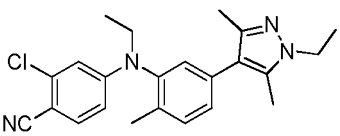 | 391.1 | $^1H$ NMR (400 MHz, CDCl3) δ 7.82 - 7.77 (m, 2H), 7.76 (d, J = 8.5 Hz, 1H), 7.58 (dd, J = 8.5, 1.9 Hz, 1H), 6.98 - 6.94 (m, 1H), 6.92 - 6.87 (m, 1H), 3.03 (q, J = 6.9 Hz, 4H), 2.40 (s, 3H), 2.32 (d, J = 9.6 Hz, 6H), 1.03 (t, J = 7.0 Hz, 6H). |
| **4** | 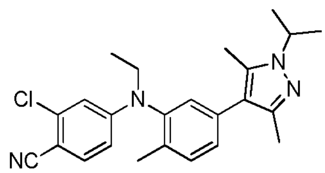 | 407.2 | $^1H$ NMR (400 MHz, CDCl3) δ 7.37 (t, J = 8.0 Hz, 2H), 7.19 (dd, J = 7.8, 1.7 Hz, 1H), 6.95 (d, J = 1.6 Hz, 1H), 6.54 (s, 1H), 6.37 (d, J = 8.3 Hz, 1H), 4.42 (dq, J = 13.4, 6.7 Hz, 1H), 3.71 (s, 2H), 2.27 (s, 3H), 2.25 (s, 3H), 2.13 (s, 3H), 1.52 (s, 3H), 1.50 (s, 3H), 1.28 (dd, J = 7.1, 4.9 Hz, 3H). |
| **5** | 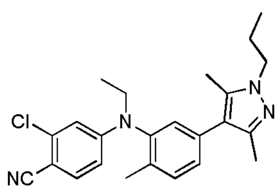 | 407.2 | $^1H$ NMR (400 MHz, CDCl3) δ 7.38 (dd, J = 10.1, 8.5 Hz, 2H), 7.19 (d, J = 7.1 Hz, 1H), 6.95 (s, 1H), 6.52 (d, J = 15.6 Hz, 1H), 6.37 (d, J = 8.3 Hz, 1H), 4.03 (t, J = 7.2 Hz, 2H), 2.28 (s, 3H), 2.26 (s, 3H), 2.13 (s, 3H), 1.90 - 1.84 (m, 2H), 1.32 -1.22 (m, 5H), 0.97 (t, J = 7.4 Hz, 3H). |
| 7 | 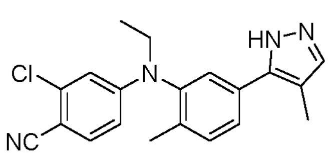 | 350.9 | $^1H$ NMR (400 MHz, $CDCl_3$): δ 8.04 (d, *J* = 1.1 Hz, 1H), 7.69 (dd, *J* = 7.9, 1.8 Hz, 1H), 7.50 (d, *J* = 1.7 Hz, 1H), 7.46 (d, *J* = 8.0 Hz, 1H), 7.37 (d, *J* = 8.9 Hz, 1H), 6.53 (s, 1H), 6.36 (d, *J* = 7.9 Hz, 1H), 3.73 (q, *J* = 7.0 Hz, 2H), 2.26 (s, *J* = 1.1 Hz, 3H), 2.16 (s, 3H), 1.32 - 1.19 (m, 3H). |

(continued)

| Compound No. | Structure | LC-MS (M+H)+ | 1H NMR |
|---|---|---|---|
| **8** | 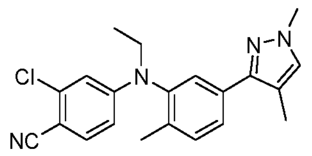 | 364.9 | 1H NMR (400 MHz, $CDCl_3$): δ 7.63 (dd, *J* = 7.9, 1.6 Hz, 1H), 7.46 (d, *J* = 1.4 Hz, 1H), 7.40 (d, *J* = 8.0 Hz, 1H), 7.34 (d, *J* = 8.9 Hz, 1H), 7.26 (s, *J* = 4.6 Hz, 1H), 6.53 (s, 1H), 6.36 (d, *J* = 8.2 Hz, 1H), 3.96 (s, 3H), 3.73-3.69(m, 2H), 2.22 (s, 3H), 2.12 (s, 3H), 1.28 (dd, *J* = 13.3, 6.1 Hz, 3H). |
| **14** | 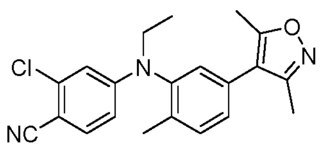 | 366.2 | 1H NMR (400 MHz, $CDCl_3$): δ 7.43 (d, *J* = 8.0 Hz, 1H), 7.38 (d, *J* = 8.8 Hz, 1H), 7.21 (d, *J* = 7.9 Hz, 1H), 6.98 (d, *J* = 1.6 Hz, 1H), 6.53 (s, 1H), 6.37 (s, 1H), 3.69 (s, 2H), 2.42 (s, 3H), 2.28 (s, 3H), 2.15 (s, 3H), 1.28 (t, *J*= 7.2 Hz, 3H). |
| **16** | 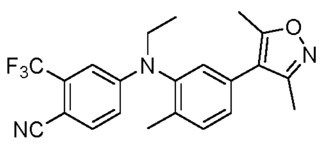 | 400.3 | 1H NMR (400 MHz, $CDCl_3$): δ 7.54 (d, *J* = 8.7 Hz, 1H), 7.46 (d, *J* = 7.9 Hz, 1H), 7.23 (dd, *J* = 7.8, 1.7 Hz, 1H), 6.99 (d, *J* = 1.6 Hz, 1H), 6.78 (s, 1H), 6.59 (d, *J* = 7.0 Hz, 1H), 3.75 (s, 2H), 2.41 (s, 3H), 2.27 (s, 3H), 2.16 (s, 3H), 1.28 (dd, *J* = 15.3, 8.2 Hz, 3H). |
| **21** | 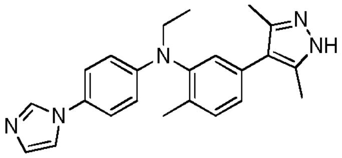 | 371.9 | 1H NMR (400 MHz, DMSO) δ 12.31 (s, 1H), 7.99 (d, J = 0.9 Hz, 1H), 7.51 (t, J = 1.2 Hz, 1H), 7.40 (d, J = 8.0 Hz, 1H), 7.34 (dd, J = 7.1, 5.2 Hz, 2H), 7.19 (dd, J = 7.8,1.7 Hz, 1H), 7.02 (dd,J = 7.5, 1.3 Hz, 2H), 6.56 (dd, J = 9.3, 2.5Hz, 2H), 3.69 (q, J = 7.0 Hz, 2H), 2.29 - 2.13 (m, 6H), 2.10 (d, J = 4.4 Hz,3H), 1.18 (t, J = 7.0 Hz, 3H). |
| **23** | 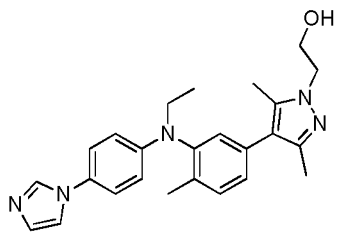 | 416.3 | 1H NMR (400 MHz, DMSO) δ 7.99 (s, 1H), 7.51 (s, 1H), 7.41(d, J = 7.8 Hz, 1H), 7.34 (d, J = 8.9 Hz, 2H), 7.16 (d, J = 5.9 Hz, 1H), 7.01 (d, J = 14.9 Hz, 2H), 6.56 (d, J = 9.0 Hz, 2H), 4.86 (s, 1H), 4.02 (t, J = 5.6 Hz, 2H), 3.69 (d, J = 7.1 Hz, 4H), 2.23 (s, 3H), 2.12 (d, J = 12.4 Hz, 6H), 1.18 (t, J = 7.1 Hz, 3H). |
| **25** | 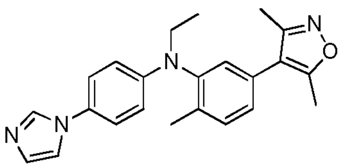 | 373.2 | 1H NMR (400 MHz, $CDCl_3$) δ 7.76 (s, 1H), 7.41 (d, J = 7.8 Hz, 1H), 7.16 (dd, J = 5.9, 2.9 Hz, 4H), 7.04 (d, J = 1.6 Hz, 1H), 6.57 (d, J = 9.0 Hz, 2H), 3.72 (q, J = 7.1 Hz, 2H), 2.42 (s, 3H), 2.28 (s, 3H), 2.19 (s, 3H), 1.28 (t, J = 7.1 Hz, 3H). |
| **26** | 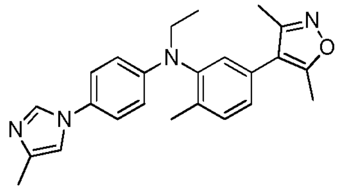 | 387.3 | 1H NMR (400 MHz, $CDCl_3$) δ 7.65 (s, 1H), 7.40 (d, J = 7.6 Hz, 1H), 7.14 (d, J = 7.2 Hz, 3H), 7.04 (s, 1H), 6.88 (s, 1H), 6.56 (d, J = 8.3 Hz, 2H), 3.71 (d, J = 6.9 Hz, 2H), 2.41 (s, 3H), 2.28 (d, J = 2.5 Hz, 6H), 2.18 (s, 3H), 1.27 (t, J = 6.9 Hz, 3H). |
| **27** | 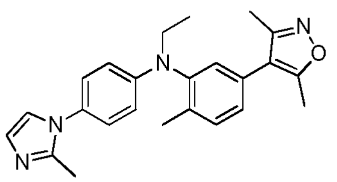 | 387.3 | 1H NMR (400 MHz, $CDCl_3$) δ 7.41 (d, J = 7.8 Hz, 1H), 7.15 (dd, J = 7.8, 1.7 Hz, 1H), 7.05 (dd, J = 5.4, 3.6 Hz, 3H), 7.01 (d, J = 1.0 Hz, 1H), 6.94 (d, J = 1.2 Hz, 1H), 6.55 (s, 2H), 3.72 (q, J = 7.1 Hz, 2H), 2.42 (s, 3H), 2.35 (s, 3H), 2.28 (s, 3H), 2.20 (s, 3H), 1.29 (t, J = 7.1 Hz, 3H). |

(continued)

| Compound No. | Structure | LC-MS $(M+H)^+$ | $^1H$ NMR |
|---|---|---|---|
| **29** | 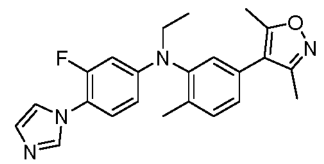 | 391.2 | $^1H$ NMR (400 MHz, $CDCl_3$): δ 7.79 (s, 1H), 7.40 (d, *J* = 7.8 Hz, 1H), 7.20 (s, 2H), 7.14 (dd, *J* = 7.8, 1.8 Hz, 1H), 7.06 (dd, *J* = 9.0, 4.0 Hz, 1H), 7.03 (t, *J* = 3.3 Hz, 1H), 6.47 - 6.41 (m, 2H), 3.68 (q, *J* = 7.1 Hz, 2H), 2.44 - 2.37 (m, 3H), 2.27 (s, 3H), 2.18 (s, 3H), 1.27 (q, *J* = 7.2 Hz, 3H). |
| **30** | 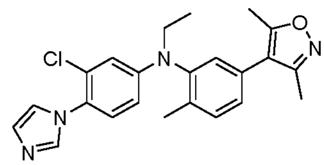 | 407.2 | $^1H$ NMR (400 MHz, d-DMSO): δ 7.73 (s, 1H), 7.52 (d, *J* = 7.8 Hz, 1H), 7.34 (d, *J* = 7.2 Hz, 1H), 7.31 - 7.22 (m, 2H), 7.20 (s, 1H), 7.03 (s, 1H), 6.65 (s, 1H), 6.46 (d, *J* = 8.7 Hz, 1H), 3.71 (d, *J* = 6.8 Hz, 2H), 2.41 (s, 3H), 2.23 (s, 3H), 2.14 (s, 3H), 1.18 (t, *J* = 6.9 Hz, 3H). |
| **31** | 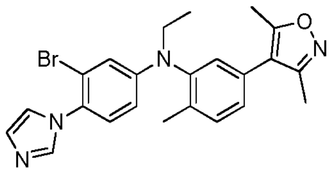 | 451.1 | $^1H$ NMR (400 MHz, $CDCl_3$) δ 7.66 (s, 1H), 7.43 (d, *J* = 7.8 Hz, 1H), 7.18 (dd, *J* = 7.7, 1.8 Hz, 2H), 7.10 - 7.04 (m, 2H), 7.03 (d, *J* = 1.7 Hz, 1H), 6.80 (d, *J* = 2.7 Hz, 1H), 6.45 (dd, *J* = 8.8, 2.7 Hz, 1H), 3.70 (q, *J* = 7.1 Hz, 2H), 2.43 (s, 3H), 2.27 (s, 3H), 2.21 (s, 3H), 1.27 (dt, *J* = 9.7, 7.1 Hz, 3H). |
| **32** | 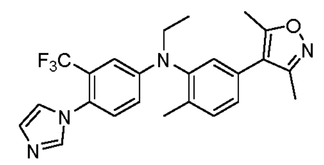 | 441.2 | $^1H$ NMR (400 MHz, $CDCl_3$): δ 7.61 (s, 1H), 7.45 (d, *J* = 7.9 Hz, 1H), 7.20 (dd, *J* = 7.8, 1.8 Hz, 1H), 7.17 (s, 1H), 7.12 (d, *J* = 8.8 Hz, 1H), 7.06 - 7.02 (m, 2H), 6.84 (d, *J* = 2.8 Hz, 1H), 6.64 (dd, *J* = 8.8, 2.8 Hz, 1H), 3.81 - 3.66 (m, 2H), 2.41 (s, 3H), 2.27 (s, 3H), 2.21 (s, 3H), 1.34 - 1.23 (m, 3H). |
| **33** | 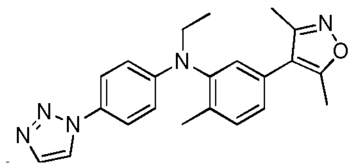 | 374.2 | $^1H$ NMR (400 MHz, CDCl3) δ 8.56 (s, 1H), 8.11 (s, 1H), 7.42 (t, J = 8.2 Hz, 3H), 7.16 (dd, J = 7.8, 1.7 Hz, 1H), 7.05 (d, J = 1.6 Hz, 1H), 6.60 (d, J = 7.9 Hz, 2H), 3.73 (d, J = 7.1 Hz, 2H), 2.42 (s, 3H), 2.28 (s, 3H), 2.18 (s, 3H), 1.28 (t, J = 7.1 Hz, 3H). |
| **34** | 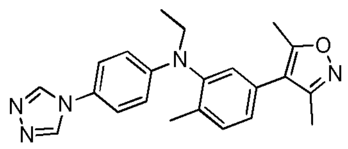 | 374.2 | $^1H$ NMR (400 MHz, CDCl3) δ 8.45 - 8.32 (m, 2H), 7.42 (d, J = 7.8 Hz, 1H), 7.20-7.08 (m, 3H), 7.04 (d, J = 1.5 Hz, 1H), 6.59 (d, J = 7.9 Hz, 2H), 3.72 (d, J = 7.1 Hz, 2H), 2.42 (s, 3H), 2.28 (s, 3H), 2.19 (s, 3H), 1.29 (t, J = 7.1 Hz, 3H). |
| **35** | 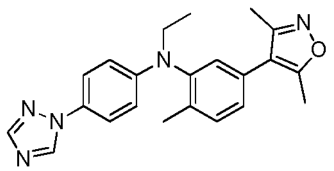 | 374.2 | $^1H$ NMR (400 MHz, CDCl3) δ 8.56 (s, 1H), 8.11 (s, 1H), 7.42 (t, J = 8.2 Hz, 3H), 7.16 (dd, J = 7.8, 1.7 Hz, 1H), 7.05 (d, J = 1.6 Hz, 1H), 6.60 (d, J = 7.9 Hz, 2H), 3.73 (d, J = 7.1 Hz, 2H), 2.42 (s, 3H), 2.28 (s, 3H), 2.18 (s, 3H), 1.28 (t, J = 7.1 Hz, 3H). |
| **45** | 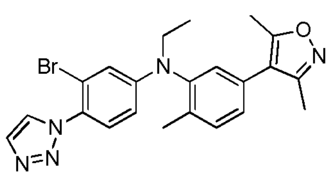 | 452.0 | $^1H$ NMR (400 MHz, $CDCl_3$): δ 7.89 - 7.74 (m, 2H), 7.44 (d, *J* = 7.9 Hz, 1H), 7.24 (d, 1H), 7.19 (dd, *J* = 7.8, 1.7 Hz, 1H), 7.02 (t, *J* = 7.4 Hz, 1H), 6.81 (d, *J* = 2.6 Hz, 1H), 6.50 (dd, *J* = 8.8, 2.5 Hz, 1H), 3.71 (q, *J* = 7.0 Hz, 2H), 2.46 - 2.37 (s, 3H), 2.32 - 2.24 (s, 3H), 2.19 (s, *J* = 6.4 Hz, 3H), 1.28 (dt, *J* = 12.3, 7.1 Hz, 3H). |

(continued)

| Compound No. | Structure | LC-MS $(M+H)^+$ | $^1H$ NMR |
|---|---|---|---|
| **47** | 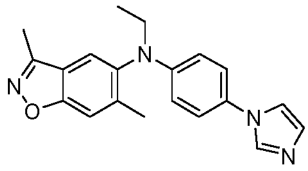 | 333.3 | 1H NMR (400 MHz, CDCl3) δ 7.82 (s, 1H), 7.53 (d, J = 6.0 Hz, 1H), 7.42 (s, 1H), 7.22 - 7.13 (m, 4H), 6.54 - 6.49 (m, 2H), 3.71 (d, J = 7.0 Hz, 2H), 2.55 (s, 3H), 2.29 (s, 3H), 1.30 (t, J = 7.1 Hz, 3H). |
| **48** | 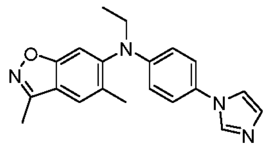 | 333.3 | 1H NMR (400 MHz, CDCl3) δ 7.79 (s, 1H), 7.57 (s, 1H), 7.38 (s, 1H), 7.20 - 7.15 (m, 4H), 6.58 (d, J =8.9 Hz, 2H), 3.76 (dt, J = 16.3, 8.2 Hz, 2H), 2.58 (d, J = 7.4 Hz, 3H), 2.22 (s, 3H), 1.28 (dd, J = 13.5, 6.4Hz, 3H). |
| **57** | 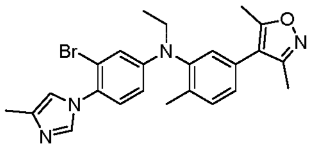 | 465.1 | 1H NMR (400 MHz, CDCl3) δ 7.49 (s, 1H), 7.43 (d, J = 7.9 Hz, 1H), 7.18 (d, J = 6.9 Hz, 1H), 7.11 - 6.98 (m, 2H), 6.77 (d, J = 16.8 Hz, 2H), 6.44 (d, J = 6.3 Hz, 1H), 3.69 (d, J = 6.8 Hz, 2H), 2.42 (s, 3H), 2.29 (d, J = 2.9 Hz, 6H), 2.20 (s, 3H), 1.28 (t, J = 6.9 Hz, 3H). |
| **58** | 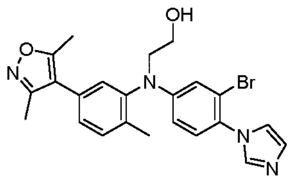 | 467 | |
| **67** | 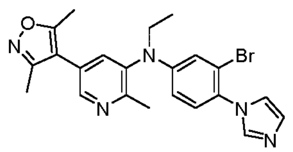 | 452.2 | 1H NMR (400 MHz, CDCl3) δ 8.48 (s, 1H), 7.94 - 7.61 (m, 1H), 7.40 (s, 1H), 7.27 (s, 1H), 7.25 - 6.98 (m, 2H), 6.92-6.76 (m, 1H), 6.59 - 6.42 (m, 1H), 3.75 (d, J = 6.1 Hz, 2H), 2.47 (s, 6H), 2.31 (s, 3H), 1.31 (t, J = 6.8 Hz, 3H). |
| **81** | 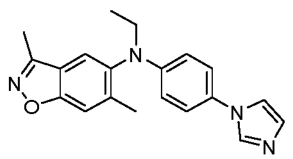 | 333.2 | $^1H$ NMR (400 MHz, $CDCl_3$): δ 7.82 (s, 1H), 7.54 (s, 1H), 7.42 (s, 1H), 7.22 - 7.13 (m, 4H), 6.55 - 6.49 (m, 2H), 3.71 (d, *J* = 7.0 Hz, 2H), 2.55 (s, 3H), 2.29 (s, 3H), 1.27 (dt, *J*= 7.1, 5.6 Hz, 3H). |
| **82** | 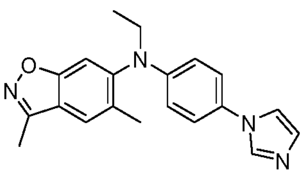 | 333.3 | $^1H$ NMR (400 MHz, $CDCl_3$): δ 7.79 (s, 1H), 7.57 (s, 1H), 7.38 (s, 1H), 7.21 - 7.13 (m, 4H), 6.58 (d, *J* = 8.9 Hz, 2H), 3.76 (dt, *J*= 16.3, 8.2 Hz, 2H), 2.59 (s, 3H), 2.22 (s, 2H), 1.34 - 1.24 (m, 2H). |
| **83** | 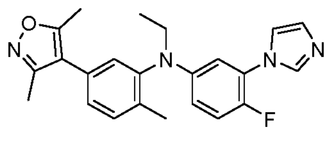 | 391.0 | $^1H$ NMR (400 MHz, $CDCl_3$): δ 7.78 (s, 1H), 7.42 (d, *J* = 7.9 Hz, 1H), 7.22 - 7.09 (m, 4H), 7.03 (d, *J* = 1.7 Hz, 1H), 6.37 - 6.27 (m, 2H), 3.70 (q, *J* = 7.1 Hz, 2H), 2.43 (d, *J*= 8.9 Hz, 3H), 2.29 (s, 3H), 2.20 (s, 3H), 1.28 (dd, *J* = 13.3, 6.2 Hz, 3H). |

(continued)

| Compound No. | Structure | LC-MS $(M+H)^+$ | $^1H$ NMR |
|---|---|---|---|
| **97** | 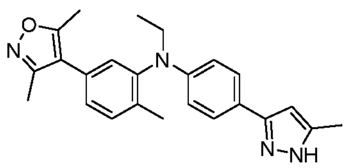 | 387.2 | $^1H$ NMR (400 MHz, $CDCl_3$): $\delta$ 7.54 (m, 2H), 7.38 (dd, $J$ = 8.0, 2.4 Hz, 1H), 7.12 (m, 1H), 7.04 (d, $J$= 2.0 Hz, 1H), 6.56 (dd, $J$ = 8.4, 2.0 Hz, 1H), 6.24 (d, $J$ = 7.2 Hz, 1H), 3.71 (q, $J$ =7.2 Hz, 2H), 2.40 (s, 3H), 2.32 (s, 3H), 2.27 (s, 3H), 2.15 (s, 3H), 1.26 (td, $J$ = 7.2, 2.0 Hz, 3H). |
| **98** | 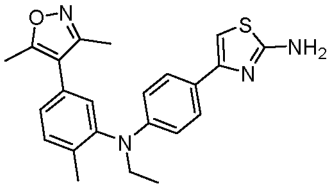 | 405.2 | $^1H$ NMR (400 MHz, $CDCl_3$): $\delta$ 7.69 (m, 2H), 7.20 (dd, $J$ = 7.6, 2.0 Hz, 1H), 7.11 (m, 1H), 6.96 (m, 2H), 6.81 (m, 2H), 3.70 (q, $J$ =7.2 Hz, 2H), 2.32 (s, 3H), 2.29 (s, 3H), 2.25 (s, 3H), 1.26 (t, $J$ = 6.4 Hz, 3H). |
| **99** | 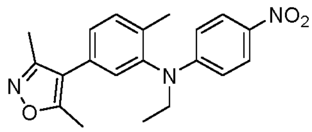 | 352.1 | 1H NMR (400 MHz, CDCl3) δ 8.07 (d, J = 9.4 Hz, 2H), 7.44 (d, J = 7.9 Hz, 1H), 7.22 (dd, J = 7.8, 1.7 Hz, 1H), 7.02 (d, J = 1.7 Hz, 1H), 6.47 (d, J = 8.8 Hz, 2H), 3.77 (s, 2H), 2.42 (s, 3H), 2.28 (s, 3H), 2.16 (s, 3H), 1.31 (t, J = 7.1 Hz, 3H). |
| **100** | 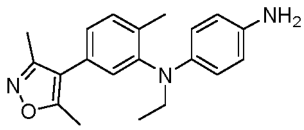 | 322.1 | |
| **101** | 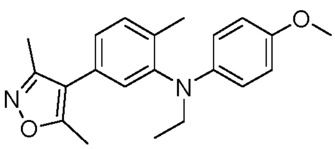 | 337.1 | 1H NMR (400 MHz, CDCl3) δ 7.32 (d, J = 7.5 Hz, 1H), 7.07 - 7.00 (m, 2H), 6.78 (d, J = 9.1 Hz, 2H), 6.58 (d, J = 9.0 Hz, 2H), 3.74 (d, J = 5.5 Hz, 3H), 3.65 (d, J = 7.0 Hz, 2H), 2.39 (s, 3H), 2.26 (d, J = 2.7 Hz, 3H), 2.16 (s, 3H), 1.23 (t, J = 7.1 Hz, 4H). |
| **102** | 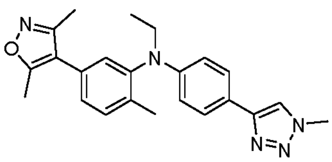 | 388.2 | $^1H$ NMR (400 MHz, $CDCl_3$) δ 7.66 (d, J = 8.7 Hz, 2H), 7.39 (d, $J$ = 7.8 Hz, 1H), 7.13 (dd, $J$ = 7.8, 1.7 Hz, 1H), 7.05 (d, $J$ = 1.7 Hz, 1H), 6.59 (d, $J$ = 8.5 Hz, 2H), 4.14 (s, 3H), 3.72 (q, $J$ = 7.1 Hz, 2H), 2.41 (s, 3H), 2.28 (s, 3H), 2.18 (s, 3H), 1.27 (t, $J$ = 7.1 Hz, 3H). |
| **104** | 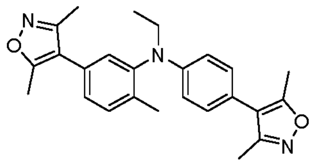 | 402.2 | 1H NMR (400 MHz, CDCl3) δ 7.40 (d, J = 7.8 Hz, 1H), 7.15 - 7.11 (m, 1H), 7.06 (s, 2H), 7.04 (s, 1H), 6.59 (d, J = 8.8 Hz, 2H), 3.71 (q, J = 7.1 Hz, 2H), 2.41 (d, J = 4.9 Hz, 3H), 2.38 (s, 3H), 2.28 (s, 3H), 2.26 (s, 3H), 2.20 (s, 3H), 1.28 (t, J = 7.1 Hz, 3H). |
| **105** | 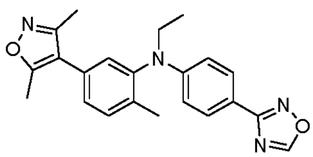 | 375.1 | $^1H$ NMR (400 MHz, $CDCl_3$) δ 8.65 (s, 1H), 7.91 (d, $J$ = 9.0 Hz, 2H), 7.41 (d, $J$ = 7.9 Hz, 1H), 7.16 (dd, $J$ = 7.8, 1.7 Hz, 1H), 7.05 (d, $J$ = 1.7 Hz, 1H), 6.59 (d, $J$ = 9.0 Hz, 2H), 3.74 (q, $J$ = 7.1 Hz, 2H), 2.42 (s, 3H), 2.28 (s, 3H), 2.18 (s, 3H), 1.29 (t, $J$ = 7.1 Hz, 3H). |
| **106** | 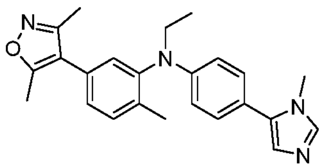 | 387.3 | 1H NMR (400 MHz, CDCl3) δ 7.78 - 7.63 (m, 2H), 7.53 (s, 1H), 7.46 - 7.35 (m, 2H), 7.34 (s, 1H), 7.23 (s, 1H), 7.21 (s, 1H), 7.12 (s, 1H), 3.84 (s, 3H), 3.82 (s, 1H), 3.39 (s, 1H), 2.21(s, 6H), 2.13 (s, 3H), 1.23 (s, 3H). |

(continued)

| Compound No. | Structure | LC-MS $(M+H)^+$ | $^1$H NMR |
|---|---|---|---|
| **107** | 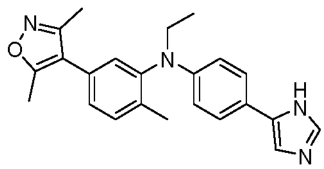 | 373.1 | 1H NMR (400 MHz, CDCl3) δ 7.73 - 7.56 (m, 3H), 7.25 (dd, J = 10.5, 5.3 Hz, 4H), 7.16(s, 1H), 7.03 (d, J = 6.4 Hz, 2H), 3.64 (s, 1H), 3.45 (s, 1H), 2.27 (d, J = 11.9 Hz, 9H), 1.23 (s, 3H). |
| **108** | 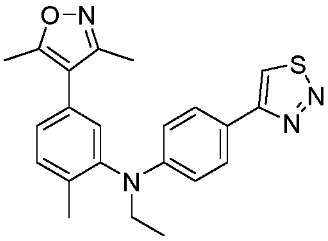 | 391.2 | $^1$ H NMR (400 MHz, $CDCl_3$): $\delta$ 8.19 (d, *J* = 1.6 Hz, 1H), 7.73 (d, *J* = 8.8 Hz, 2H), 7.37 (d, *J* = 8.0 Hz, 1H), 7.23 (dd, *J* = 7.6, 2.0 Hz, 1H), 6.99 (d, *J* = 1.6 Hz, 1H), 6.57 (m, 2H), 3.63 (q, *J* = 6.8 Hz, 2H), 2.48 (s, 3H), 2.25 (s, 3H), 2.17 (s, 3H), 1.24 (t, *J* = 6.8 Hz, 3H). |
| **109** | 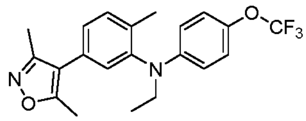 | 391.1 | 1H NMR (400 MHz, CDCl3) δ 7.38 (d, J = 7.8 Hz, 1H), 7.12 (dd, J = 7.8, 1.6 Hz, 1H), 7.01 (d, J = 7.5 Hz, 3H), 6.47 (d, J = 9.2 Hz, 2H), 3.67 (q, J = 7.1 Hz, 2H), 2.40 (s, 3H), 2.27 (s, 3H), 2.16 (s, 3H), 1.25 (t, J = 7.1 Hz, 3H). |
| **110** | 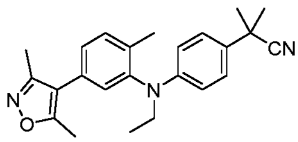 | 374.2 | $^1$H NMR (400 MHz, $CDCl_3$): $\delta$ 7.34 (m, 2H), 7.24 (s, 1H), 7.09 (d, *J* = 1.6 Hz, 1H), 6.98 (m, 2H), 6.82 (dd, *J* = 7.6, 1.6 Hz, 1H), 3.68 (q, *J* = 6.8 Hz, 2H), 2.37 (s, 3H), 2.28 (s, 3H), 2.24 (s, 3H), 1.69 (s, 6H), 1.24 (t, *J* = 6.8 Hz, 3H). |
| **111** | 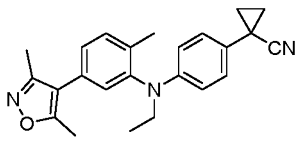 | 372.2 | $^1$H NMR (400 MHz, $CDCl_3$): $\delta$ 7.38 (d, *J* = 8.0 Hz, 1H), 7.11 (m, 3H), 7.00 (d, *J* = 2.0 Hz, 1H), 6.48 (m, 2H), 3.66 (q, *J*= 6.8 Hz, 2H), 2.40 (s, 3H), 2.26 (s, 3H), 2.15 (s, 3H), 1.59 (m, 2H), 1.28 (m, 2H), 1.25 (t, *J* = 6.8 Hz, 3H). |
| **112** | 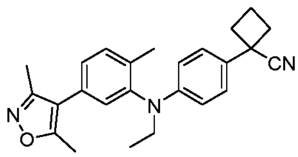 | 386.2 | $^1$H NMR (400 MHz, $CDCl_3$): $\delta$ 7.38 (d, *J* = 8.0 Hz, 1H), 7.19 (m, 2H), 7.12 (dd, *J* = 8.0, 2.0 Hz, 1H), 7.02 (d, *J* = 2.0 Hz, 1H), 6.52 (m, 2H), 3.68 (q, *J* =7.2 Hz, 2H), 2.76 (m, 2H), 2.55 (m, 2H), 2.40 (s, 3H), 2.34 (m, 1H), 2.27 (s, 3H), 2.16 (s, 3H), 2.02 (m, 1H), 1.25 (t, *J* = 6.4 Hz, 3H). |
| **113** | 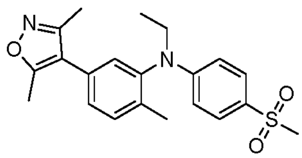 | 385.1 | 1H NMR (400 MHz, CDCl3) δ 7.68 (d, J = 8.8 Hz, 2H), 7.43 (d, J = 7.8 Hz, 1H), 7.20 (d, J = 7.7 Hz, 1H), 7.01 (s, 1H), 6.55 (d, J = 8.5 Hz, 2H), 3.73 (s, 2H), 3.01 (s, 3H), 2.42 (s, 3H), 2.28 (s, 3H), 2.16 (s, 3H), 1.29 (t, J = 7.1 Hz, 3H). |
| **114** | 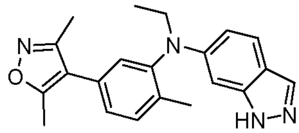 | 347.2 | 1H NMR (400 MHz, CDCl3) δ 7.99 - 7.78 (m, 1H), 7.39 (d, J = 8.1 Hz, 2H), 7.14 (d, J = 6.7 Hz, 1H), 7.05 (s, 1H), 6.57 (d, J = 38.5 Hz, 1H), 6.50 - 6.36 (m, 1H), 3.86 - 3.67 (m, 2H), 2.41 (s, 2H), 2.27 (s, 3H), 2.17 (s, 3H), 1.28 (d, J = 7.4 Hz, 3H). |

(continued)

| Compound No. | Structure | LC-MS $(M+H)^+$ | $^1H$ NMR |
|---|---|---|---|
| **115** | 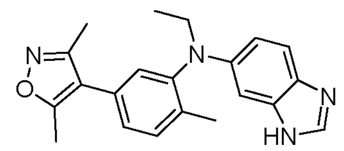 | 347.2 | 1H NMR (400 MHz, CDCl3) δ 8.17 (s, 1H), 7.46 (d, J = 7.8 Hz, 1H), 7.37 (d, J = 7.8 Hz, 1H), 7.10 (d, J = 7.5 Hz, 1H), 7.04 (s, 1H), 6.80 (s, 1H), 6.60 (d, J = 8.4 Hz, 1H), 3.73 (d, J = 6.9 Hz, 2H), 2.40 (s, 3H), 2.26 (s, 3H), 2.15 (s, 3H), 1.29 - 1.25 (m, 3H). |
| **116** | 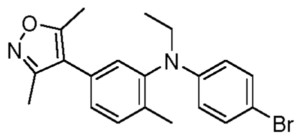 | 385 | 1H NMR (400 MHz, CDCl3) δ 7.30 (d, J = 7.8 Hz, 1H), 7.17 - 7.14 (m, 2H), 7.04 (dd, J = 7.8, 1.8 Hz, 1H), 6.94 (d, J = 1.7 Hz, 1H), 6.35 - 6.31 (m, 2H), 3.63 - 3.52 (m, 2H), 2.34 (d, J = 4.8 Hz, 3H), 2.20 (d, J= 5.2 Hz, 3H), 2.08 (s, 3H), 1.16 (dd, J = 9.5, 4.7 Hz, 3H). |
| **117** | 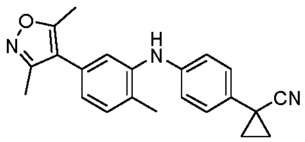 | 344.2 | $^1H$ NMR (400 MHz, $CDCl_3$): δ 7.26 (d, *J* = 8.0 Hz, 1H), 7.20 (m, 2H), 7.07 (d, *J* = 2.0 Hz, 1H), 6.95 (m, 2H), 6.83 (dd, *J* = 7.6, 1.6 Hz, 1H), 2.38 (s, 3H), 2.29 (s, 3H), 2.25 (s, 3H), 1.66 (q, *J* = 4.0 Hz, 2H), 1.35 (q, *J*= 4.0 Hz, 2H). |
| **123** | 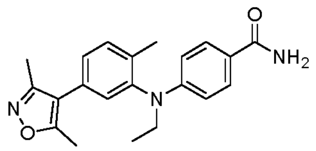 | 350.1 | 1H NMR (400 MHz, DMSO) δ 7.68 (d, J = 9.0 Hz, 2H), 7.63 (s, 1H), 7.49 (d, J = 7.9 Hz, 1H), 7.31 (dd, J = 7.8, 1.8 Hz, 1H), 7.16 (d, J = 1.7 Hz, 1H), 6.94 (s, 2H), 6.46 (d, J = 8.9 Hz, 2H), 3.70 (q, J = 6.9 Hz, 2H), 2.40 (s, 3H), 2.23 (s, 3H), 2.09 (s, 3H), 1.20 - 1.14 (m, 3H). |
| **124** | 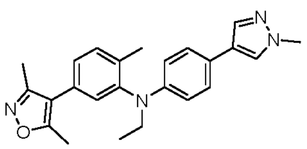 | 387.3 | 1H NMR (400 MHz, CDCl3) δ 7.60 (s, 1H), 7.42 (s, 1H), 7.31 (d, J = 7.8 Hz, 1H), 7.21 (d, J = 2.0 Hz, 2H), 7.04 (dd, J = 7.8, 1.8 Hz, 1H), 6.98 (d, J = 1.7 Hz, 1H), 6.48 (d, J = 8.8 Hz, 2H), 3.87 (s, 3H), 3.63 (q, J = 7.1 Hz, 2H), 2.34 (s, 3H), 2.20 (s, 3H), 2.12 (d, J = 10.3 Hz, 3H), 1.19 (t, J = 7.1 Hz, 3H). |
| **125** | 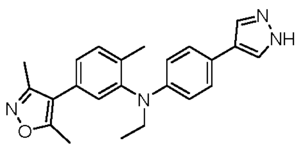 | 373.1 | 1H NMR (400 MHz, CDCl3) δ 7.69 (s, 2H), 7.33 - 7.29 (m, 1H), 7.25 (d, J = 8.5 Hz, 2H), 7.04 (dd, J = 7.8, 1.7 Hz, 1H), 6.98 (s, 1H), 6.50 (t, J = 7.5 Hz, 2H), 3.63 (q, J = 7.0 Hz, 2H), 2.34 (s, 3H), 2.21 (s, 3H), 2.11 (s, 3H), 1.21 - 1.17 (m, 3H). |
| **126** | 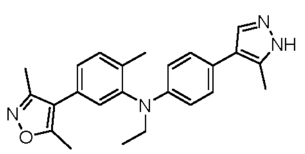 | 387.3 | 1H NMR (400 MHz, CDCl3) δ 7.57 (s, 1H), 7.48 (s, 1H), 7.32 (d, J = 7.8 Hz, 1H), 7.14 (d, J = 8.7 Hz, 2H), 7.05 (dd, J = 7.7, 1.7 Hz, 1H), 6.99 (s, 1H), 6.52 (d, J = 8.7 Hz, 2H), 3.64 (q, J = 7.0 Hz, 2H), 2.40 (s, 3H), 2.34 (s, 3H), 2.21 (s, 3H), 2.13 (s, 3H), 1.21 (dd, J = 9.0, 5.1 Hz, 3H). |
| **127** | 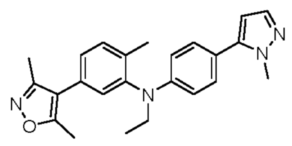 | 387.3 | 1H NMR (400 MHz, CDCl3) δ 7.44 (t, J = 2.9 Hz, 1H), 7.34 (d, J = 7.8 Hz, 1H), 7.15 (d, J = 8.9 Hz, 2H), 7.08 (dd, J = 7.8, 1.8 Hz, 1H), 6.99 (d, J = 1.7 Hz, 1H), 6.52 (d, J = 8.8 Hz, 2H), 6.17 (d, J = 1.9 Hz, 1H), 3.84 (s, 3H), 3.66 (q, J = 7.1 Hz, 2H), 2.35 (s, 3H), 2.21 (s, 3H), 2.13 (s, 3H), 1.25 - 1.17 (m, 3H). |

(continued)

| Compound No. | Structure | LC-MS $(M+H)^+$ | $^1H$ NMR |
|---|---|---|---|
| **128** | 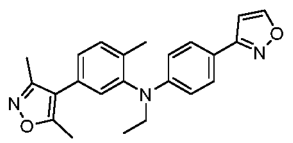 | 374.2 | $^1H$ NMR (400 MHz, $CDCl_3$) δ 8.37 (d, J = 1.6 Hz, 1H), 7.63 (d, *J* = 8.8 Hz, 2H), 7.41 (d, *J* = 8.0 Hz, 1H), 7.15 (dd, *J* = 7.6, 2.0 Hz, 1H), 7.05 (d, *J* = 1.6 Hz, 1H), 6.57 (m, 3H), 3.73 (q, *J* = 6.8 Hz, 2H), 2.41 (s, 3H), 2.28 (s, 3H), 2.18 (s, 3H), 1.28 (t, *J* = 6.8 Hz, 3H). |
| **129** | 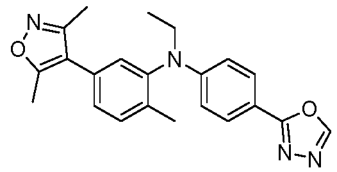 | 375.2 | $^1H$ NMR (400 MHz, $CDCl_3$) δ 8.29 (s, 1H), 7.81 (d, *J* = 8.8 Hz, 2H), 7.35 (d, *J* = 7.9 Hz, 1H), 7.11 (dd, *J* = 7.8, 1.7 Hz, 1H), 6.97 (d, *J* = 1.7 Hz, 1H), 6.52 (d, *J* = 8.7 Hz, 2H), 3.68 (dd, *J* = 14.1, 7.0 Hz, 2H), 2.35 (s, 3H), 2.22 (s, 3H), 2.10 (s, 3H), 1.23 (t, *J* = 7.1 Hz, 3H) |
| **130** | 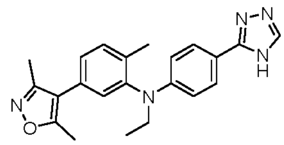 | 374.2 | |
| **131** | 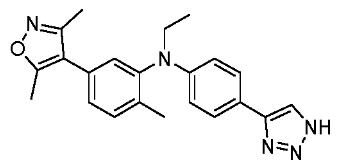 | 374.2 | $^1H$ NMR (400 MHz, $CDCl_3$) δ 7.64 (d, J = 8.5 Hz, 1H), 7.59 (d, *J* = 8.8 Hz, 1H), 7.40 (dd, *J* = 7.9, 2.9 Hz, 1H), 7.15 (td, *J* = 7.7, 1.7 Hz, 1H), 7.04 (dd, *J* = 7.4, 1.6 Hz, 1H), 6.59 (d, *J* = 8.1 Hz, 2H), 5.80 (s, 1H), 3.72 (q, *J* = 7.0 Hz, 2H), 2.41 (s, 3H), 2.28 (s, 3H), 2.18 (s, 3H), 1.27 (t, *J* = 7.1 Hz, 3H). |
| **132** | 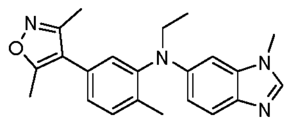 | 361.1 | |
| **133** | 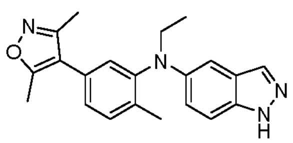 | 347.1 | |

In the following, the beneficial effects of the compounds according to the present invention were demonstrated by experimental examples.

**Experimental example 1. The inhibitory activity of the compound according to the present invention on proliferation of prostate cancer cells**

**Experimental method:**

**1. Biological determination of the inhibitory effect on the proliferation of prostate cancer cells LNCaP/AR**

(1) Experimental materials and instruments:

LNCaP/AR cell line (Provided by Sichuan Kangcheng BioTechnology Co., Ltd.) Fetal bovine serum FBS (Gibco, Cat. No. 10099-141)
0.01M PBS (Biosharp, Cat. No. 162262)

RIPM1640 media (Hyclone, Cat. No. 308090.01)
Penicillin-Streptomycin (Hyclone, Cat. No. SV30010)
Cell counting kit-8 (Signalway Antibody, Cat. No. CP002)
Dimethylsulfoxide DMSO (Sigma, Cat. No. D5879)
Centrifuge Tube, 15 ml (Excell Bio, Cat. No. CS015-0001)
Cell Culture Dish (Excell Bio, Cat. No. CS016-0128)
96-well cell culture cluster (Corning, Cat. No. 3599)
Microplate reader (Thermo Multiskan MK3 type)

(2) Experimental method:

a. Buffer preparation

Cell culture medium: RIPM1640 media, 10% FBS, 1% Pen Strep;
PBS buffer: PBS powder was dissolved in 2 L ultrapure water and sterilized.

b. Experimental procedures:

1) LNCaP/AR cells were subcultured in cell culture medium, and the cells in good growth condition were seeded in a 96-well plate, with 80 μL per well (i.e. 1000 cells per well), and cultured overnight in a 37 °C, 5% $CO_2$ cell incubator.
2) The drug was prepared with dimethylsulfoxide (DMSO) as a 10 mM stock solution. Immediately before use, the stock solution was diluted 3 times with DMSO, and then diluted as a 3-fold gradient to obtain 9 concentration gradients. Then, the compound of each concentration was diluted 200 times with the culture media (to ensure that the DMSO concentration in the culture system was 0.1%), and two multiple holes were set for each concentration. 20 μL of the diluted compound was added to the cell culture well (with a final concentration of 10 μM, 3.3 μM, 1.1 μM...), and then shook gently to mix. In addition, three negative control wells with only cells and three blank control wells with only culture medium were included (6 wells being each added with DMSO diluted 200 times with 20 μL culture medium).

c. Result detection:

1) After culturing for 6 days, 10 μL of CCK-8 was added to each well and continued culturing for 1 hour in a 37 °C, 5% $CO_2$ cell incubator.
2) The absorbance (OD value) was measured at 450 nm using a multifunctional microplate reader.
3) The data were analyzed by the Dose-response-inhibition equation in the software GraphPad Prism5, and the $IC_{50}$ value was obtained.

2. Biological determination of the inhibitory effect on the proliferation of other prostate cancer cells

The inhibitory activity of the compound according to the present invention on other drug-resistant prostate cancer cells was measured by the same method as described above, in which VCaP and 22RV1 cells were commercially available.

**Result analysis:**

The results for the inhibitory activity of the compound according to the present invention against the proliferation of various prostate cancer cells are shown in Table 2 and Table 3. It could be seen that the compound of the present invention could not only inhibit the proliferation of the prostate cancer cell line LNCaP/AR with overexpression of the androgen receptor AR, but also have a good inhibitory effect on the prostate cancer cell lines VCaP and 22RV1, which are resistant to the marketed prostate cancer drug (enzalutamide).

Table 2. The inhibitory activity of the compound according to the present invention on prostate cancer cell LNCap/AR

| Compound | $IC_{50}$ (μM) | Compound | $IC_{50}$ (μM) | Compound | $IC_{50}$ (μM) |
|---|---|---|---|---|---|
| **1** | 0.33 | **33** | 0.75 | **108** | 0.76 |
| **2** | 0.51 | **34** | 0.95 | **109** | 4.46 |

(continued)

| Compound | $IC_{50}$ (μM) | Compound | $IC_{50}$ (μM) | Compound | $IC_{50}$ (μM) |
|---|---|---|---|---|---|
| **3** | 1.4 | **35** | 1.2 | **110** | 0.93 |
| **4** | 1.4 | **43** | 2.79 | **111** | 0.14 |
| **5** | 1.45 | **45** | 0.82 | **112** | 1.86 |
| **6** | 3.67 | **47** | 3.3 | **113** | 2.53 |
| **7** | 2.19 | **48** | 3.7 | **114** | 0.89 |
| **14** | 0.55 | **57** | 2.38 | **115** | 1.3 |
| **16** | 1.66 | **58** | 2.32 | **116** | 0.61 |
| **21** | 3.98 | **67** | 2.03 | **119** | 0.17 |
| **23** | 6.42 | **97** | 3.23 | **121** | 2.79 |
| **25** | 1.25 | **98** | 4.77 | **123** | 3.03 |
| **26** | 1.14 | **99** | 0.4 | **129** | 1.13 |
| **27** | 0.38 | **100** | 0.7 | **130** | 1.16 |
| **29** | 0.49 | **102** | 1.45 | **131** | 0.88 |
| **30** | 0.9 | **105** | 0.71 | **132** | 2.54 |
| **31** | 0.54 | **106** | 0.7 | **133** | 8.74 |
| **32** | 1.4 | **107** | 3.67 | **134** | 0.89 |
| **126** | 0.99 | | | | |

Table 3. The inhibitory activity of the compound according to the present invention on other prostate cancer cells.

| Compound | $IC_{50}$ (μM) | |
|---|---|---|
| | VCap | 22RV1 |
| **2** | 0.78 | 5.2 |
| **26** | 1.16 | 4.08 |
| **27** | 0.99 | 3.77 |
| **29** | 1.22 | 5.6 |
| **30** | 2.27 | > 10 |
| **31** | 1.35 | 9.37 |
| **32** | 0.98 | 3.5 |
| **33** | 0.88 | 2.41 |
| **34** | 1 | 2.58 |
| **45** | 0.75 | 7.75 |
| **83** | 1.27 | 5.2 |
| **101** | - | 0.07 |
| **111** | - | 0.27 |
| **116** | - | 0.63 |
| **120** | - | 0.63 |
| **123** | - | 5.64 |

(continued)

| Compound | $IC_{50}$ (μM) | |
|---|---|---|
| | VCap | 22RV1 |
| **126** | - | 3.17 |
| **129** | - | 9.79 |
| **134** | - | 6.06 |
| In Table 3, "-" means that no test was performed. | | |

**Experimental example 2 The inhibitory activity of the compound according to the present invention on the function of androgen receptor (AR)**

**Experimental materials:**

HEK293 cell line
DMEM with phenol red (Invitrogen, Cat. No. 11960051)
DMEM without phenol red (Gibco, Cat. No.31053028)
FBS (Hyclone, Cat. No.SV30087.03)
Dialyzed FBS (Biological Industries, Cat. No.04-011-1A)
Fugene (Promega, Cat. No. E2311)
Opti-MEM (Gibco, Cat. No.11058021)
Androgen receptor clone (Origene, Cat. No.RC235415)
pGL4.36 vector (Promega, Cat. No. E1360)
Steady-Glo (Promega, Cat. No. E2550)
Assay plate (Greiner, Cat. No.655098)

**Experimental method:**

1. Reagent preparation

| | | |
|---|---|---|
| Cell culture medium 88% DMEM with phenol red, 10% FBS, 1% Pen Strep, and 1% GlutaMax. | Medium for seeding cells 89% DMEM without phenol red, 10% dialyzed FBS, and 1% GlutaMax | Transfection reagent 6.37 μL AR + 22 μL pGL4.36 + 69.3 Fugene + 2102 μL Opti-MEM |

2. Experimental procedures:

1) The cell suspension was diluted with the culture medium for seeding cells to a concentration of $4.0\times10^5$ cells/ml, for plating and adding transfection reagents.
2) The diluted cell suspension was inoculated into a cell culture plate, with 100 μL per well, and the plate was placed in a cell incubator and cultured overnight.
3) The transfection reagent was prepared as 10 μL/well, and added into the cell culture plate after 15 min at room temperature, then the plate was placed in a cell incubator and cultured overnight.
4) The compound was diluted by a 3-fold gradient with TECAN sampler, to obtain 8 concentration gradients, and the final DMSO concentration was 0.25%. The diluted compound was cultured in a 37 °C incubator for 30 min.
5) 250 nL of testosterone at a final concentration of 2 nM was added to the control well with an EC80 sampler, placed in a cell incubator, and cultured overnight.
6) Steady-Glo was added at 100 (μL/well to the cell culture plate, and then the plate was left at room temperature for 15 min, followed by reading on Envision.

**Analysis of results:**

The data were analyzed according to the formula %Effect = (Sample value - LC) / (HC - LC) * 100, using XL-fit software (provided by Business Solutions Ltd., version number XL fit 5.0). The inhibition rate of each compound against androgen receptor (AR) was calculated, and the half-inhibitory concentration $IC_{50}$ of each compound against androgen receptor (AR) was further calculated.

Table 4. The inhibitory activity of the compound according to the present invention on the function of androgen receptor (AR).

| Compound | $IC_{50}$ (μM) |
|---|---|
| **2** | 0.78 |
| **14** | 1.5 |
| **27** | 0.89 |
| **29** | 0.45 |
| **31** | 2.1 |
| **45** | 1.7 |
| **83** | 0.83 |
| **104** | 1.3 |
| **110** | 1.6 |
| **111** | 1.6 |
| **116** | 4.9 |
| **127** | 3.4 |

It could be seen that the compound of the present invention could effectively inhibit the activity of androgen receptor (AR).

**Experimental example 3 The inhibitory activity of the compound according to the present invention on BRD4**

Homogeneous time-resolved fluorescence technique (HTRF) was used to detect the binding effect of compound to BRD4 (D1+D2) protein.

**Experimental materials:**

BRD4(1,2) (BPS, Cat. No. 31044),
I-CBP 112 (MCE, Cat. No. HY-19541),
(+)-JQ1 (BPS, Cat. No. 27402).

**Experimental method:**

Using homogeneous time-resolved fluorescence method, the detection steps are as follows:

1. According to the layout of the detection plate, the test compound was gradiently diluted on the Echo plate, and the final dilution concentration of DMSO was 0.1%.
2. The compound or DMSO was transferred to the 384-well detection plate with the Echo automatic sampler.
3. Two-fold concentration of protein and peptide mixture was added to the detection plate.
4. Two-fold concentration of the detection mixture was added to the detection plate, and shook for 30 s.
5. Incubating for 2 h at room temperature.
6. The fluorescence signal was read on the Envision multifunctional microplate reader (excitation wavelength at 340 nm; emission wavelength at 615 nm and 665 nm).

**Analysis of the results:**

Curve Fitting

1. The experimental data were entered into an Excel file, and equation (1) was used to calculate the inhibition rate:

$$\text{Equation (1): Inh \%=( Max-Signal)/(Max-Min)*100}$$

2. The obtained data were input into GraphPad software, and equation (2) was used to calculate the $IC_{50}$ value.

$$\text{Equation (2): Y=Bottom + (Top-Bottom) / (1+10^ ((LogIC50-X)*Hill Slope))}$$

wherein, Y axis is the inhibition rate, while X axis is the compound concentration.

The half-inhibitory concentration $IC_{50}$ of the compound according to the present invention on BRD4 was shown in Table 5. It might be seen that the compound of the present invention could effectively inhibit the activity of BRD4. Combined with the above conclusion that the compound of the present invention could effectively inhibit the activity of the androgen receptor (AR), it might be concluded that the compound of the present invention was able to recognize AR and BRD4 at the same time, and simultaneously exert an effective inhibitory effect on AR and BRD4.

Table 5: The inhibitory activity of the compound according to the present invention on BRD4

| Compound | $IC_{50}$ (μM) |
|---|---|
| **27** | 4.1 |
| **31** | 6.3 |
| **45** | 1.9 |
| **83** | 6.7 |

In summary, the present invention provided a compound of formula I, which had a dual inhibitory effect on AR and BRD4. The compound of the present invention could not only inhibit the proliferation of the prostate cancer cell line LNCaP/AR with overexpression of the androgen receptor AR, but also show good inhibitory effects on the prostate cancer cell lines VCaP and 22RV1 that were resistant to the marketed prostate cancer drug (enzalutamide). The compound of the present invention itself, as a compound that could recognize both AR and BRD4 dual targets, could be used as AR/BRD4 dual inhibitors, and also be used to prepare proteolytic targeting chimeras (PROTACs) that induced the degradation of AR/BRD4 dual targets, as well as have a good application prospect in the preparation of drugs for the treatment of AR- and BRD4-related diseases.

**Claims**

1. Compound of formula I, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereoisomer thereof, or a mixture thereof, or a metabolite thereof, or a metabolic precursor thereof, or an isotope-substituted form thereof:

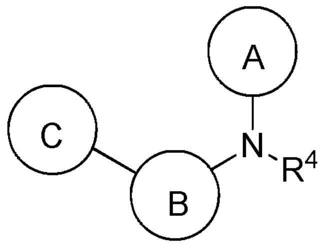

Formula I

wherein

each of rings A, B, and C is independently selected from the group consisting of none, substituted or unsubstituted unsaturated heterocycles, substituted or unsubstituted unsaturated carbocyclic rings, substituted or unsubstituted fused rings; preferably, none, substituted or unsubstituted monocyclic aromatic ring, substituted or unsubstituted monocyclic heteroaromatic ring, and substituted or unsubstituted fused ring; more preferably, none, substituted or unsubstituted 3-8 membered monocyclic aromatic ring, substituted or unsubstituted 3-8-membered monocyclic heteroaromatic ring, substituted or unsubstituted heteroaromatic ring-fused heteroaromatic ring, substituted or unsubstituted benzo-aromatic ring, substituted or unsubstituted benzo-heteroaromatic ring, substituted or unsubstituted benzo-saturated carbocyclic ring, and substituted or unsubstituted benzo-saturated heterocyclic ring; rings A, B, and C are not none at the same time;

each of the substituents on rings A, B, and C is independently selected from the group consisting of deuterium, halogen, -CN, hydroxyl, nitro, amino,

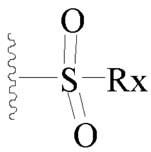

, $-L_0$-OH, $-L_3$-C(O)$R_7$, $-L_4$-CO(O)$R^8$, $-L_5$-(O)COR$^9$, $-L_6$-NHC(O)$R^{10}$, $-L_7$-C(O)NHR$^{11}$, $-L_8$-CN, an alkenyl substituted with one or more $R^{12}$, an alkynyl substituted with one or more $R^{13}$, an alkyl substituted with one or more $R^1$, an alkoxy substituted with one or more $R^2$, an aryl or an heteroaryl substituted with one or more $R^3$, a cycloalkyl substituted with one or more $R^5$, and a heterocyclic group substituted with one or more $R^6$; each of said $R_X$, $R^1$, $R^2$, $R^3$, $R^5$, $R^6$, $R^7$, $R^8$, $R^9$, $R^{10}$, $R^{11}$, $R^{12}$, $R^{13}$ is independently selected from the group consisting of H, deuterium, halogen, -CN, hydroxyl, nitro, amino, alkyl or a deuterated or halogenated compound thereof, and -Lo-OH, where each of $L_0$, $L_3$, $L_4$, $L_5$, $L_6$, $L_7$, $L_8$ is independently selected from the group consisting of none, $C_1$-$C_8$ alkyl, and cycloalkyl;

$R^4$ is independently selected from the group consisting of H, deuterium, halogen, -CN, hydroxyl, nitro, amino,

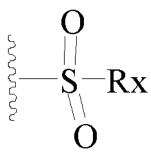

, $-L_0$-OH, $-L_3$-C(O)$R_7$, $-L_4$-CO(O)$R^8$, $-L_5$-(O)COR$^9$, $-L_6$-NHC(O)$R^{10}$, $-L_7$-C(O)NHR$^{11}$, an alkenyl substituted with one or more $R^{12}$, an alkynyl substituted with one or more $R^{13}$, an alkyl substituted with one or more $R^1$, an alkoxy substituted with one or more $R^2$, an aryl or an heteroaryl substituted with one or more $R^3$, a cycloalkyl substituted with one or more $R^5$, and a heterocyclic group substituted with one or more $R^6$; each of said $R_X$, $R^1$, $R^2$, $R^3$, $R^5$, $R^6$, $R^7$, $R^8$, $R^9$, $R^{10}$, $R^{11}$, $R^{12}$, $R^{13}$ is independently selected from the group consisting of H, deuterium, halogen, -CN, hydroxyl, nitro, amino, alkyl or a deuterated or halogenated compound thereof, and -Lo-OH, where each of $L_0$, $L_3$, $L_4$, $L_5$, $L_6$, $L_7$ is independently selected from 0-8 methylene groups;

or, any two groups of the substituents on rings A, B, and C, and $R^4$, together with the substituted atom to which they are linked are connected to form a ring.

2. The compound according to claim 1, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereoisomer thereof, or a mixture thereof, or a metabolite thereof, or a metabolic precursor thereof, or an isotope-substituted form thereof, **characterized in that** said compound has the structure of formula II:

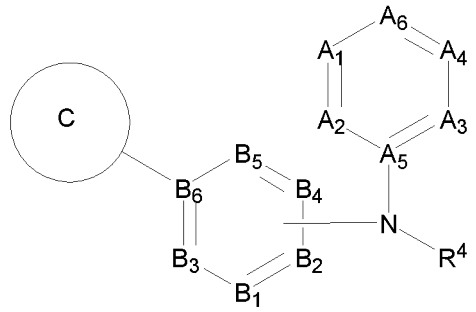

Formula II

each of $B_1$, $B_2$, $B_3$, $B_4$, $B_5$, $B_6$, $A_1$, $A_2$, $A_3$, $A_4$, $A_5$, $A_6$ is independently selected from the group consisting of $CR^0$ and N; $R^0$ is selected from the group consisting of H, -CN, amino, nitro, halogen, -Lo-OH,

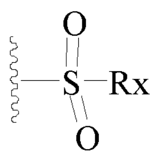

, $-C(O)NHR^{11}$, $C_1$-$C_5$ alkyl or a deuterated or halogenated or cyano-substituted compound thereof, $C_1$-$C_5$ alkoxy or a deuterated or halogenated or cyano-substituted compound thereof, substituted or unsubstituted 3-6 membered cycloalkyl, substituted or unsubstituted 3-6 membered cycloalkyl, or two adjacent substituents on the ring, together with the substituted atom to which they are linked, form a substituted or unsubstituted 3-6 membered heterocyclic ring; wherein each of the substituents in said 36 membered cycloalkyl is independently selected from the group consisting of -CN, amino, nitro, halogen, $C_1$~$C_3$ alkyl or a deuterated or halogenated compound thereof, and $-L_1$-OH; each of said $L_0$ and $L_1$ is independently 0-5 methylene groups; each of said $R_x$ and $R^{11}$ is independently selected from the group consisting of H and $C_1$-$C_3$ alkyl;
ring C and $R^4$ are as described in claim 1.

**3.** The compound according to claim 2, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereoisomer thereof, or a mixture thereof, or a metabolite thereof, or a metabolic precursor thereof, or an isotope-substituted form thereof, **characterized in that** said compound has the structure of formula III-A:

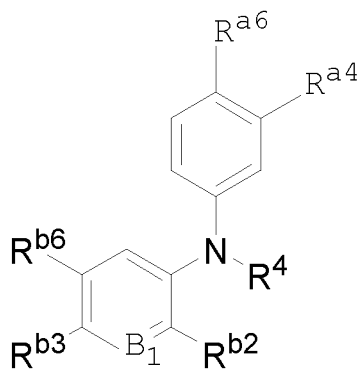

Formula III-A

wherein each of $R^{a4}$ and $R^{a6}$ is independently selected from the group consisting of H, halogen, and substituted or unsubstituted five-membered unsaturated heterocyclic ring;

wherein the substituents on the five-membered unsaturated heterocyclic ring are selected from the group consisting of deuterated or non-deuterated $C_1$-$C_2$ alkyl groups, and -$L_1$-OH; $L_1$ is 0-2 methylene groups;
$R^4$ is selected from the group consisting of H, deuterated or non-deuterated $C_1$-$C_2$ alkyl groups, and -$L_1$-OH;
$L_2$ is 0-5 methylene groups;
$B_1$ is selected from the group consisting of CH and N;
$R^{b2}$ is deuterated or non-deuterated methyl;
each of $R^{b3}$ and $R^{b6}$ is independently selected from the group consisting of H, -CN, amino, nitro, halogen, -Lo-OH,

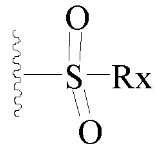

, -C(O)NHR[11], $C_1$-$C_3$ alkyl or a deuterated or halogenated or cyano-substituted compound thereof, $C_1$-$C_3$ alkoxy or a deuterated or halogenated or cyano-substituted compound thereof, substituted or unsubstituted 3-6-membered saturated cycloalkyl, and substituted or unsubstituted 5-membered unsaturated heterocyclic group, or $R^{b3}$ and $R^{b6}$, together with the substituted atom to which they are linked, form a substituted or unsubstituted 5-membered unsaturated heterocyclic ring; wherein each of the substituents in said 5-membered unsaturated heterocyclic group and 5-membered unsaturated heterocyclic ring is independently selected from the group consisting of -CN, amino, nitro, halogen, $C_1$-$C_2$ alkyl or a deuterated or halogenated compound thereof, and -$L_1$-OH; $L_1$ is 0-2 methylene groups; the substituent in said 3-6 membered saturated cycloalkyl is independently selected from CN.

**4.** The compound according to claim 2, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereoisomer thereof, or a mixture thereof, or a metabolite thereof, or a metabolic precursor thereof, or an isotope-substituted form thereof, **characterized in that** said compound has the structure of formula III-B:

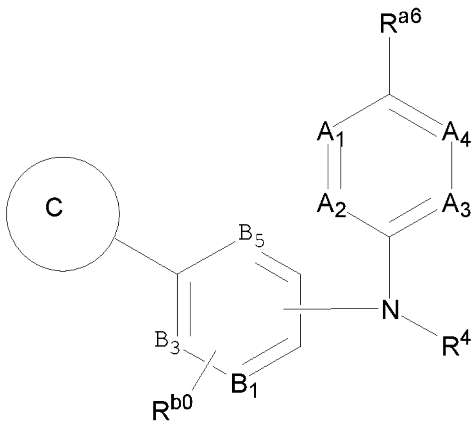

Formula III-B

wherein ring C is selected from the group consisting of

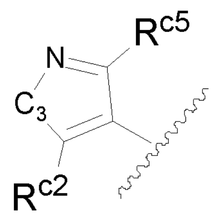

,

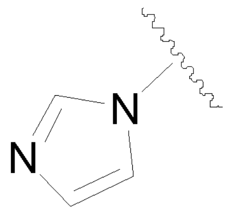

,

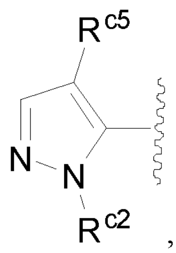

,

and

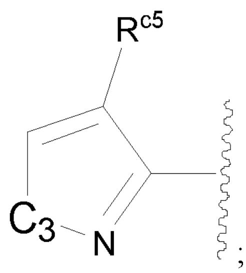

;

$C_3$ is selected from the group consisting of O and S; or each of $NR^{c3}$, $R^{c2}$, $R^{c3}$, and $R^{c5}$ is independently selected from the group consisting of H, halogen, $C_1$-$C_3$ alkoxy or a deuterated or halogenated compound thereof, and $-L_0$-OH; $L_0$ is 0-5 methylene groups;

each of $B_1$, $B_3$, and $B_5$ is independently selected from the group consisting of CH and N;
$R^{b0}$ is selected from the group consisting of H, halogen, and $C_1$-$C_3$ alkoxy or a deuterated or halogenated compound thereof;
$R^4$ is selected from the group consisting of H, $C_1$-$C_2$ alkyl or a deuterated or halogenated compound thereof, and $-L_2$-OH; $L_2$ is 0-5 methylene groups;
each of $A_1$, $A_2$, $A_3$, $A_4$ is independently selected from the group consisting of N and $CR^{a0}$; $A_5$ is C; $A_6$ is selected from the group consisting of N and $CR^{a6}$; wherein, each of $R^{a0}$ and $R^{a6}$ is independently selected from the group consisting of H, -CN, amino, nitro, halogen,

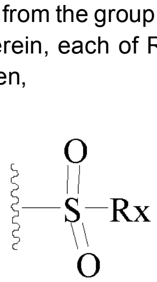

, -C(O)NHR$^{11}$, $C_1$-$C_3$ alkyl or a deuterated or halogenated or cyano-substituted compound thereof, $C_1$-$C_3$ alkoxy or a deuterated or halogenated or cyano-substituted compound thereof, substituted or unsubstituted 3-6-membered cycloalkyl, and substituted or unsubstituted 5-membered heterocyclic group, or $R^{a6}$ and the substituent at the ortho position of $R^{a6}$, together with the substituted atom to which they are linked, form a substituted or unsubstituted 5-membered heterocyclic ring; wherein each of the substituents in said 5-membered heterocyclic ring, 3-6-membered cycloalkyl, and 5-membered heterocyclic group is independently selected from the group consisting of -CN, amino, nitro, halogen, $C_1$-$C_2$ alkyl or a deuterated or halogenated compound thereof, and $-L_1$-OH; $L_1$ is 0-2 methylene groups; each of $R_x$ and $R^{11}$ is independently selected from the group consisting of H and $C_1$-$C_2$ alkyl; preferably, the 5-membered heterocyclic ring mentioned above is a 5-membered unsaturated

heterocyclic group, the 5-membered heterocyclic group mentioned above is a 5-membered unsaturated heterocyclic group, and the heteroatom is selected from the group consisting of N, S, and O; the 3-6 membered cycloalkyl mentioned above is 3-6 membered saturated cycloalkyl.

**5.** The compound according to claim 4, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereoisomer thereof, or a mixture thereof, or a metabolite thereof, or a metabolic precursor thereof, or an isotope-substituted form thereof, **characterized in that** said compound has the structure of formula III-B1:

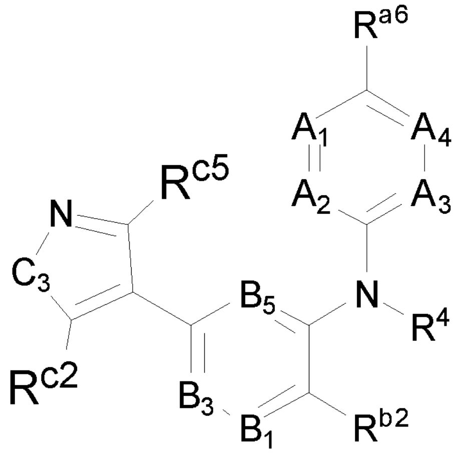

Formula III-B1

wherein $C_3$ is selected from the group consisting of O and S; or each of $NR^{c3}$, $R^{c2}$, $R^{c5}$, and $R^{c3}$ is independently selected from the group consisting of H, halogen, $C_1$-$C_3$ alkyl or a deuterated or halogenated compound thereof, and -$L_0$-OH; $L_0$ is 0-5 methylene groups;

each of $B_1$, $B_3$, and $B_5$ is independently selected from the group consisting of CH and N;
$R^{b2}$ is deuterated or undeuterated methyl;
$R^4$ is selected from the group consisting of H, deuterated or undeuterated $C_1$-$C_2$ alkyl, and -$L_2$-OH; $L_2$ is 0-5 methylene groups;
each of $A_1$, $A_2$, $A_3$, $A_4$ is independently selected from the group consisting of N and $CR^{a0}$; wherein each of $R^{a0}$ and $R^{a6}$ is independently selected from the group consisting of H, -CN, amino, nitro, halogen,

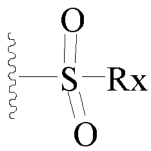

, -C(O)NHR$^{11}$, $C_1$-$C_3$ alkyl or a deuterated or halogenated or cyano-substituted compound thereof, $C_1$-$C_3$ alkoxy or a deuterated or halogenated or cyano-substituted compound thereof, substituted or unsubstituted 3-6-membered cycloalkyl, and substituted or unsubstituted 5-membered unsaturated heterocyclic group, or $R^{a6}$ and the substituent at the ortho position of $R^{a6}$, together with the substituted atom to which they are linked, form a substituted or unsubstituted 5-membered unsaturated heterocyclic ring; wherein each of the substituents in said 5-membered unsaturated heterocyclic group and 5-membered unsaturated heterocyclic ring is independently selected from the group consisting of - CN, amino, nitro, halogen, $C_1$-$C_2$ alkyl or a deuterated or halogenated compound thereof, and -$L_1$-OH; $L_1$ is 0-2 methylene groups; the substituent in said 3-6 membered saturated cycloalkyl is -CN; each of $R_x$ and $R^{11}$ is independently selected from the group consisting of H and $C_1$-$C_2$ alkyl.

**6.** The compound according to claim 5, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereoisomer thereof, or a mixture thereof, or a metabolite thereof, or a metabolic precursor thereof, or an isotope-substituted form thereof, **characterized in that** said compound has the structure

of formula III-B1a:

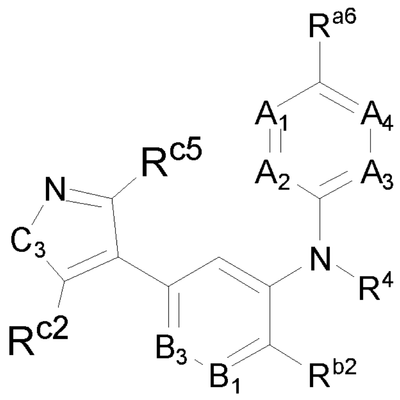

Formula III-B1a

wherein $C_3$ is selected from the group consisting of O and S; or each of $R^{c2}$ and $R^{c5}$ is independently selected from the group consisting of H and $C_1$-$C_3$ alkyl or a deuterated or halogenated compound thereof, and preferably, $R^{c2}$ and $R^{c5}$ are methyl;
each of $B_1$, $B_3$, and $B_5$ is independently selected from the group consisting of CH and N;
$R^{b2}$ is deuterated or undeuterated methyl;
$R^4$ is selected from the group consisting of H, deuterated or undeuterated $C_1$-$C_2$ alkyl, and -$L_2$-OH; $L_2$ is 0-5 methylene groups;
each of $A_1$, $A_2$, $A_3$, $A_4$ is independently selected from the group consisting of N and $CR^{a0}$; wherein each of $R^{a0}$ and $R^{a6}$ is independently selected from the group consisting of H, -CN, amino, nitro, halogen,

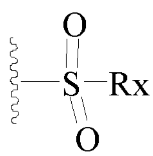

, -C(O)NHR$^{11}$, $C_1$-$C_3$ alkyl or a deuterated or halogenated or cyano-substituted compound thereof, $C_1$-$C_3$ alkoxy or a deuterated or halogenated or cyano-substituted compound thereof, substituted or unsubstituted 3-6-membered saturated cycloalkyl, and substituted or unsubstituted 5-membered unsaturated heterocyclic group, or $R^{a6}$ and the substituent at the ortho position of $R^{a6}$, together with the substituted atom to which they are linked, form a substituted or unsubstituted 5-membered unsaturated heterocyclic ring; wherein each of the substituents in said 5-membered unsaturated heterocyclic group and 5-membered unsaturated heterocyclic ring is independently selected from the group consisting of - CN, amino, nitro, halogen, $C_1$-$C_2$ alkyl or a deuterated or halogenated compound thereof, and -$L_1$-OH; $L_1$ is 0-2 methylene groups; the substituent in said 3-6 membered saturated cycloalkyl is -CN; each of $R_x$ and $R^{11}$ is independently selected from the group consisting of H and $C_1$-$C_2$ alkyl;
or, the compound has a structure of formula III-B1b:

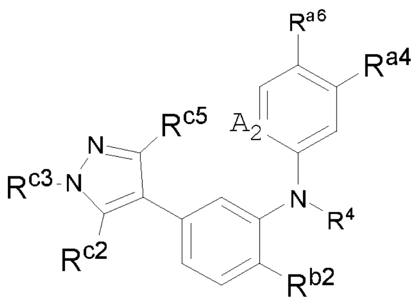

Formula III-B1b

wherein each of $R^{c2}$, $R^{c3}$and $R^{c5}$ is independently selected from the group consisting of H, $C_1$-$C_3$ alkyl or a deuterated or halogenated compound thereof, and -$L_0$-OH; preferably, $R^{c3}$ is selected from the group consisting of H, $C_1$-$C_3$ alkyl or a deuterated or halogenated compound thereof, and -Lo-OH, while $R^{c2}$and $R^{c5}$ are methyl; wherein, $L_0$ is 0-5 methylene groups;
$R^4$ is selected from the group consisting of H, deuterated or undeuterated $C_1$-$C_2$ alkyl, and -$L_2$-OH; $L_2$ is 0-5 methylene groups;
$R^{b2}$ is deuterated or undeuterated methyl;
$A_2$ is N or CH;
each of $R^{a4}$ and $R^{a6}$ is independently selected from the group consisting of H, -CN, amino, nitro, halogen,

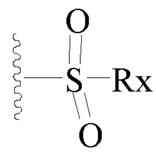

, -C(O)NHR$^{11}$, $C_1$-$C_3$ alkyl or a deuterated or halogenated or cyano-substituted compound thereof, $C_1$-$C_3$ alkoxy or a deuterated or halogenated or cyano-substituted compound thereof, substituted or unsubstituted 3-6-membered saturated cycloalkyl, and substituted or unsubstituted 5-membered unsaturated heterocyclic group, or $R^{a6}$ and the substituent at the ortho position of $R^{a6}$, together with the substituted atom to which they are linked, form a substituted or unsubstituted 5-membered unsaturated heterocyclic ring; wherein each of the substituents in said 5-membered unsaturated heterocyclic group and 5-membered unsaturated heterocyclic ring is independently selected from the group consisting of - CN, amino, nitro, halogen, $C_1$-$C_2$ alkyl or a deuterated or halogenated compound thereof, and -$L_1$-OH; $L_1$ is 0-2 methylene groups; the substituent in said 3-6 membered saturated cycloalkyl is -CN; each of $R_x$ and $R^{11}$ is independently selected from the group consisting of H and $C_1$-$C_2$ alkyl.

7. The compound according to claim 4, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereoisomer thereof, or a mixture thereof, or a metabolite thereof, or a metabolic precursor thereof, or an isotope-substituted form thereof, **characterized in that** said compound has the structure of formula III-B2:

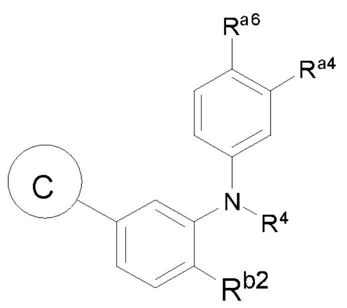

Formula III-B2

wherein ring C is selected from

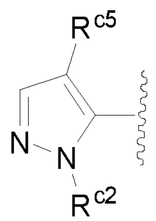

and

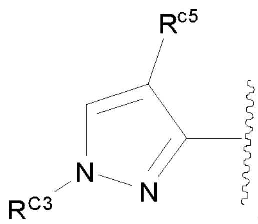

;

each of $R^{c2}$, $R^{c3}$, and $R^{c5}$ is independently selected from the group consisting of H, and $C_1$-$C_3$ alkyl or a deuterated or halogenated compound thereof, and preferably selected from the group consisting of H and methyl;

$R^4$ is selected from the group consisting of H, deuterated or undeuterated $C_1$-$C_2$ alkyl, and $-L_2$-OH; $L_2$ is 0-5 methylene groups;

$R^{b2}$ is deuterated or undeuterated methyl;

each of $R^{a4}$ and $R^{a6}$ is independently selected from the group consisting of H, -CN, amino, nitro, halogen,

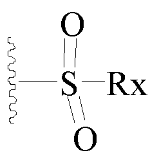

, -C(O)NHR$^{11}$, $C_1$-$C_3$ alkyl or a deuterated or halogenated or cyano-substituted compound thereof, $C_1$-$C_3$ alkoxy or a deuterated or halogenated or cyano-substituted compound thereof, substituted or unsubstituted 3~6-membered saturated cycloalkyl, and substituted or unsubstituted 5-membered unsaturated heterocyclic group, or $R^{a6}$ and the substituent at the ortho position of $R^{a6}$, together with the substituted atom to which they are linked, form a substituted or unsubstituted 5-membered unsaturated heterocyclic ring; wherein each of the substituents in said 5-membered unsaturated heterocyclic group and 5-membered unsaturated heterocyclic ring is independently selected from the group consisting of - CN, amino, nitro, halogen, $C_1$-$C_2$ alkyl or a deuterated or halogenated compound thereof, and $-L_1$-OH; $L_1$ is 0-2 methylene groups; the substituent in said 3-6 membered saturated cycloalkyl is -CN; each of $R_x$ and $R^{11}$ is independently selected from the group consisting of H and $C_1$-$C_2$ alkyl.

**8.** The compound according to any one of claims 1 to 7, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereoisomer thereof, or a mixture thereof, or a metabolite thereof, or a metabolic precursor thereof, or an isotope-substituted form thereof, **characterized in that** said compound is selected from the group consisting of:

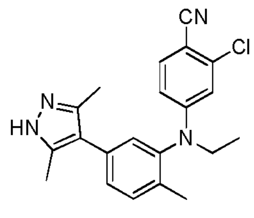
1

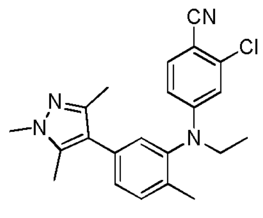
2

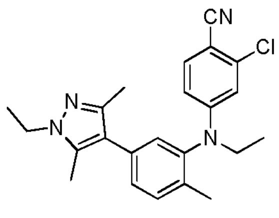
3

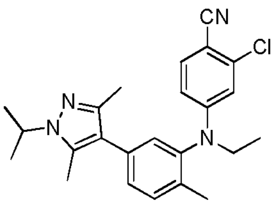
4

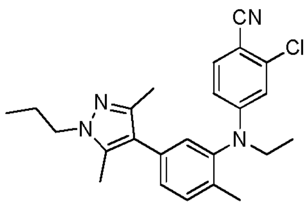
5

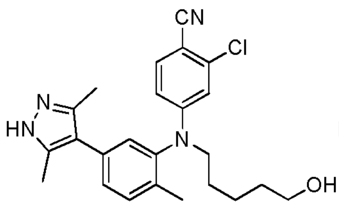
6

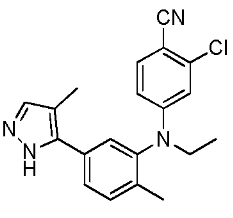
7

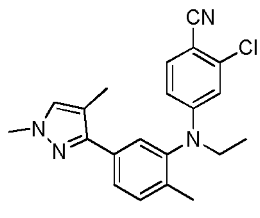
8

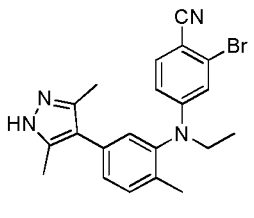
9

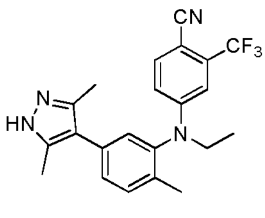
10

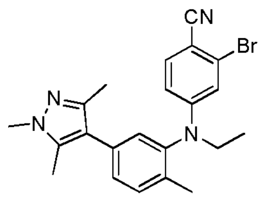
11

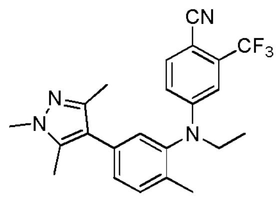
12

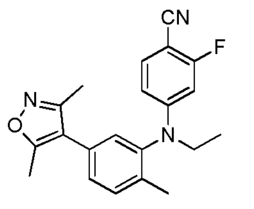
13

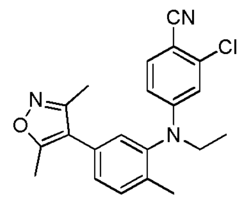
14

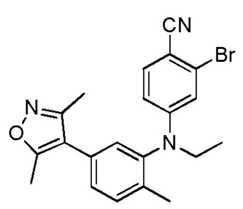
15

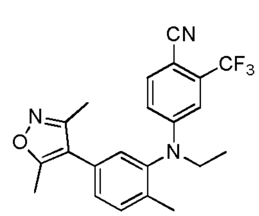
16

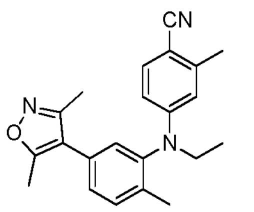
17

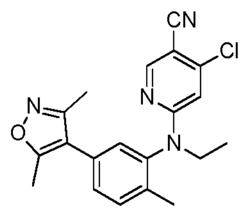
18

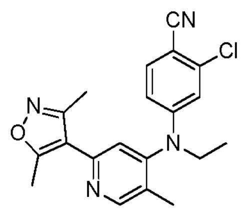
19

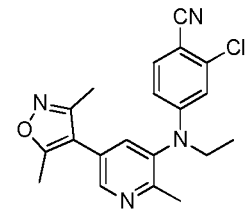
20

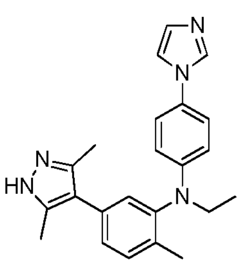
21

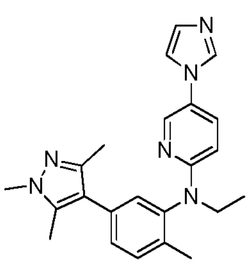
22

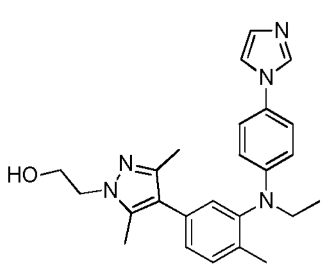
23

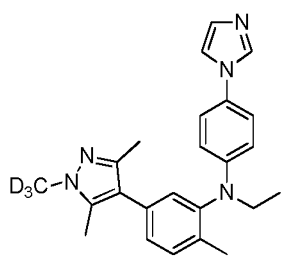
24

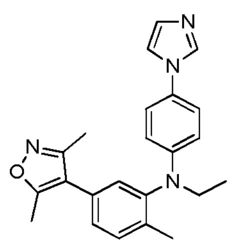

25

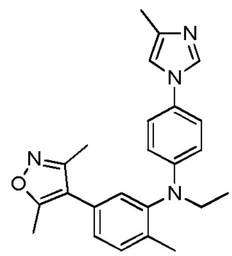

26

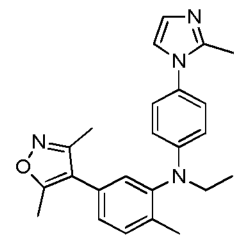

27

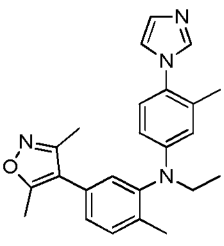

28

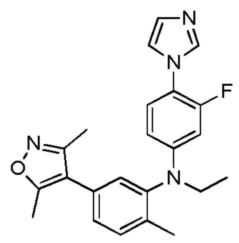

29

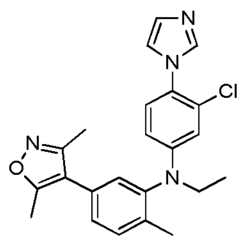

30

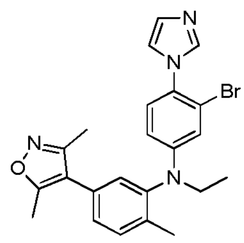

31

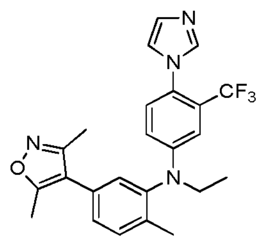

32

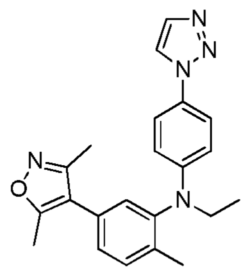

33

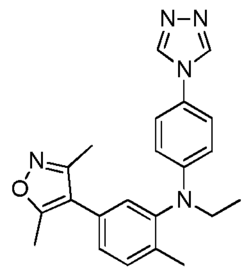

34

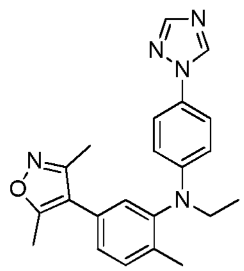

35

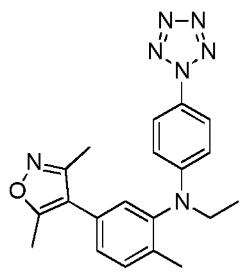

36

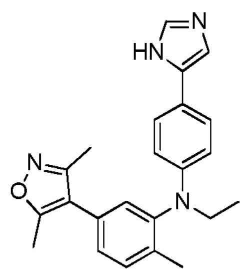

37

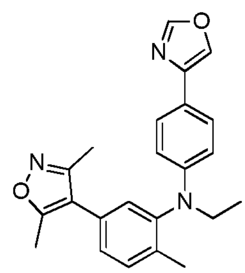

38

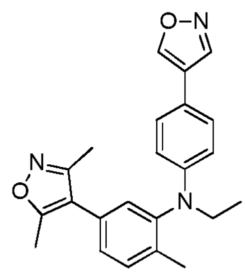

39

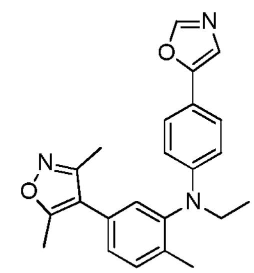

40

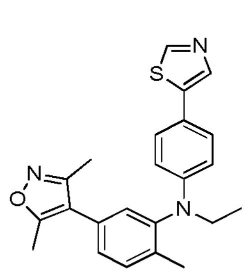

41

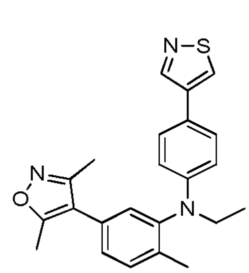

42

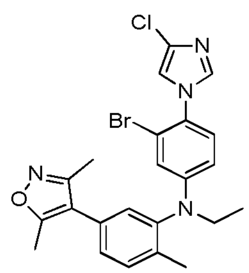

43

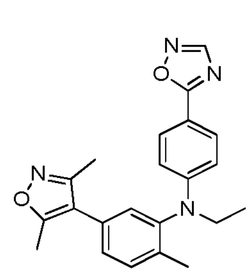

44

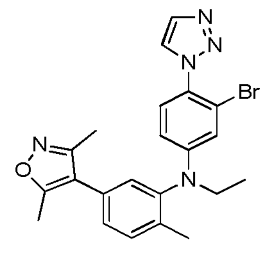

45

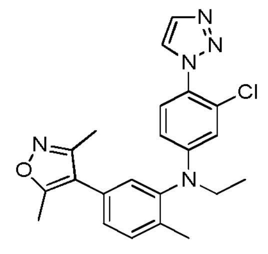

46

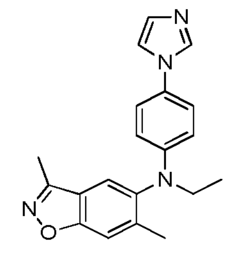

47

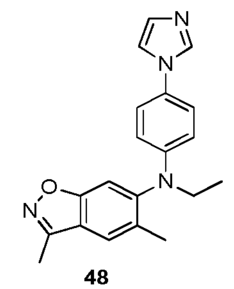

48

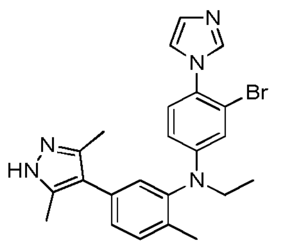

49

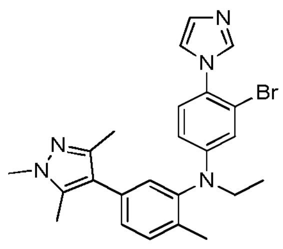

50

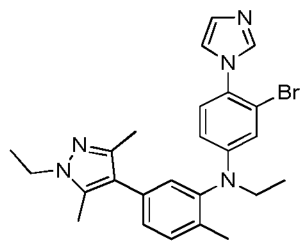

51

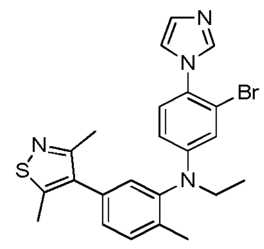

52

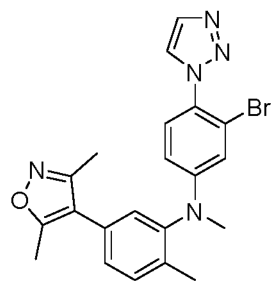

53

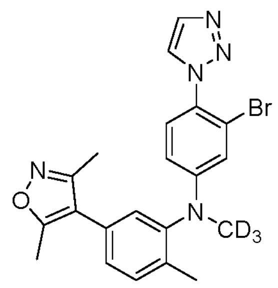

54

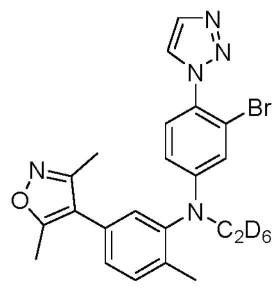

55

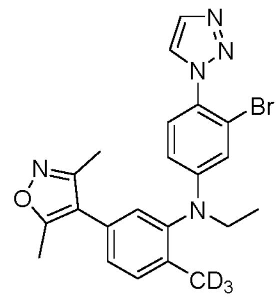

56

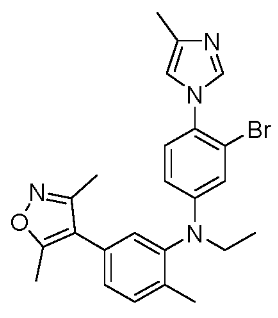

57

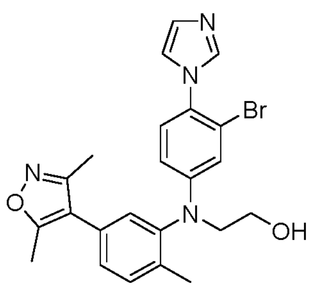

58

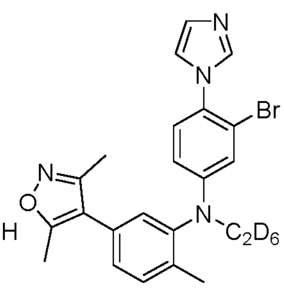

59

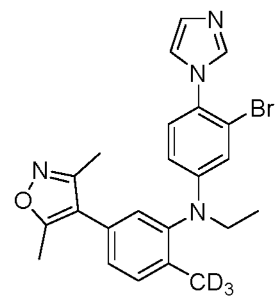

60

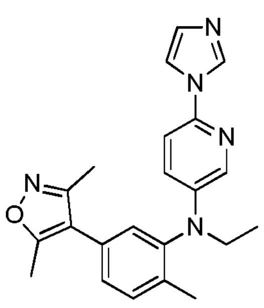

61

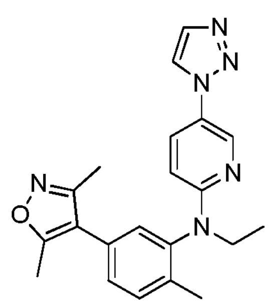

62

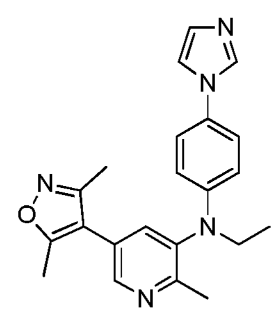

63

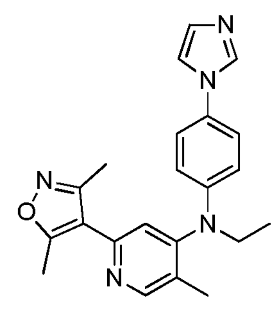

64

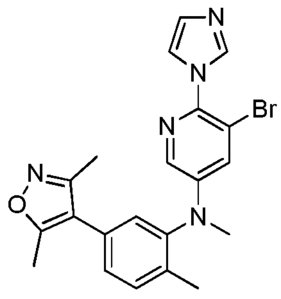

**65**

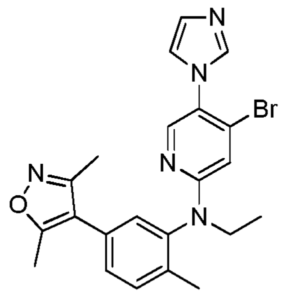

**66**

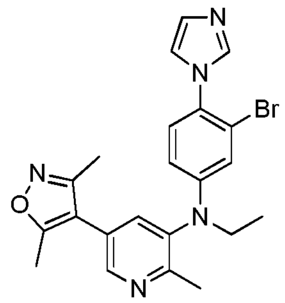

**67**

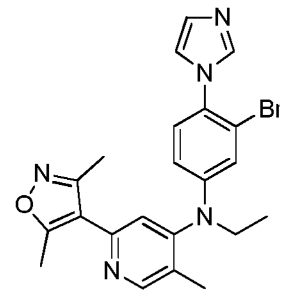

**68**

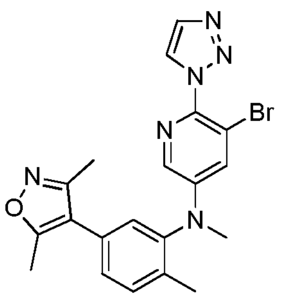

**69**

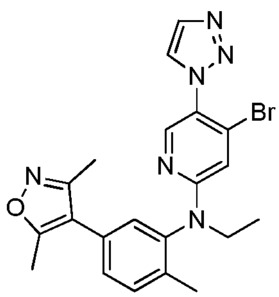

**70**

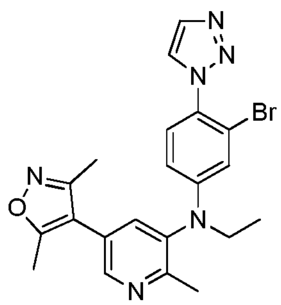

**71**

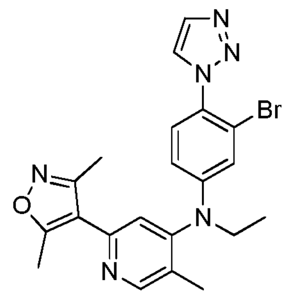

**72**

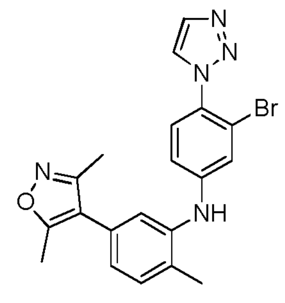

**73**

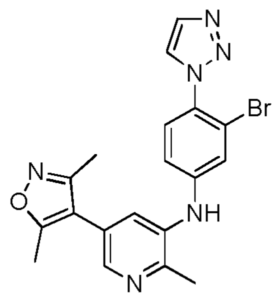

**74**

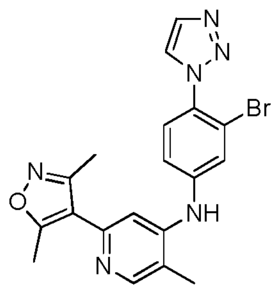

**75**

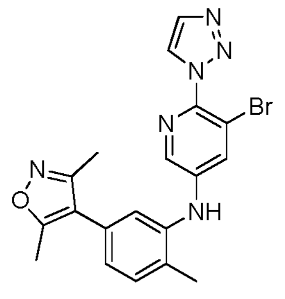

**76**

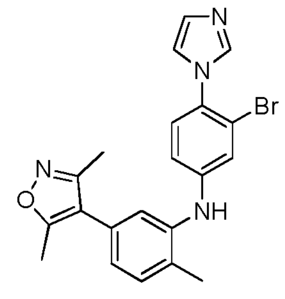

**77**

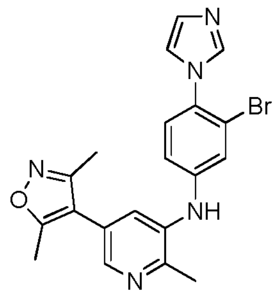

**78**

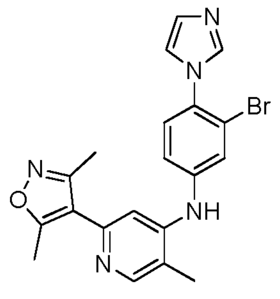

**79**

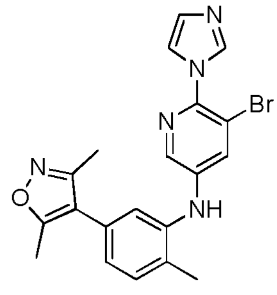

**80**

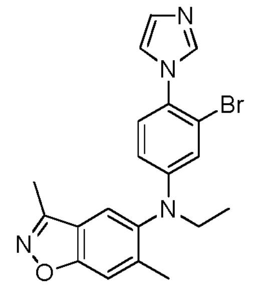

81

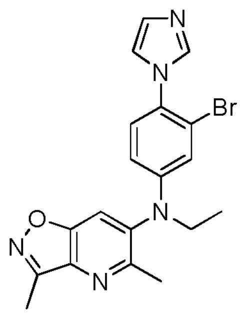

82

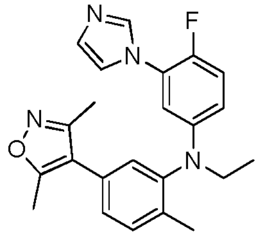

83

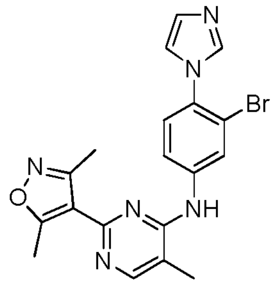

84

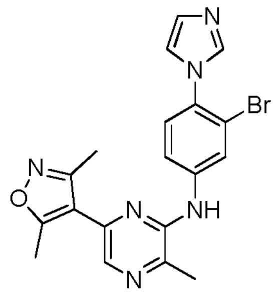

85

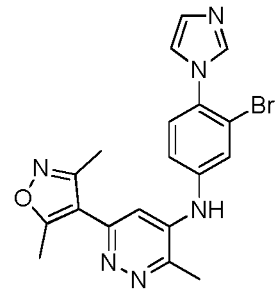

86

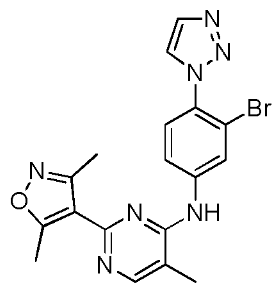

87

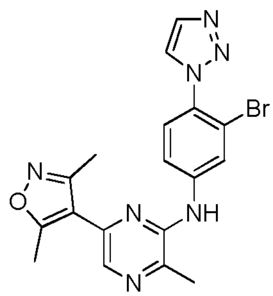

88

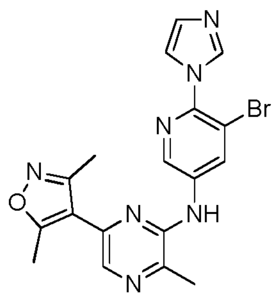

89

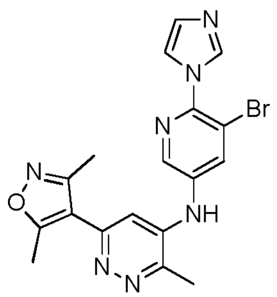

90

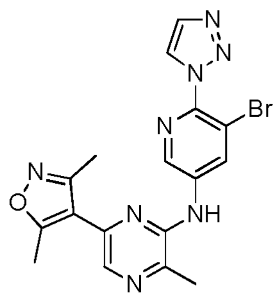

91

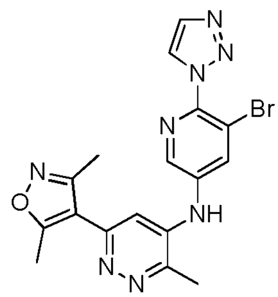

92

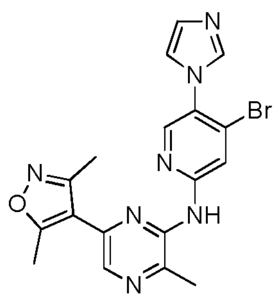

93

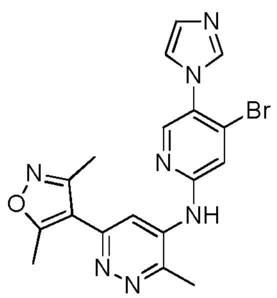

94

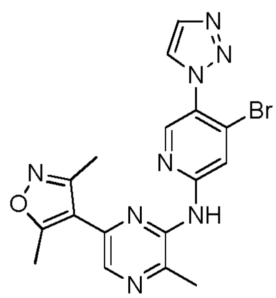

95

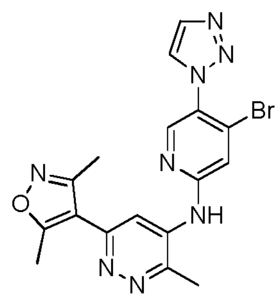

96

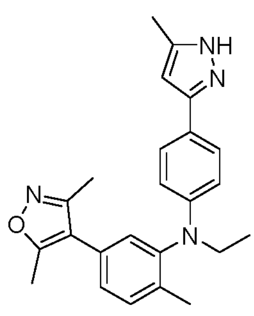

**97**

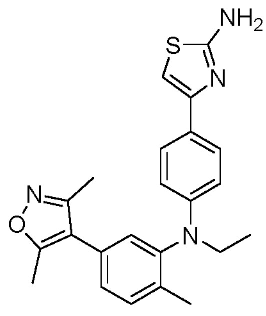

**98**

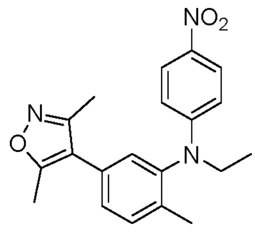

**99**

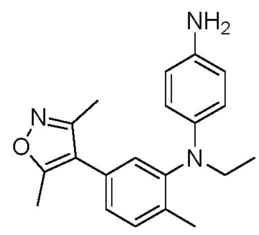

**100**

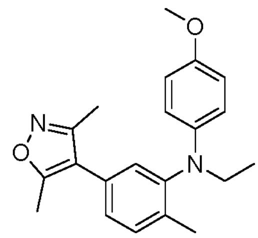

**101**

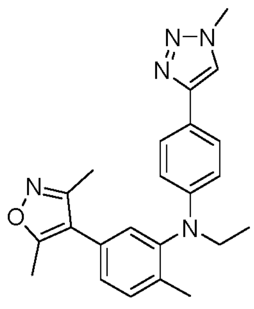

**102**

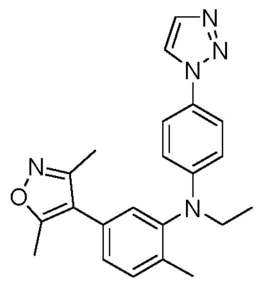

**103**

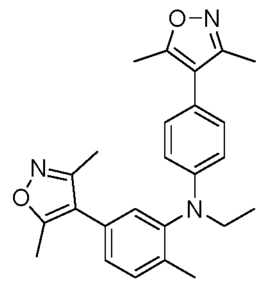

**104**

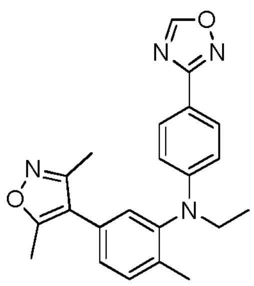

**105**

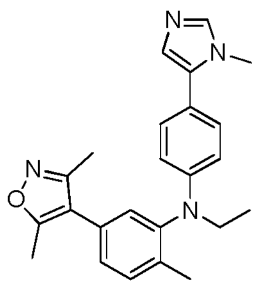

**106**

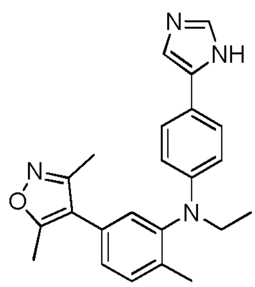

**107**

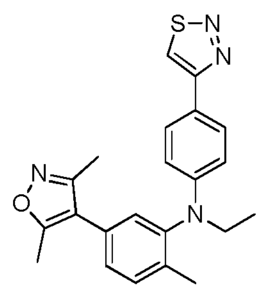

**108**

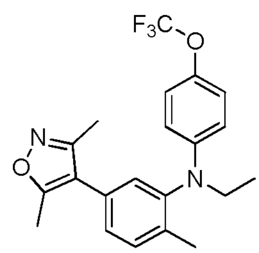

**109**

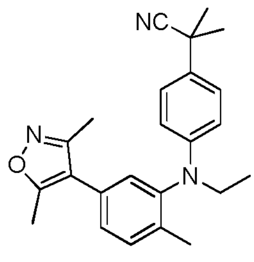

**110**

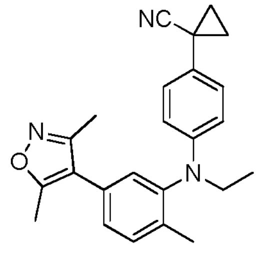

**111**

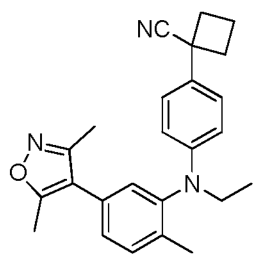

**112**

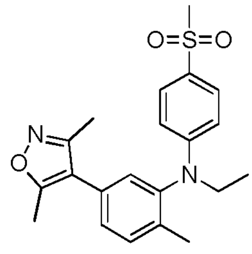
113

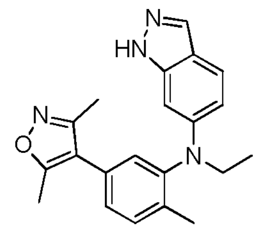
114

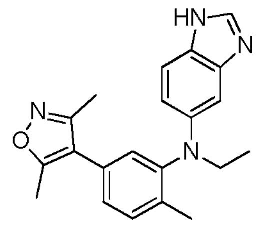
115

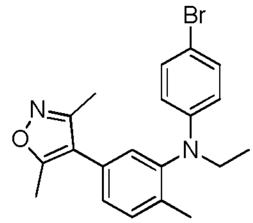
116

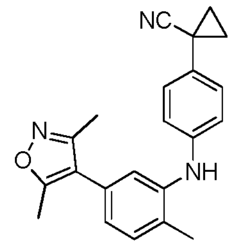
117

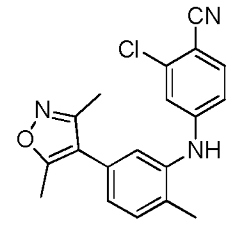
118

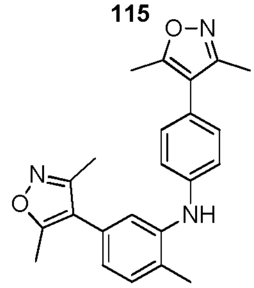
119

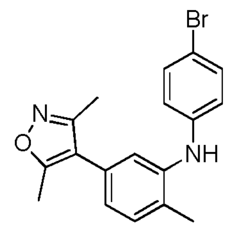
120

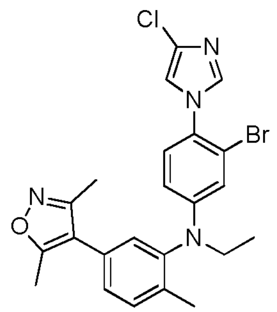
121

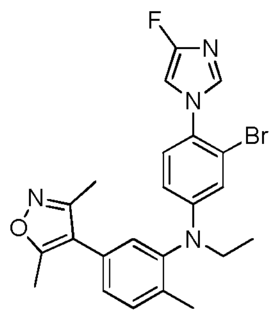
122

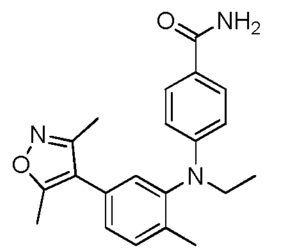
123

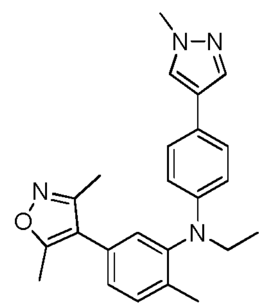
124

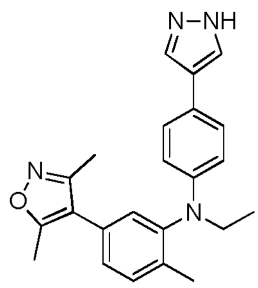
125

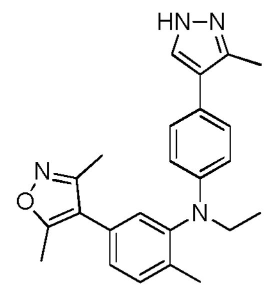
126

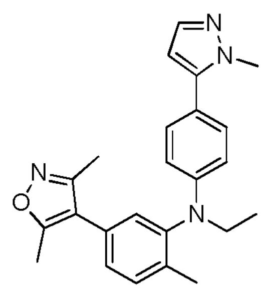
127

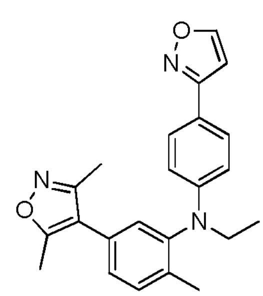
128

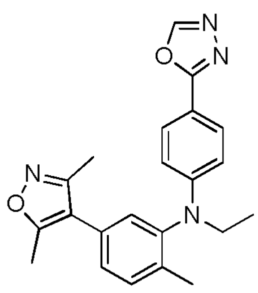
129

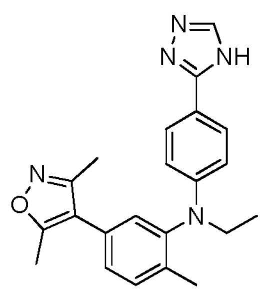
130

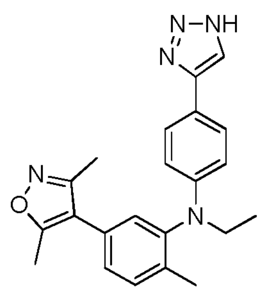
131

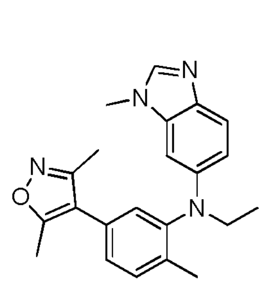
132

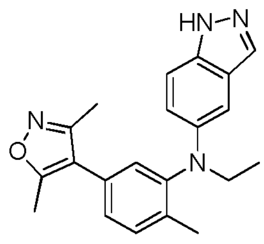

133

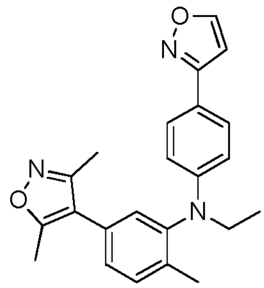

134

9. Use of the compound according to any one of claims 1 to 8, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereoisomer thereof, or a mixture thereof, or a metabolite thereof, or a metabolic precursor thereof, or an isotope-substituted form thereof in the preparation of AR and/or BRD4 inhibitors, and/or proteolytic targeting chimera; preferably, said inhibitors and/or proteolytic targeting chimera is a drug for the treatment of diseases related to AR and/or BRD4; the drug is preferably those for treating prostate cancer, and the prostate cancer is preferably resistant to enzalutamide.

10. The use according to claim 9, **characterized in that** said proteolytic targeting chimera can target the degradation of AR and/or BRD4, and/or down-regulate the expression of full-length androgen receptor and/or androgen receptor variants.

**INTERNATIONAL SEARCH REPORT**

International application No.
**PCT/CN2020/082513**

**A. CLASSIFICATION OF SUBJECT MATTER**

C07D 231/12(2006.01)i; C07D 261/08(2006.01)i; C07D 413/04(2006.01)i; C07D 413/12(2006.01)i; C07D 413/14(2006.01)i; C07D 401/14(2006.01)i; C07D 403/12(2006.01)i; C07D 403/14(2006.01)i; C07D 417/12(2006.01)i; C07D 498/04(2006.01)i; A61K 31/4155(2006.01)i; A61K 31/422(2006.01)i; A61K 31/423(2006.01)i; A61K 31/427(2006.01)i; A61K 31/4439(2006.01)i; A61K 31/497(2006.01)i; A61K 31/501(2006.01)i; A61K 31/506(2006.01)i; A61K 31/4355(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNPAT, CNKI, 万方, EPODOC, WPI, STN: 海创药业, 陈元伟, 雄激素受体, 溴结构域, 前列腺癌, 胺, 异噁唑, 吡唑, 异噻唑, androgen receptor, AR, bromodomain, BET, BRD, prostate cancer, isoxazole, pyrazole, isothiazole, amine, structural search

**C. DOCUMENTS CONSIDERED TO BE RELEVANT**

| Category* | Citation of document, with indication, where appropriate, of the relevant passages | Relevant to claim No. |
|---|---|---|
| X | CN 107814785 A (SICHUAN UNIVERSITY) 20 March 2018 (2018-03-20)<br>see abstract, claims 1-22 | 1-10 |
| X | YU, Jiang, et al. "Discovery and biological evaluation of novel androgen receptor antagonist for castration-resistant prostate cancer"<br>*European Journal of Medicinal Chemistry,* Vol. 171, 22 March 2019 (2019-03-22), ISSN: 0223-5234,<br>pp. 265-281 | 1-10 |
| X | CN 108697710 A (JANSSEN PHARMACEUTICA NV) 23 October 2018 (2018-10-23)<br>see abstract, claims 1, 13 | 1, |
| X | CN 104918919 A (PTC THERAPEUTICS INC) 16 September 2015 (2015-09-16)<br>see abstract, claim 1, description paragraph [0099] | 1-2, |
| X | "RN 179336-31-3, et al."<br>*STN Registry,* 09 August 1996 (1996-08-09),<br>see pages 2-4 | 1, 2, 4 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 June 2020** | **01 July 2020** |
| Name and mailing address of the ISA/CN<br>**China National Intellectual Property Administration (ISA/CN)**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/082513**

**C. DOCUMENTS CONSIDERED TO BE RELEVANT**

| Category* | Citation of document, with indication, where appropriate, of the relevant passages | Relevant to claim No. |
|---|---|---|
| PX | CN 109608394 A (HUNAN UNIVERSITY) 12 April 2019 (2019-04-12)<br>see description embodiment 4 | 1, |
| PX | WANG, Xia, et al. "Anticancer-Active N-Heteroaryl Amines Syntheses: Nucleophilic Amination of N-Heteroaryl Alkyl Ethers with Amines"<br>*Organic Letters,* Vol. 21, No. 13, 14 June 2019 (2019-06-14),<br>ISSN: 1523-7052,<br>pp. 5111-5115 | 1, |
| E | WO 2020063968 A1 (SICHUAN UNIVERSITY) 02 April 2020 (2020-04-02)<br>see claims 1-21 | 1-10 |

**INTERNATIONAL SEARCH REPORT**
**Information on patent family members**

International application No.
**PCT/CN2020/082513**

| Patent document cited in search report | Publication date (day/month/year) | Patent family member(s) | Publication date (day/month/year) |
|---|---|---|---|
| CN 107814785 A | 20 March 2018 | None | |
| CN 108697710 A | 23 October 2018 | EP 3405196 B1 | 04 December 2019 |
| | | AU 2017209935 A1 | 09 August 2018 |
| | | LT 3405196 T | 25 February 2020 |
| | | DK 3405196 T3 | 09 March 2020 |
| | | MX 2018008974 A | 09 November 2018 |
| | | SI 3405196 T1 | 31 March 2020 |
| | | BR 112018014675 A2 | 11 December 2018 |
| | | JP 2019504067 A | 14 February 2019 |
| | | KR 20180100441 A | 10 September 2018 |
| | | EP 3405196 A1 | 28 November 2018 |
| | | WO 2017125530 A1 | 27 July 2017 |
| | | PH 12018501567 A1 | 28 January 2019 |
| | | CA 3011880 A1 | 27 July 2017 |
| | | TW 201728577 A | 16 August 2017 |
| CN 104918919 A | 16 September 2015 | PE 14132015 A1 | 23 October 2015 |
| | | US 2020024260 A1 | 23 January 2020 |
| | | US 2015315182 A1 | 05 November 2015 |
| | | SG 11201503982 X A | 29 June 2015 |
| | | AU 2013348009 A1 | 04 June 2015 |
| | | AR 093579 A1 | 10 June 2015 |
| | | TW 201427972 A | 16 July 2014 |
| | | NZ 708909 A | 29 November 2019 |
| | | CU 24387 B1 | 04 March 2019 |
| | | CL 2015001377 A1 | 19 February 2016 |
| | | BR 112015011760 A2 | 11 July 2017 |
| | | TW I623531 B | 11 May 2018 |
| | | WO 2014081906 A2 | 30 May 2014 |
| | | AU 2013348009 C1 | 08 August 2019 |
| | | CU 20150053 A7 | 27 November 2015 |
| | | WO 2014081906 A3 | 17 July 2014 |
| | | NZ 746607 A | 29 November 2019 |
| | | JP 2019031509 A | 28 February 2019 |
| | | IL 238871 D0 | 30 July 2015 |
| | | IL 265253 D0 | 30 May 2019 |
| | | JP 6412503 B2 | 24 October 2018 |
| | | MX 2015006469 A | 29 October 2015 |
| | | CR 20150294 A | 20 August 2015 |
| | | EA 201890142 A1 | 29 June 2018 |
| | | PE 20151413 A1 | 23 October 2015 |
| | | CA 2892045 A1 | 30 May 2014 |
| | | KR 20150086345 A | 27 July 2015 |
| | | JP 2016504290 A | 12 February 2016 |
| | | EA 201590992 A1 | 30 November 2015 |
| | | IL 238871 A | 31 March 2019 |
| | | PH 12015501130 A1 | 03 August 2015 |
| | | HK 1215032 A1 | 12 August 2016 |
| | | EP 2922828 A2 | 30 September 2015 |
| | | AU 2013348009 B2 | 17 May 2018 |
| | | EA 031405 B1 | 28 December 2018 |

Form PCT/ISA/210 (patent family annex) (January 2015)

**INTERNATIONAL SEARCH REPORT**
**Information on patent family members**

International application No.
**PCT/CN2020/082513**

| Patent document cited in search report | | | Publication date (day/month/year) | Patent family member(s) | | | Publication date (day/month/year) |
|---|---|---|---|---|---|---|---|
| | | | | US | 10428050 | B2 | 01 October 2019 |
| | | | | UA | 118094 | C2 | 26 November 2018 |
| | | | | SG | 10201600149V | A | 26 February 2016 |
| | | | | EP | 2922828 | A4 | 27 July 2016 |
| | | | | JP | 6617186 | B2 | 11 December 2019 |
| CN | 109608394 | A | 12 April 2019 | None | | | |
| WO | 2020063968 | A1 | 02 April 2020 | CN | 110960528 | A | 07 April 2020 |

Form PCT/ISA/210 (patent family annex) (January 2015)